# EUROPEAN PATENT APPLICATION

(11) **EP 4 556 481 A1**
(43) Date of publication of application: **21.05.2025**
(21) Application number: 23838947.2
(22) Date of filing: 11.07.2023
(51) Int. Cl.: C07D 519/00, A61K 31/519, A61P 35/00

(54) **PIPERAZINE BRIDGE-SUBSTITUTED HETEROCYCLIC PYRIMIDINE COMPOUND**

(30) Priority: 12.07.2022 CN 202210822828; 14.10.2022 CN 202211262711; 10.11.2022 CN 202211407253; 11.01.2023 CN 202310041534; 12.01.2023 CN 202310041762; 19.01.2023 CN 202310066868; 30.06.2023 CN 202310800516
(71) Applicant: Medshine Discovery Inc., Nanjing, Jiangsu 210032 (CN)
(72) Inventor: ZHANG, Yang, Shanghai 200131 (CN); FU, Zhifei, Shanghai 200131 (CN); YU, Chenxi, Shanghai 200131 (CN); LUO, Miaorong, Shanghai 200131 (CN); CHEN, Jian, Shanghai 200131 (CN); SUN, Jikui, Shanghai 200131 (CN); HU, Boyu, Shanghai 200131 (CN); GAO, Na, Shanghai 200131 (CN); LI, Jian, Shanghai 200131 (CN); CHEN, Shuhui, Shanghai 200131 (CN)
(74) Representative: De Clercq & Partners
(86) International application number: PCT/CN2023/106806
(87) International publication number: WO 2024/012456

(57) **Abstract**

Disclosed are a piperazine bridge-substituted heterocyclic pyrimidine compound or a pharmaceutically acceptable salt thereof. Specifically disclosed is a compound represented by formula (III-1) or a pharmaceutically acceptable salt thereof.

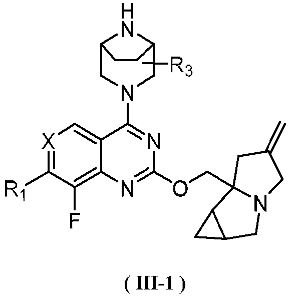

## Description

The present invention claims the right of the following priorities:
Application No.: CN202210822828.X, application date: July 12, 2022;
Application No.: CN202211262711.7, application date: October 14, 2022;
Application No.: CN202211407253.1, application date: November 10, 2022;
Application No.: CN202310041534.8, application date: January 11, 2023;
Application No.: CN202310041762.5, application date: January 12, 2023;
Application No.: CN202310066868.0, application date: January 19, 2023;
Application No.: CN202310800516.3, application date: June 30, 2023.

### TECHNICAL FIELD

The present disclosure relates to a class of piperazine bridge-substituted heterocyclic pyrimidine compounds or a pharmaceutically acceptable salt thereof, and specifically to a compound represented by formula (III-1) or a pharmaceutically acceptable salt thereof.

### BACKGROUND

Cancers caused by NRAS, HRAS, and KRAS mutations in the RAS family account for nearly a quarter of all human cancers, making them one of the most common gene mutations related to cancer. These cancers cover almost all types of cancer and cause one million deaths globally each year. Among them, KRAS is the most common oncogene (accounting for 85% of all RAS mutations), present in 90% of pancreatic cancers, 30-40% of colon cancers, and 15-20% of lung cancers (mostly non-small cell lung cancer). According to the specific mutations present, G12C, G12D, and G12R are the most common KRAS mutations in patients. In addition, there are G12A, G12S, G12V, *etc.*

RAS (Rat Sarcoma) family proteins are widely expressed in various eukaryotes and have two expression forms: a GDP (guanosine diphosphate)-bound form in the inactive state and a GTP (guanosine triphosphate)-bound form in the activated state. RAS protein regulates multiple downstream pathways including RAF-MEK-ERK and PI3K/Akt/mTOR through switching between two expression forms, thereby affecting the growth and differentiation of cells and the occurrence and development of tumors.

Due to the high affinity of mutant KRAS for guanosine triphosphate (GTP) and the difficulty in targeting factors such as small catalytic sites and smooth protein surfaces, the development of small-molecule inhibitors has been challenging, resulting in the legend of KRAS as "undruggable". With Mirati's breakthrough in KRAS G12D non-covalent inhibitors, KRAS G12D mutated tumors have gradually entered the field of precision medicine.

### CONTENT OF THE PRESENT INVENTION

The present disclosure provides a compound represented by formula (III-1) or a pharmaceutically acceptable salt thereof, 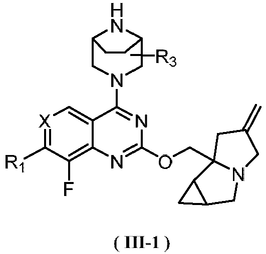 wherein
X is selected from CH, C-Rx, N, and N⁺-O⁻; preferably, X is selected from N and N⁺-O⁻; Rx is selected from F, Cl, and Br;
R₁ is selected from 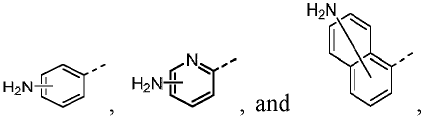 and the 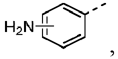 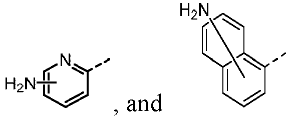 are each independently and optionally substituted by 1, 2, 3, or 4 Rₐ;
R₃ is selected from H and D;
each Rₐ is independently selected from F, Cl, Br, I, OH, NH₂, C₁₋₃ alkyl, C₁₋₃ alkoxy, C₁₋₃ haloalkyl, C₂₋₄ alkenyl, C₂₋₄ alkynyl, -C₁₋₃ alkyl-cyclopropyl, and cyclopropyl, and the C₁₋₃ alkyl, C₁₋₃ alkoxy, C₂₋₄ alkenyl, C₂₋₄ alkynyl, cyclopropyl, and -C₁₋₃ alkyl-cyclopropyl are each independently and optionally substituted by 1, 2, or 3 R;
each R is independently selected from F, Cl, Br, I, CH₂F, CHF₂, and CF₃.

The present disclosure provides a compound represented by formula (III-1) or a pharmaceutically acceptable salt thereof, 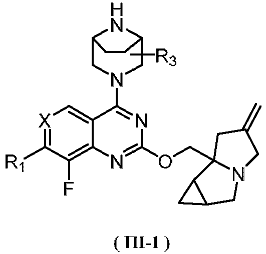 wherein
X is selected from CH, N, and N⁺-O⁻; preferably, X is selected from N and N⁺-O⁻;
R₁ is selected from 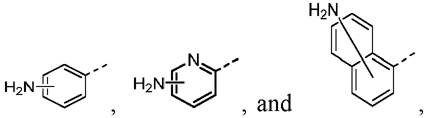 and the 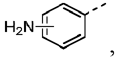 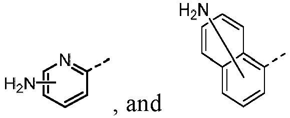 are each independently and optionally substituted by 1, 2, 3, or 4 Rₐ;
R₃ is selected from H and D;
each Rₐ is independently selected from F, Cl, Br, I, OH, NH₂, C₁₋₃ alkyl, C₁₋₃ alkoxy, C₂₋₄ alkenyl, C₂₋₄ alkynyl, -C₁₋₃ alkyl-cyclopropyl, and cyclopropyl, and the C₁₋₃ alkyl, C₁₋₃ alkoxy, C₂₋₄ alkenyl, C₂₋₄ alkynyl, cyclopropyl, and -C₁₋₃ alkyl-cyclopropyl are each independently and optionally substituted by 1, 2, or 3 R;
each R is independently selected from F, Cl, Br, I, CH₂F, CHF₂, and CF₃.

The present disclosure further provides a compound represented by formula (III-1) or a pharmaceutically acceptable salt thereof, 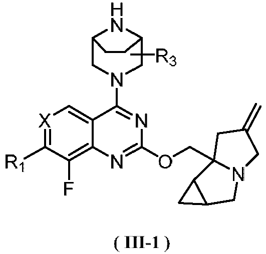 wherein
X is selected from N and N⁺-O⁻;
R₁ is selected from 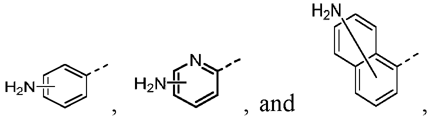 and the 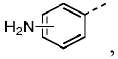 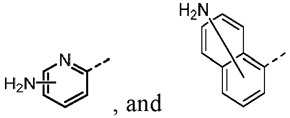 are each independently and optionally substituted by 1, 2, 3, or 4 Rₐ;
R₃ is selected from H and D;
each Rₐ is independently selected from F, Cl, Br, I, OH, NH₂, C₁₋₃ alkyl, C₁₋₃ alkoxy, C₂₋₄ alkenyl, C₂₋₄ alkynyl, -C₁₋₃ alkyl-cyclopropyl, and cyclopropyl, and the C₁₋₃ alkyl, C₁₋₃ alkoxy, C₂₋₄ alkenyl, C₂₋₄ alkynyl, cyclopropyl, and -C₁₋₃ alkyl-cyclopropyl are each independently and optionally substituted by 1, 2, or 3 R;
each R is independently selected from F, Cl, Br, I, CH₂F, CHF₂, and CF₃.

The present disclosure further provides a compound represented by formula (III-2) or a pharmaceutically acceptable salt thereof, 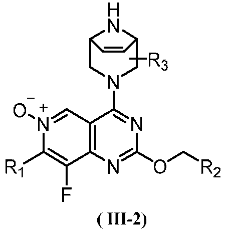 wherein
R₁ is selected from phenyl, pyridyl, and naphthyl, and the phenyl, pyridyl, and naphthyl are each independently and optionally substituted by 1, 2, 3, or 4 Rₐ;
R₂ is selected from 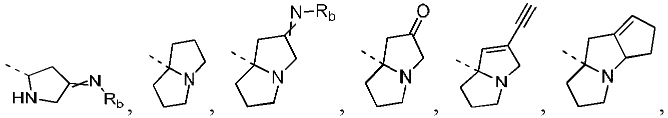 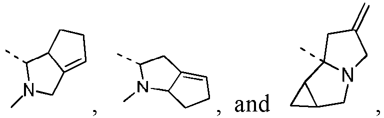 and the 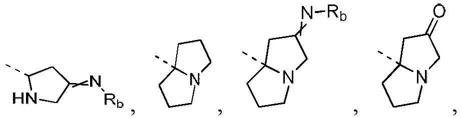 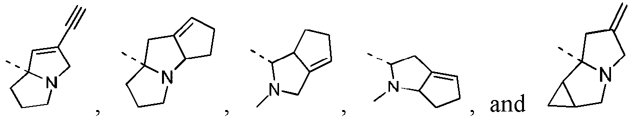 are each independently and optionally substituted by 1, 2, or 3 R_{c};
R₃ is selected from H and D;
each Rₐ is independently selected from F, Cl, Br, I, OH, NH₂, C₁₋₃ alkyl, C₁₋₃ alkoxy, C₂₋₄ alkenyl, C₂₋₄ alkynyl, -C₁₋₃ alkyl-cyclopropyl, and cyclopropyl, and the C₁₋₃ alkyl, C₁₋₃ alkoxy, C₂₋₄ alkenyl, C₂₋₄ alkynyl, cyclopropyl, and -C₁₋₃ alkyl-cyclopropyl are each independently and optionally substituted by 1, 2, or 3 R;
R_{b} is selected from H, CN, CH₃, and OCH₃;
each R_{c} is independently selected from F, Cl, Br, I, CH₂F, CHF₂, CF₃, and CH₂CF₃;
each R is independently selected from F, Cl, Br, I, CH₂F, CHF₂, and CF₃.

The present disclosure further provides a compound represented by formula (II) or a pharmaceutically acceptable salt thereof, 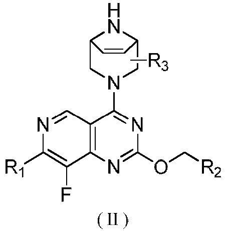 wherein
R₁ is selected from phenyl, pyridyl, and naphthyl, and the phenyl, pyridyl, and naphthyl are each independently and optionally substituted by 1, 2, 3, or 4 Rₐ;
R₂ is selected from 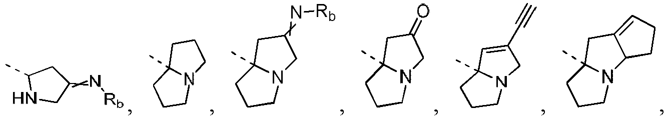 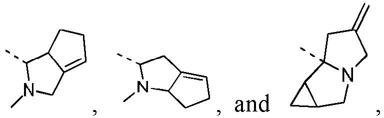 and the 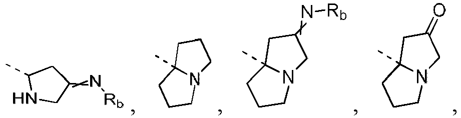 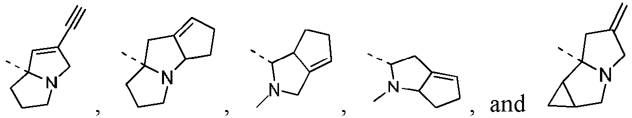 are each independently and optionally substituted by 1, 2, or 3 R_{c};
R₃ is selected from H and D;
each Rₐ is independently selected from F, Cl, Br, I, OH, NH₂, C₁₋₃ alkyl, C₁₋₃ alkoxy, C₂₋₄ alkenyl, C₂₋₄ alkynyl, -C₁₋₃ alkyl-cyclopropyl, and cyclopropyl, and the C₁₋₃ alkyl, C₁₋₃ alkoxy, C₂₋₄ alkenyl, C₂₋₄ alkynyl, cyclopropyl, and -C₁₋₃ alkyl-cyclopropyl are each independently and optionally substituted by 1, 2, or 3 R;
R_{b} is selected from H, CN, CH₃, and OCH₃;
each R_{c} is independently selected from F, Cl, Br, I, CH₂F, CHF₂, CF₃, and CH₂CF₃;
each R is independently selected from F, Cl, Br, I, CH₂F, CHF₂, and CF₃.

In some embodiments of the present disclosure, the compound or the pharmaceutically acceptable salt thereof is provided, and the compound is selected from: 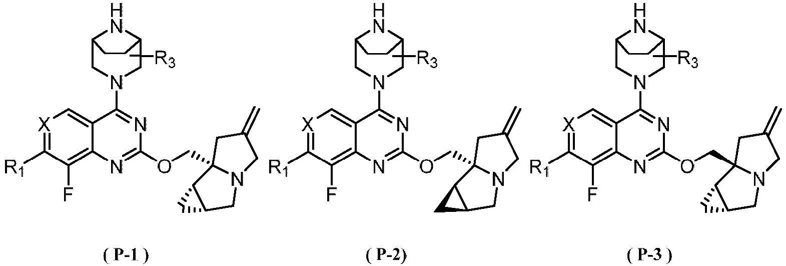 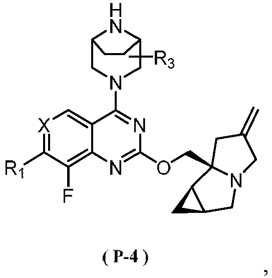 wherein X, R₁, and R₃ are as defined in the present disclosure.

In some embodiments of the present disclosure, each Rₐ is independently selected from F, Cl, OH, NH₂, CH₃, CH₂CH₃, CH(CH₃)₂, OCH₃, OCH₂CH₃, OCH(CH₃)₂, 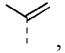 -C≡CH, -C≡CCH₃, 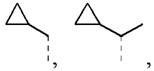 and cyclopropyl, and the CH₃, CH₂CH₃, CH(CH₃)₂, OCH₃, OCH₂CH₃, OCH(CH₃)₂, 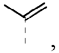 -C≡CH, -C≡CCH₃, 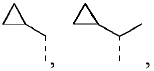 and cyclopropyl are each independently and optionally substituted by 1, 2, or 3 R, and other variables are as defined in the present disclosure.

In some embodiments of the present disclosure, each Rₐ is independently selected from F, Cl, OH, NH₂, CH₃, CHF₂, CF₃, CH₂CF₃, CH(CH₃)CF₃, OCH₃, OCF₃, 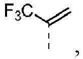 -C≡CH, -C≡CCH₃, 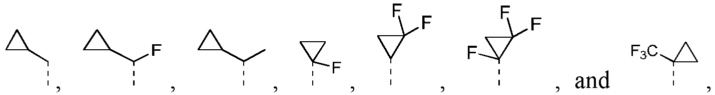 and other variables are as defined in the present disclosure.

In some embodiments of the present disclosure, the R₁ is selected from 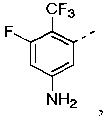 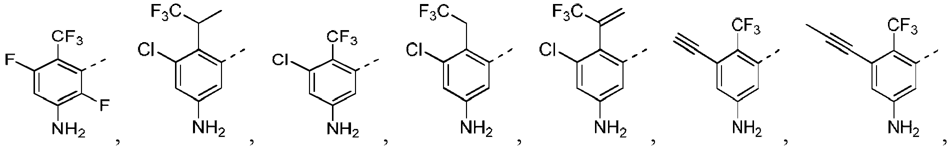 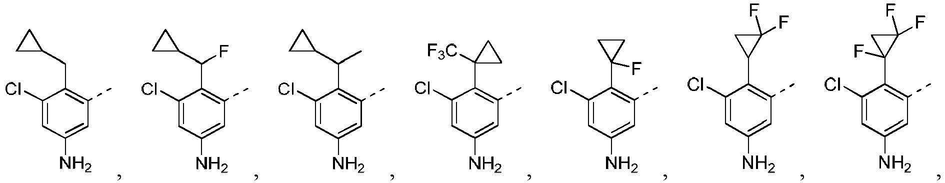 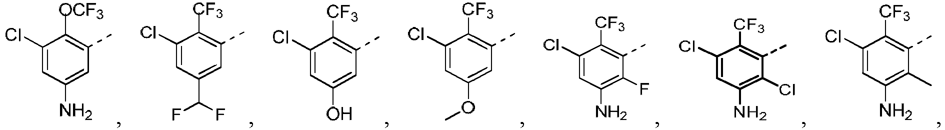 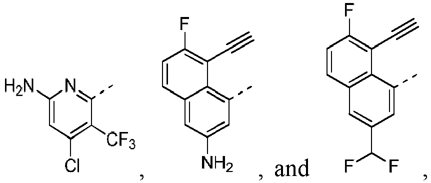 and other variables are as defined in the present disclosure.

In some embodiments of the present disclosure, the R₁ is selected from 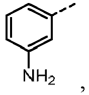 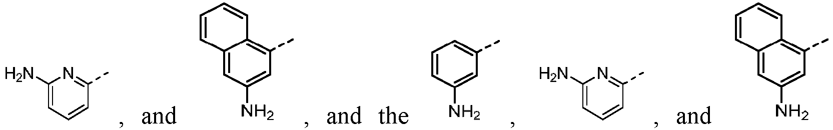 are each independently and optionally substituted by 1, 2, 3, or 4 Rₐ, and other variables are as defined in the present disclosure.

In some embodiments of the present disclosure, the R₁ is selected from 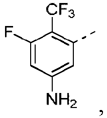 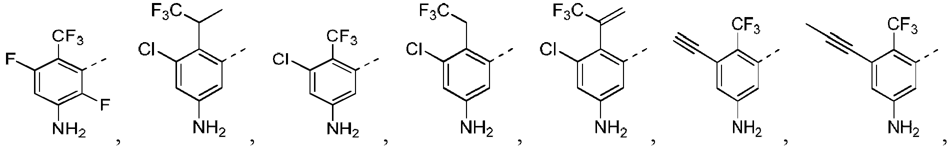 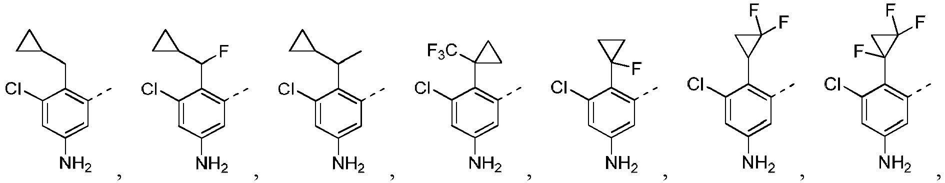 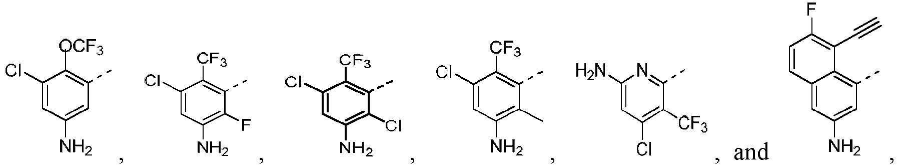 and other variables are as defined in the present disclosure.

In some embodiments of the present disclosure, the R₁ is selected from 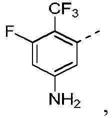 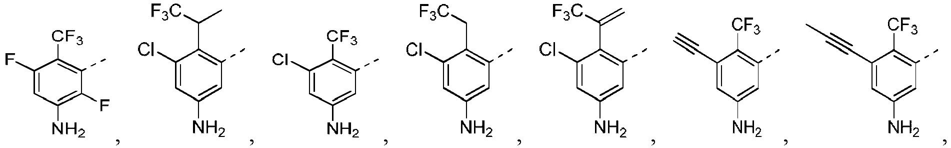 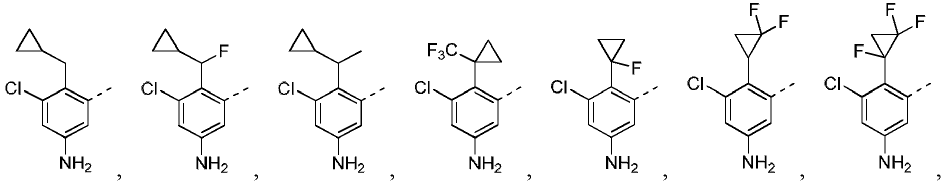 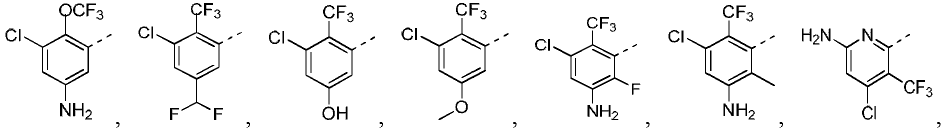 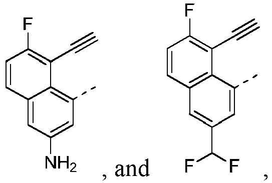 and other variables are as defined in the present disclosure.

In some embodiments of the present disclosure, the R₁ is selected from 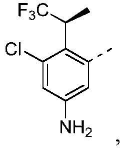 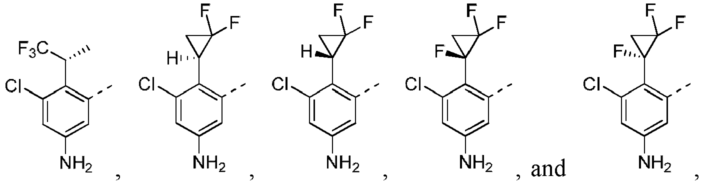 and other variables are as defined in the present disclosure.

In some embodiments of the present disclosure, the R₂ is selected from 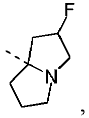 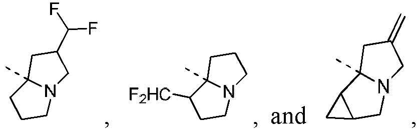 and other variables are as defined in the present disclosure.

In some embodiments of the present disclosure, the R₂ is selected from 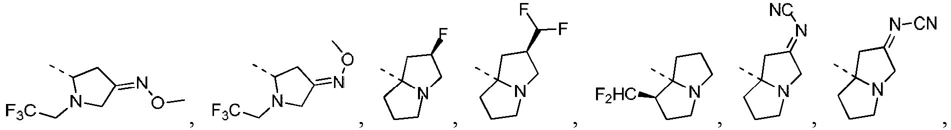 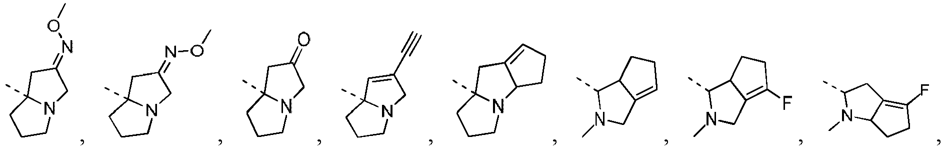 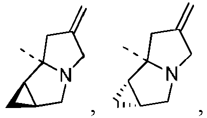 and other variables are as defined in the present disclosure.

In some embodiments of the present disclosure, the R₂ is selected from 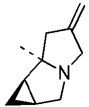 and 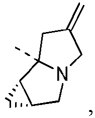 and other variables are as defined in the present disclosure.

In some embodiments of the present disclosure, the R₃ is selected from H and D, and other variables are as defined in the present disclosure.

In some embodiments of the present disclosure, the X is selected from N, and other variables are as defined in the present disclosure.

In some embodiments of the present disclosure, the compound or the pharmaceutically acceptable salt thereof is provided, and the compound is selected from: 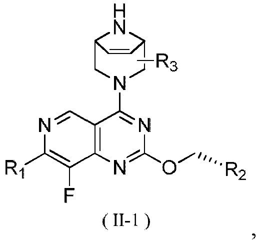 wherein R₁, R₂, and R₃ are as defined in the present disclosure.

In some embodiments of the present disclosure, the compound or the pharmaceutically acceptable salt thereof is provided, and the compound is selected from: 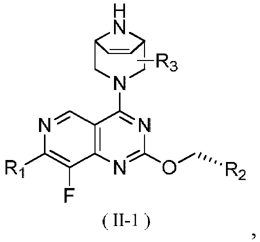 wherein R₁, R₂, and R₃ are as defined in the present disclosure;

Provided that R₂ is not

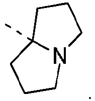

In some embodiments of the present disclosure, the compound or the pharmaceutically acceptable salt thereof is provided, and the compound is selected from: 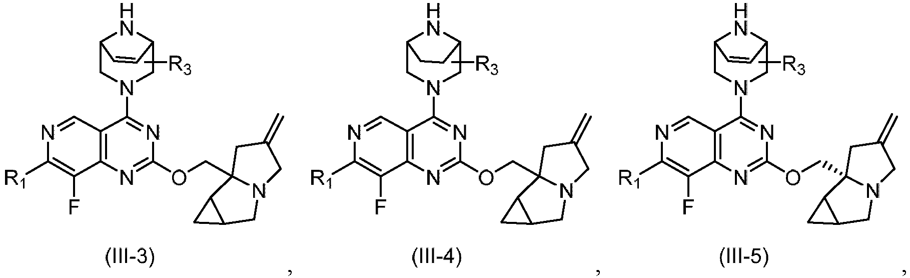 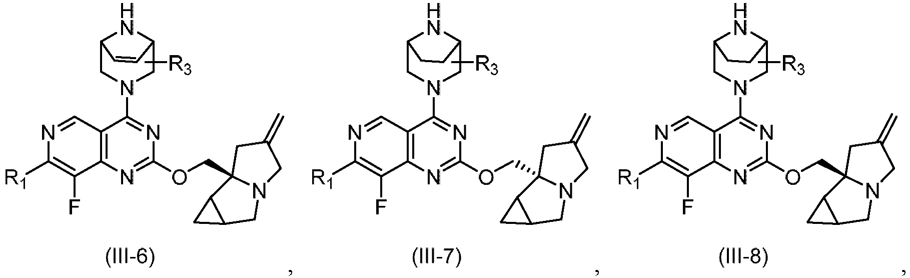 wherein R₁ and R₃ are as defined in the present disclosure.

In some embodiments of the present disclosure, the compound or the pharmaceutically acceptable salt thereof is provided, and the compound is selected from: 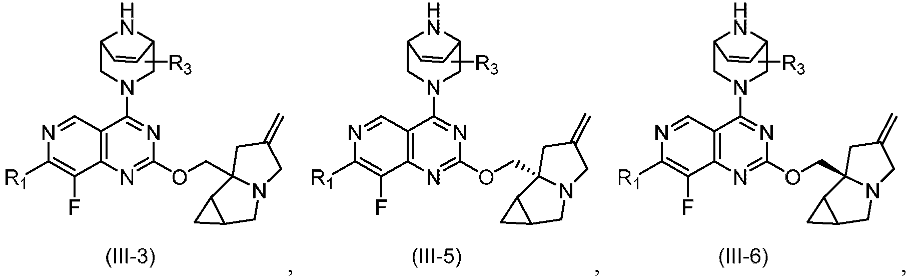 wherein R₁ and R₃ are as defined in the present disclosure.

In some embodiments of the present disclosure, the compound or the pharmaceutically acceptable salt thereof is provided, and the compound is selected from: 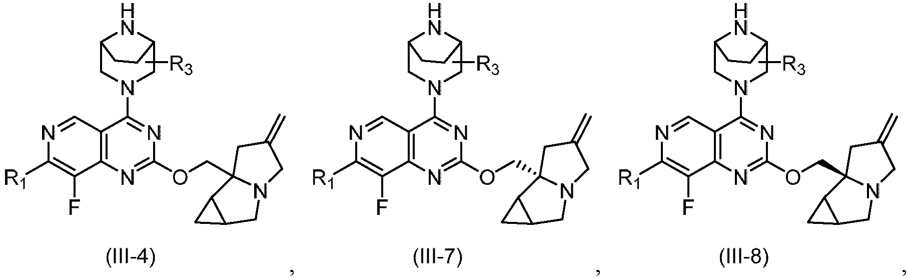 wherein R₁ and R₃ are as defined in the present disclosure.

In Examples 2 and 3 of the present disclosure, compound 3 is prepared from compound 2-4A through the steps described in Examples 2 and 3, wherein after developing twice by TLC thin-layer chromatography (petroleum ether: acetone = 5:1), the product corresponding to the smaller Rf value is compound 2-4A, and the product corresponding to the larger Rf value is its isomer.

In Examples 2 and 3 of the present disclosure, compound 3 is prepared from compound **2-4A** through the steps described in Examples 2 and 3, wherein after developing twice by TLC thin-layer chromatography (petroleum ether: acetone = 5:1), the Rf value of compound **2-4A** is 0.5, and the Rf value of its isomer is 0.55.

In Examples 2 and 3 of the present disclosure, compound **3** is prepared from compound **2-4A** through the steps described in Examples 2 and 3, wherein LCMS (chromatographic column: Agilent Poroshell 120 EC-C18 2.7 µm 3.0 * 30 mm, mobile phase: A: water (0.037% formic acid) - B: acetonitrile (0.0187% formic acid); B: 5% to 95%) shows that the retention time of **2-4A** is 0.776 min, and the retention time of its isomer is 0.801 min.

In Example 3 of the present disclosure, compound **3** is prepared from compound **3-2A** through the steps described in Example 3, wherein **3-2A** is analyzed by SFC (chromatographic column: Cellulose 2 100 mm * 4.6 mm, 3 µm, mobile phase: [A (supercritical carbon dioxide), B (methanol (0.05% diethanolamine))]; B: 40%) showing that the retention time is 4.964 min, the retention time of its enantiomer is 8.382 min.

In Example 3 of the present disclosure, compound **3** is analyzed by SFC (chromatographic column: Chiralcel OJ-3 100 * 4.6 mm, 3 µm; mobile phase: A (supercritical carbon dioxide) and B (ethanol, containing 0.05% diethylamine); gradient: B%:40% to 40%) showing a retention time of 3.761 min.

The present disclosure further provides a compound as shown below or a pharmaceutically acceptable salt thereof, 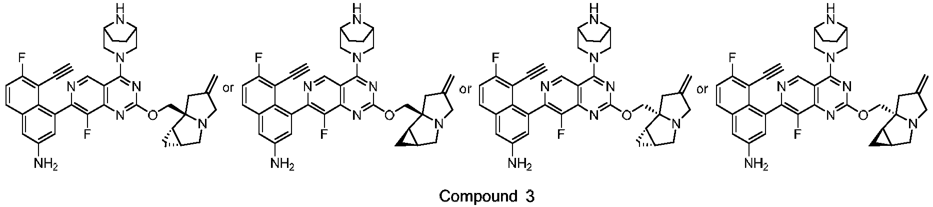 wherein the compound 3 is analyzed by SFC (chromatographic column: Chiralcel OJ-3 100 * 4.6 mm, 3 µm; mobile phase: A (supercritical carbon dioxide) and B (ethanol, containing 0.05% diethylamine); gradient: B%: 40% to 40%) showing a retention time of 3.761 min.

The present disclosure further provides a compound as shown below or a pharmaceutically acceptable salt thereof, 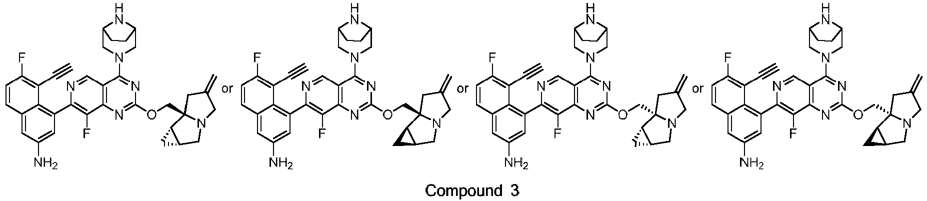 wherein the compound 3 is analyzed by chiral HPLC (chromatographic column: FLM Chiral NQ, 150 * 4.6 mm, 3 µm; mobile phase: A: (n-hexane) and B: (ethanol, containing 0.2% diethylamine, v/v); gradient: B%: isocratic 30%; elution time: 60 min; column temperature: 35°C) showing a retention time of 17.387 min.

In some embodiments of the present disclosure, the three stereoisomers of compound **3** are analyzed by chiral HPLC (chromatographic column: FLM Chiral NQ, 150 * 4.6 mm, 3 µm; mobile phase: A: (n-hexane) and B (ethanol, containing 0.2% diethylamine, v/v); gradient: B%: isocratic 30%; elution time: 60 min; column temperature: 35°C) showing retention times of 22.587 min, 26.205 min, and 44.765 min.

In a technical embodiment of the present disclosure, the present disclosure provides compound **7A2** or a pharmaceutically acceptable salt thereof; the compound **7A2** has one of the following chemical structures, which is analyzed by SFC (column: ChiralPak IG-3, 50 * 4.6 mm, 3 µm; mobile phase: A (supercritical CO₂) and B (isopropanol, containing 0.1% isopropylamine); gradient: B%: 5% to 5%, 3 min) with a retention time of 2.236 min.

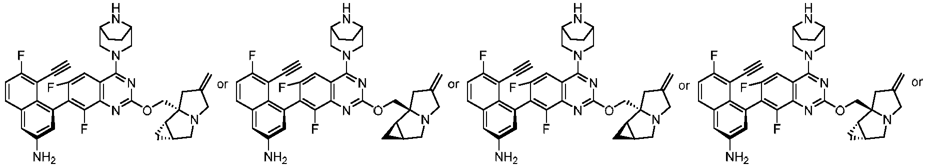

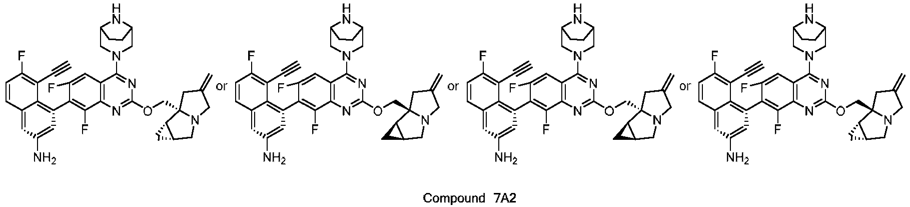

The present disclosure further provides a compound of the following formula or a pharmaceutically acceptable salt thereof, 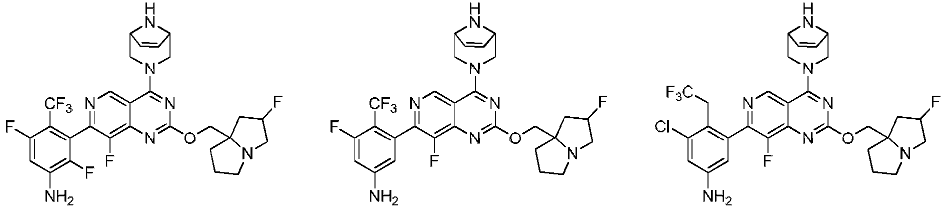 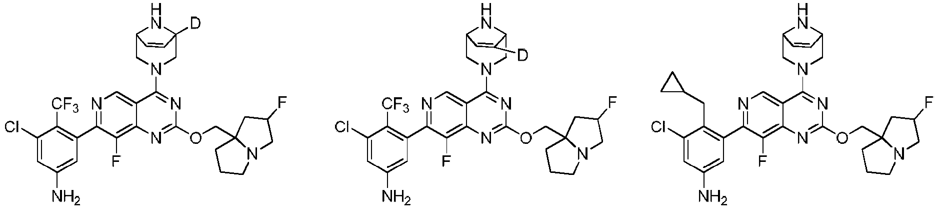 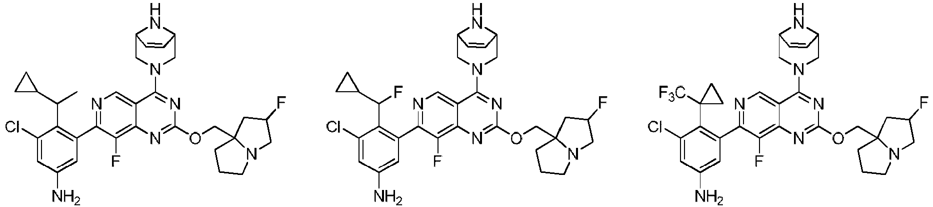 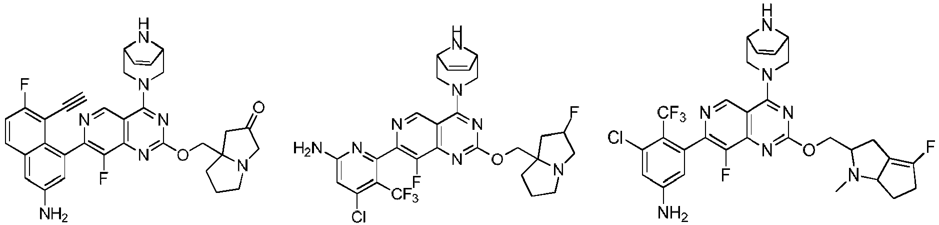 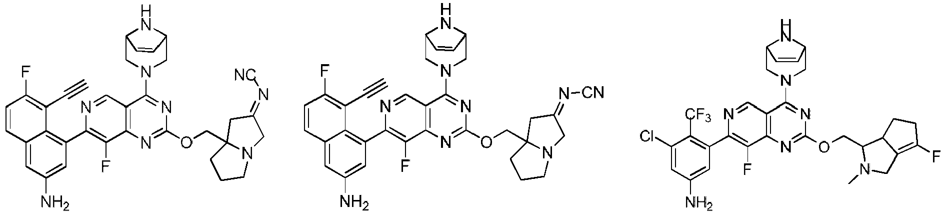 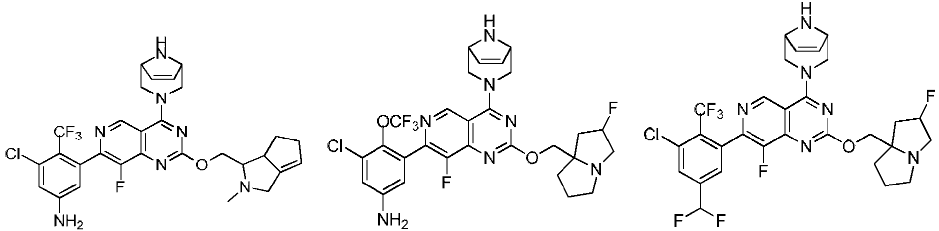 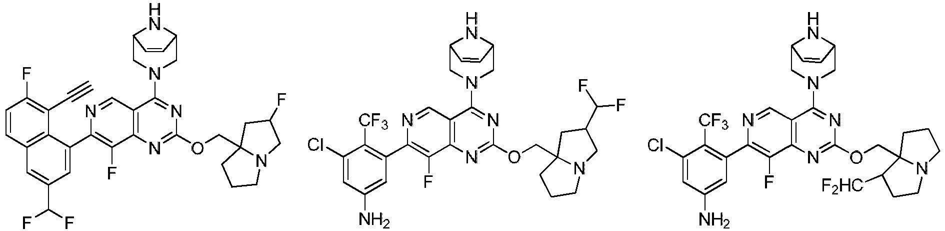 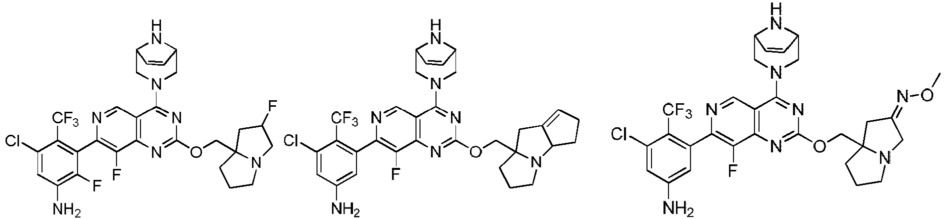 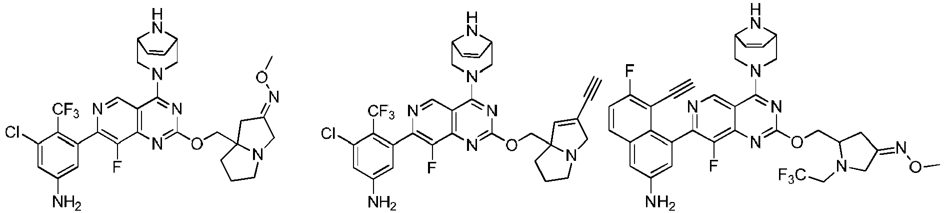 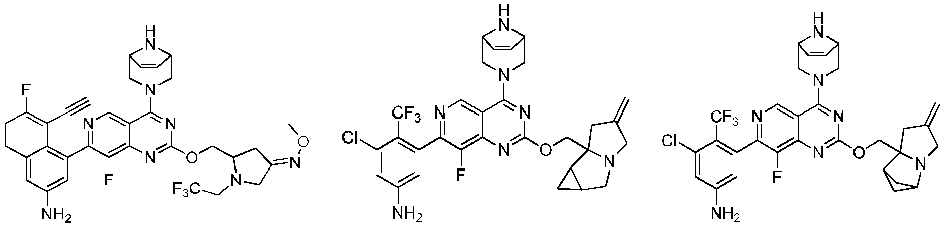 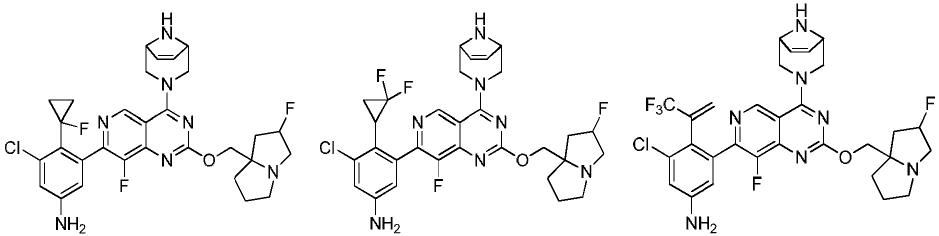 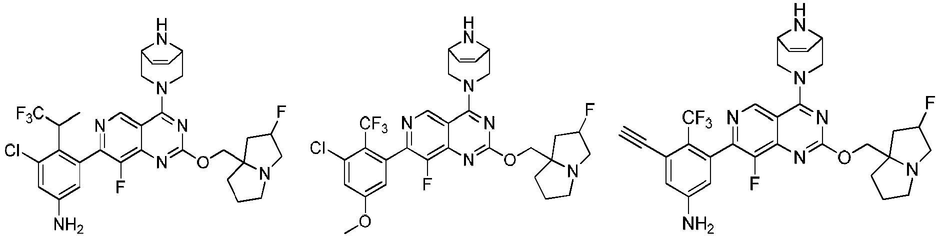 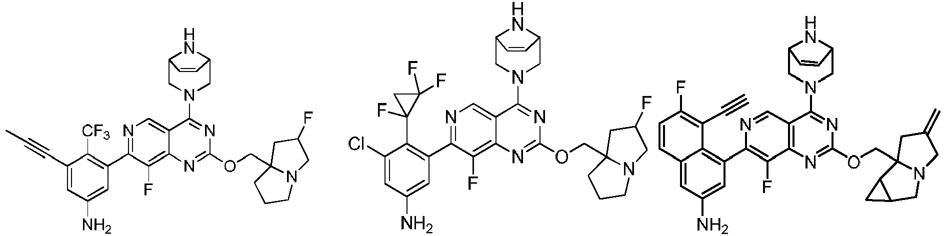 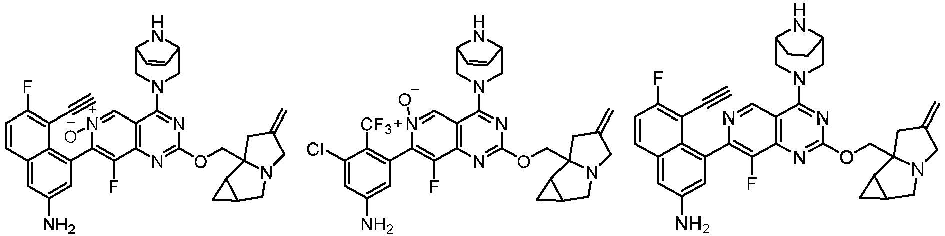 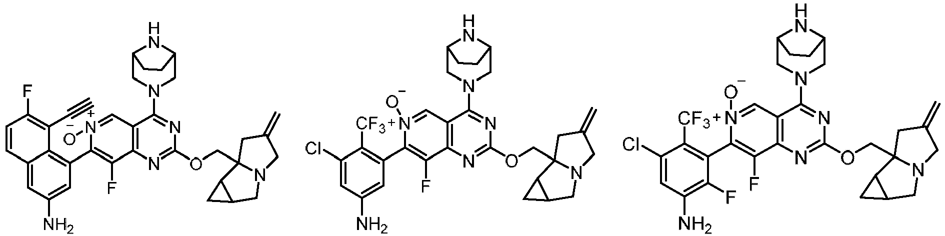 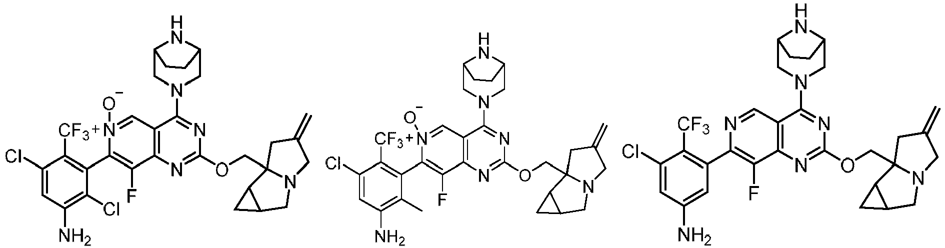 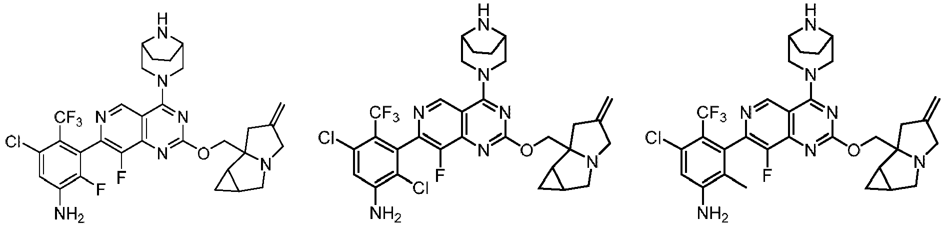 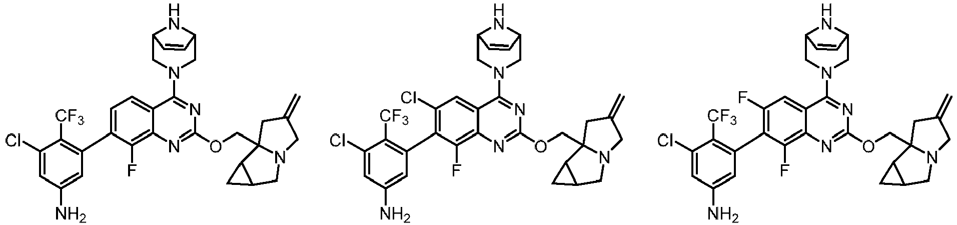 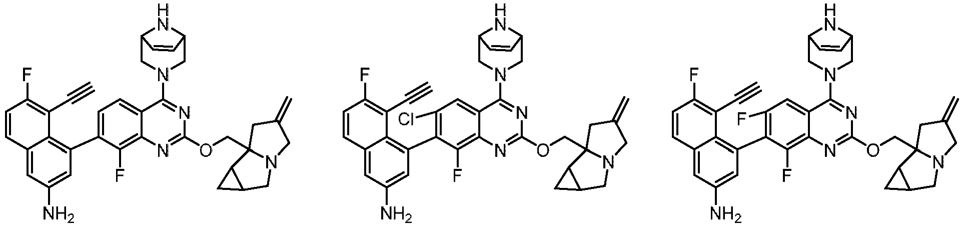 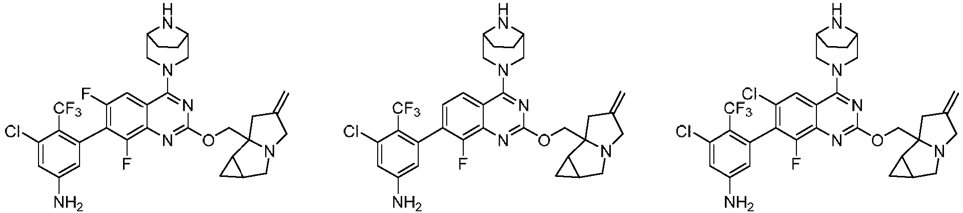 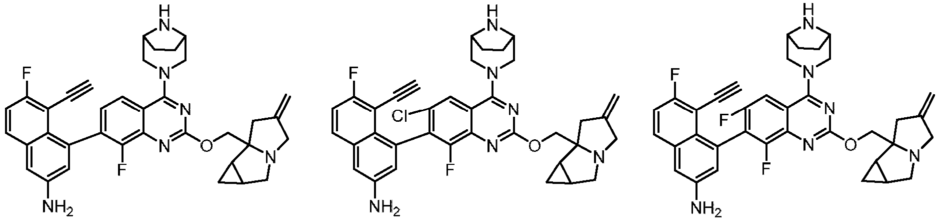 or

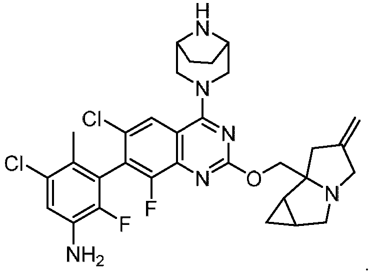

The present disclosure provides a compound of the following formula or a pharmaceutically acceptable salt thereof, 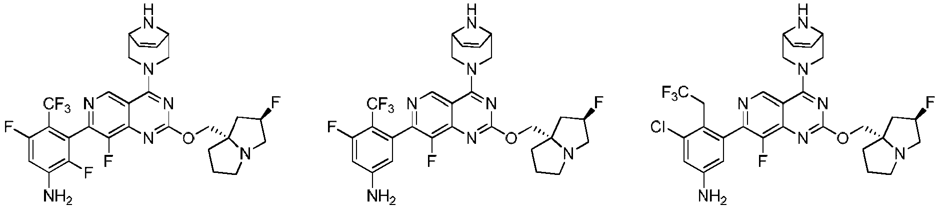 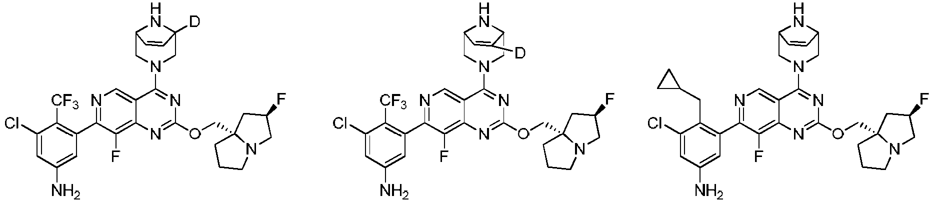 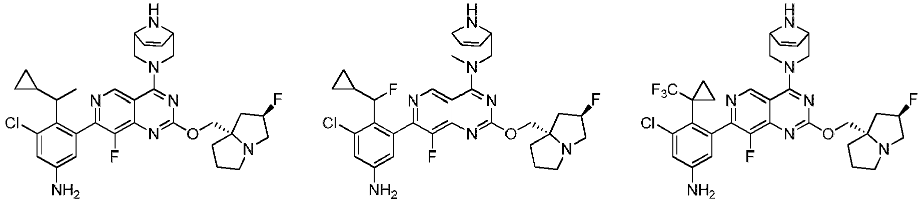 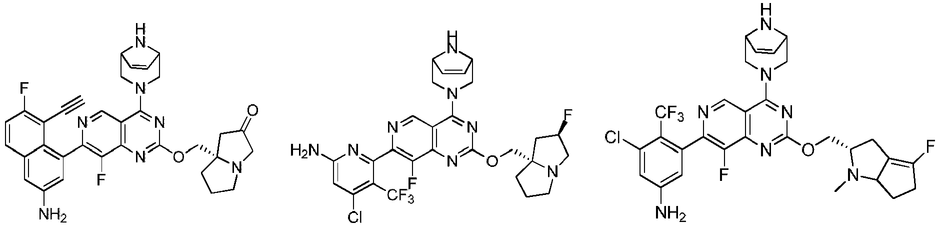 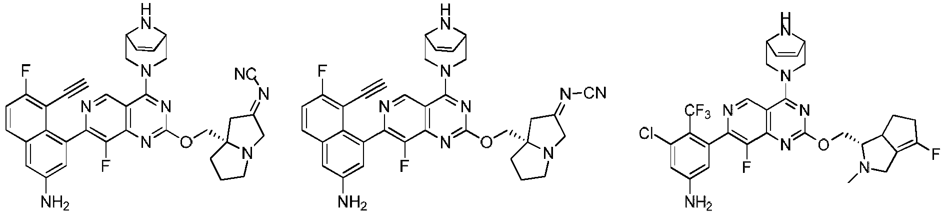 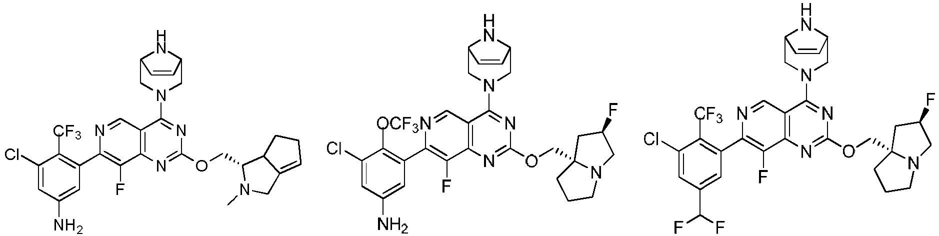 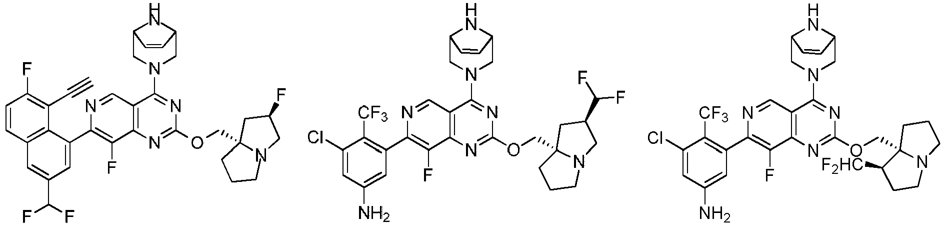 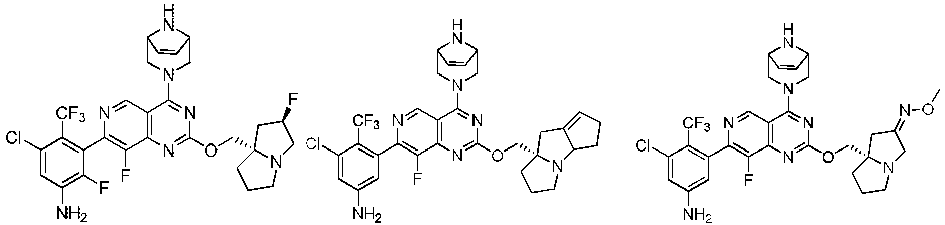 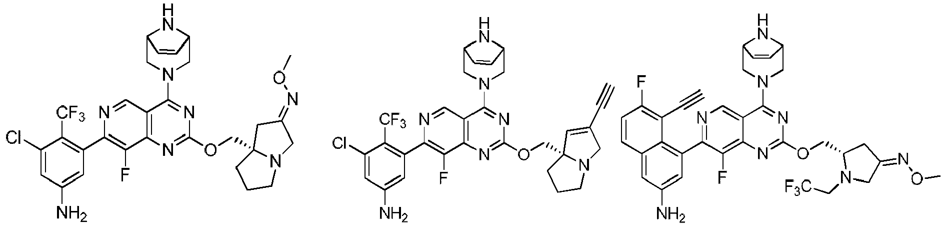 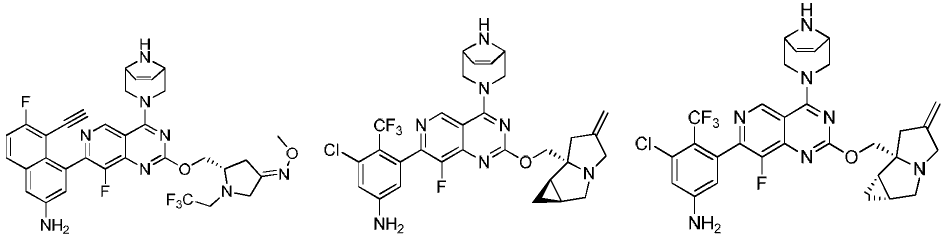 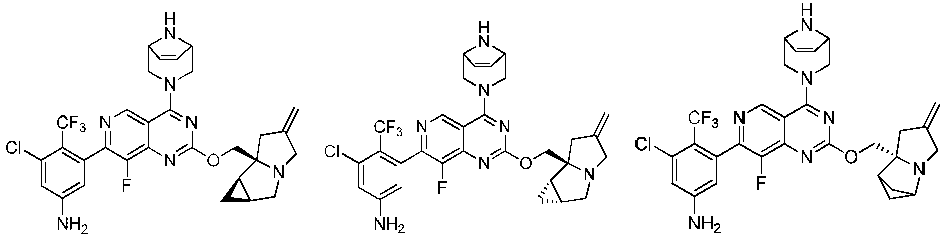 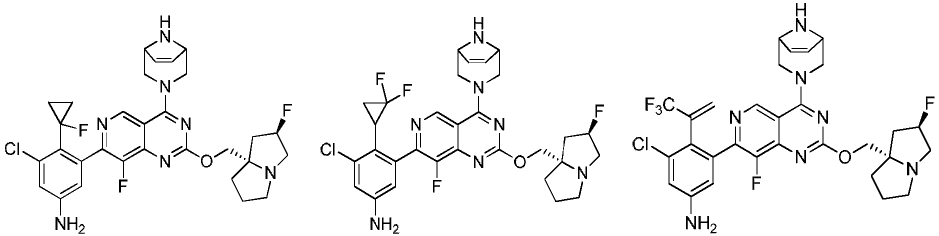 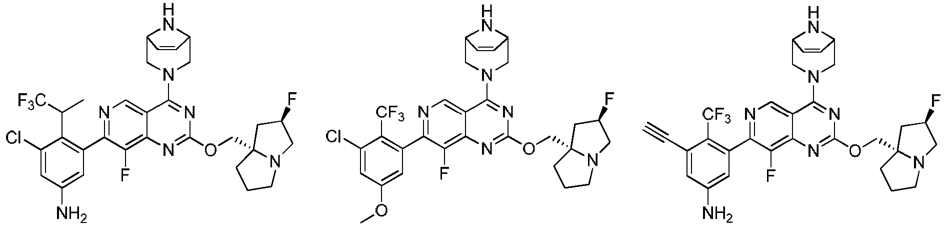 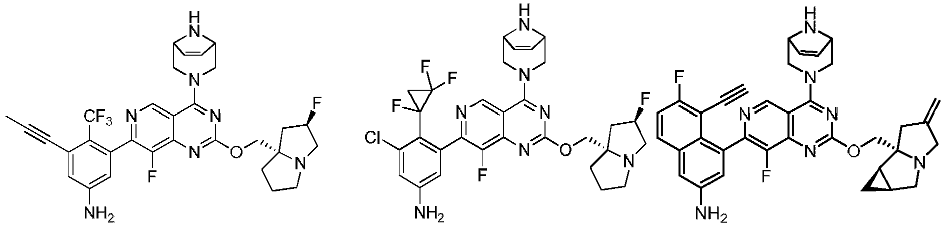 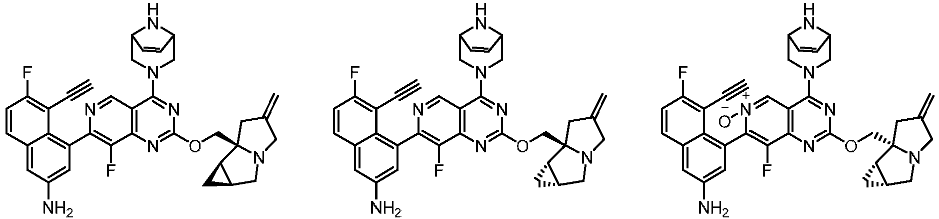 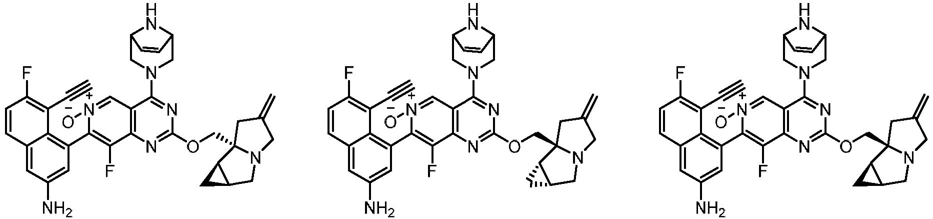 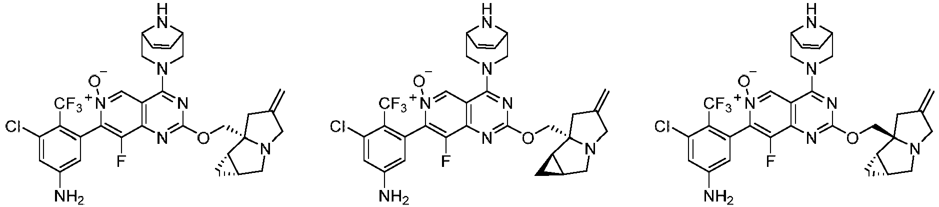 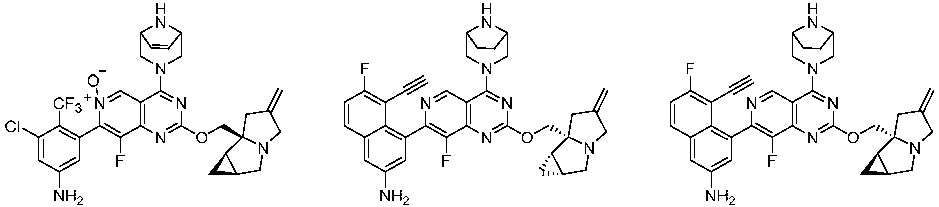 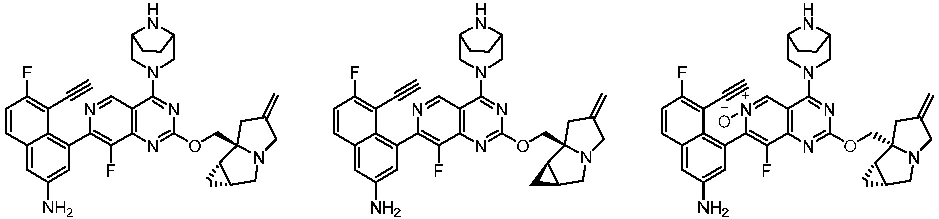 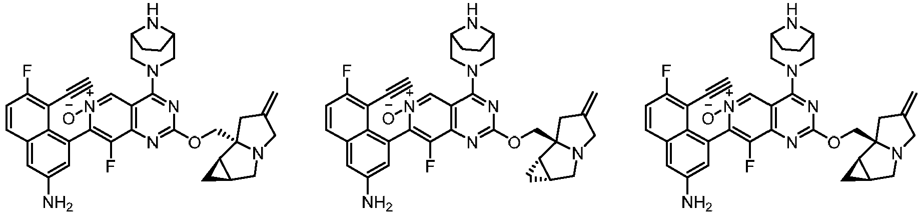 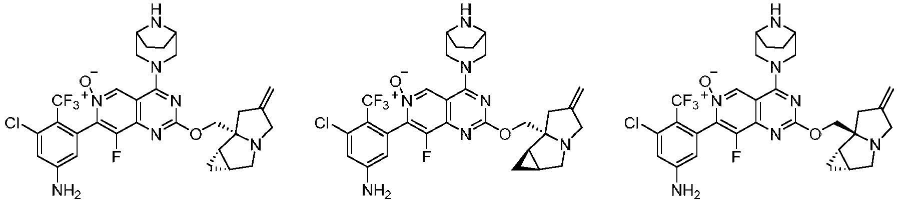 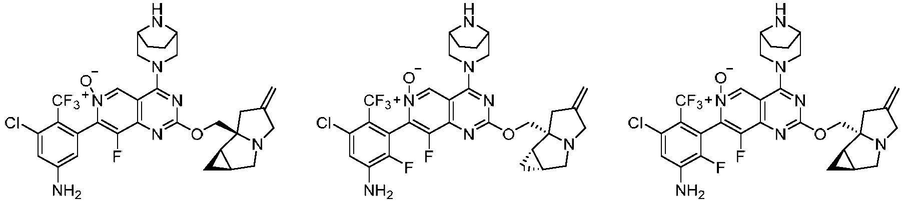 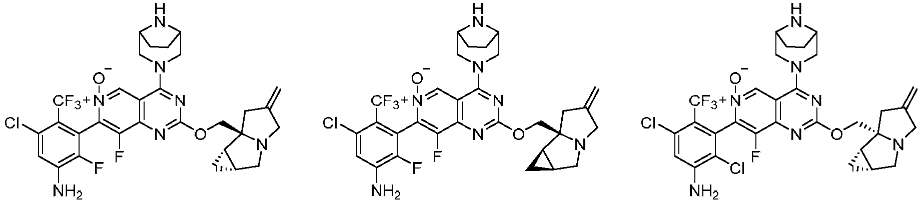 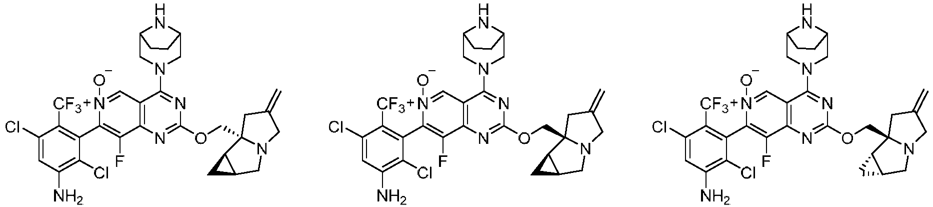 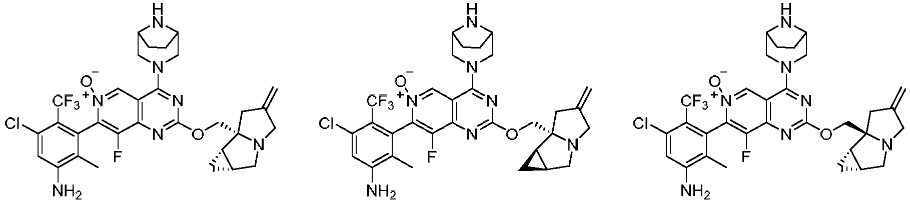 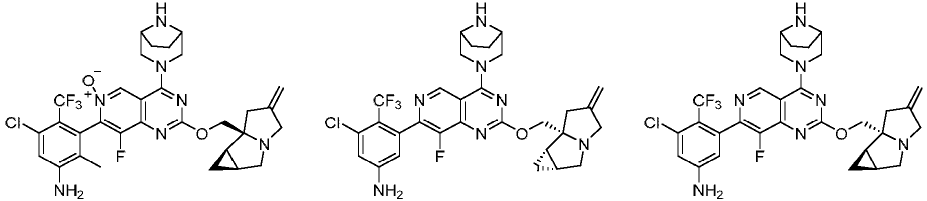 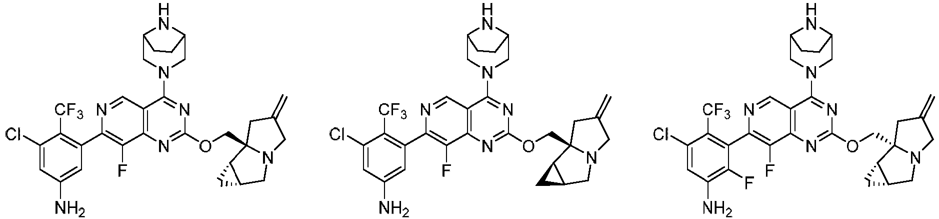 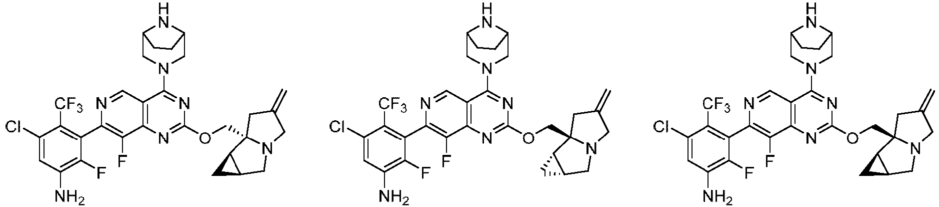 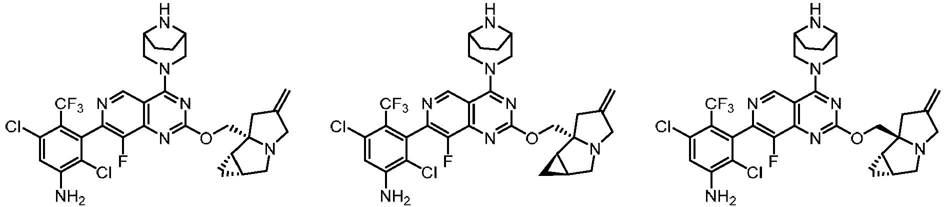 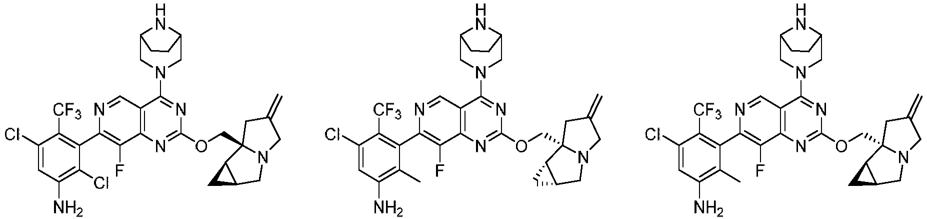 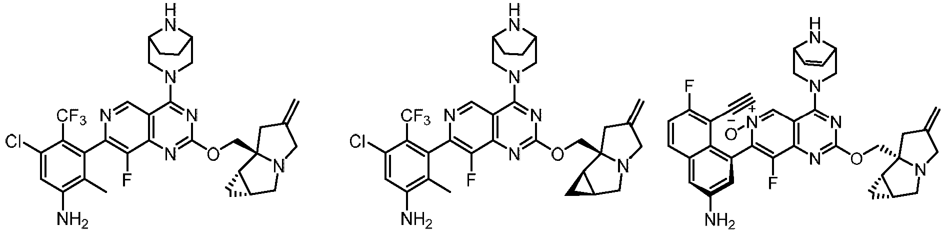 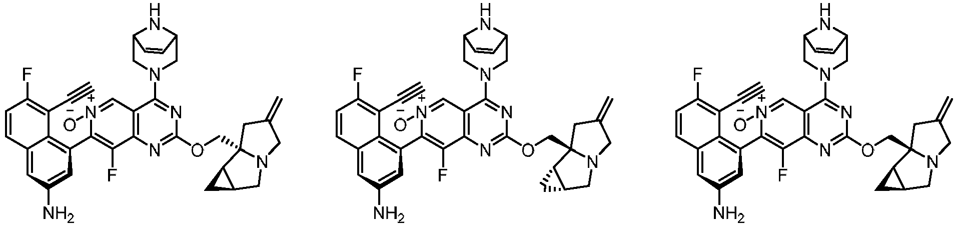 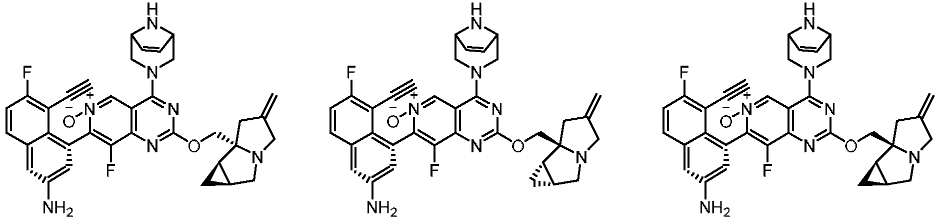 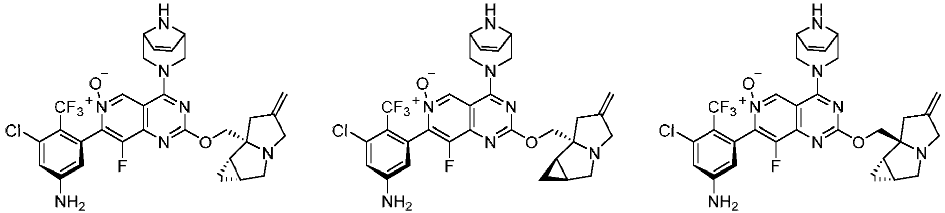 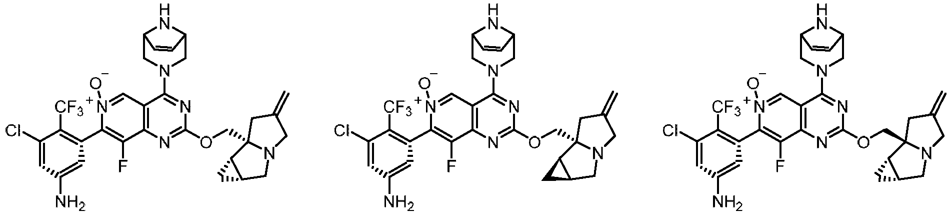 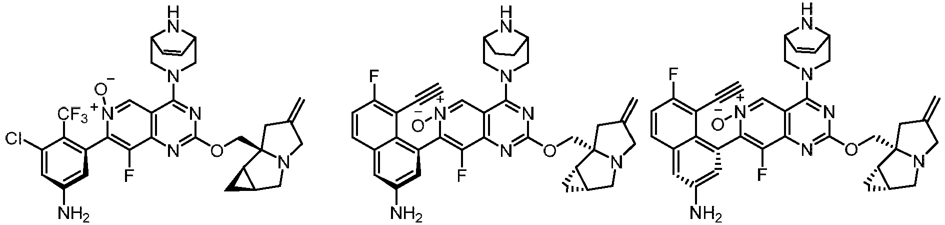 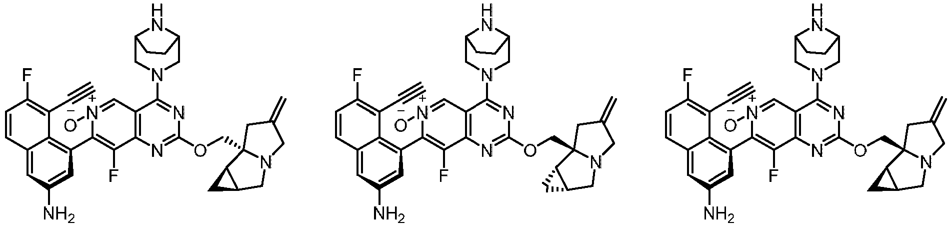 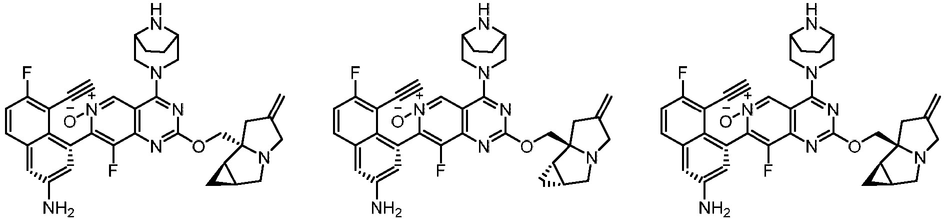 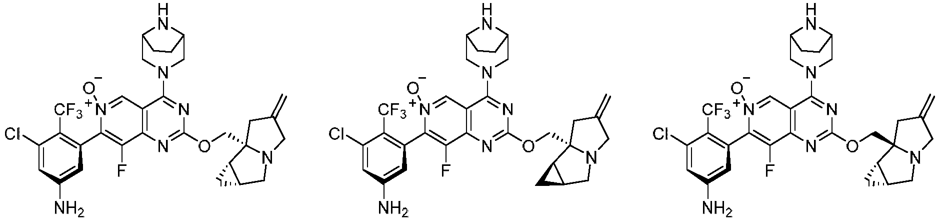 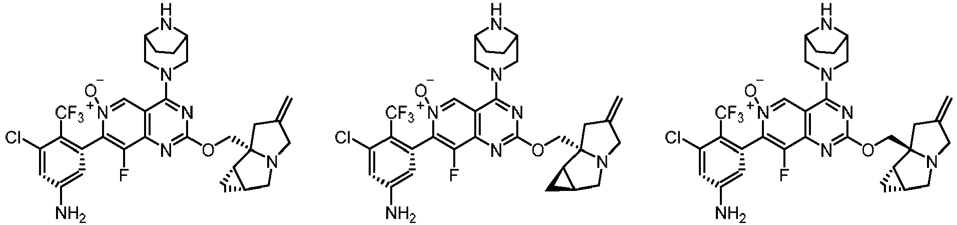 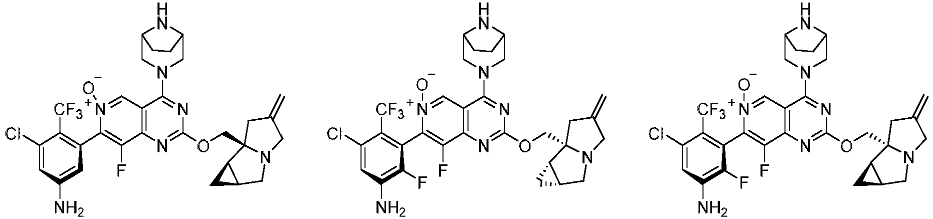 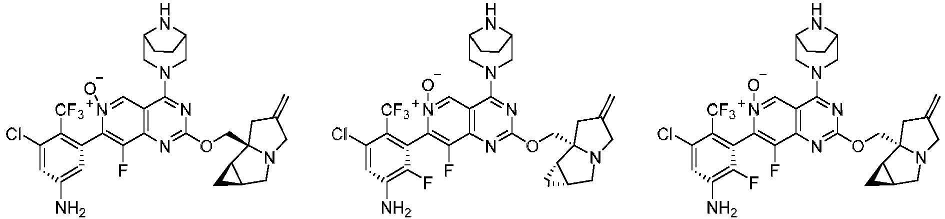 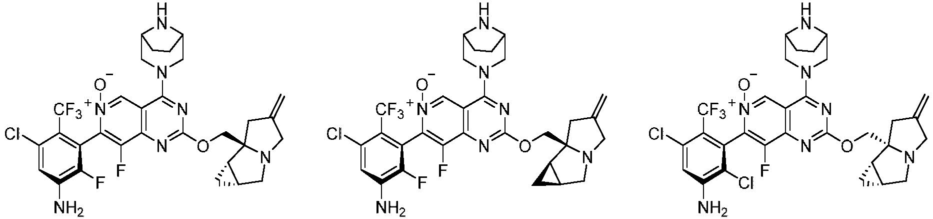 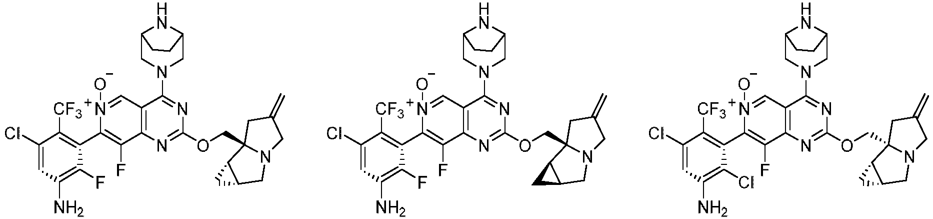 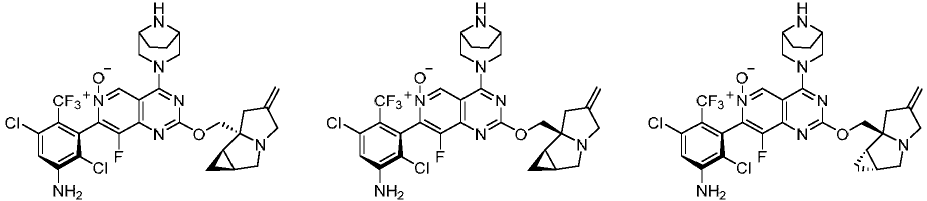 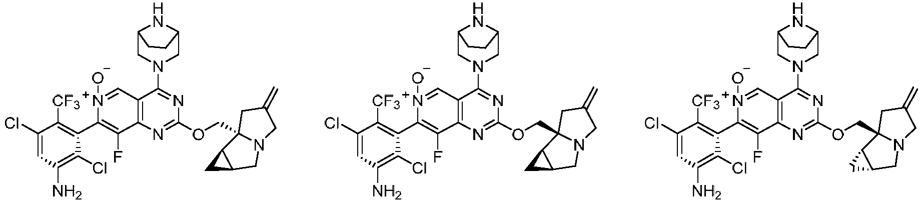 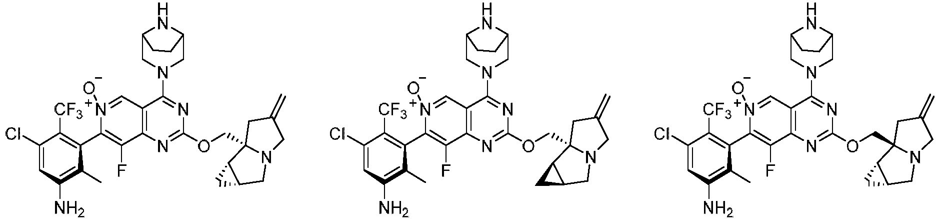 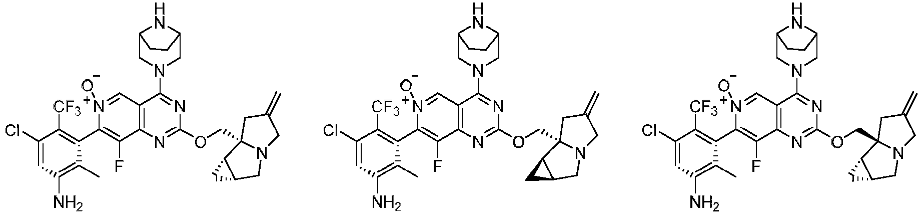 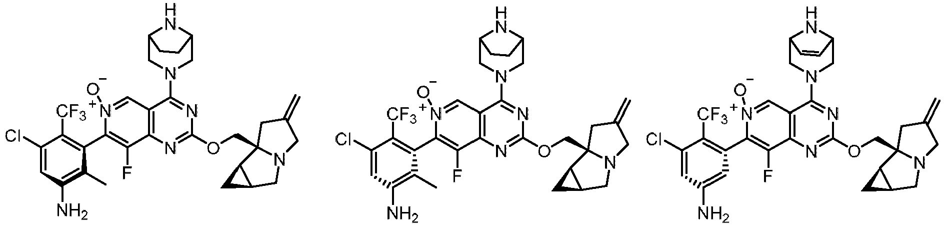 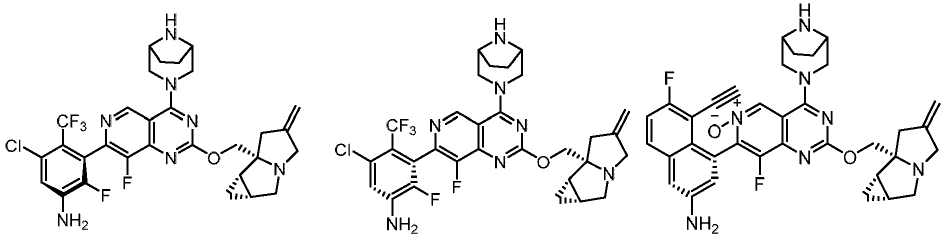 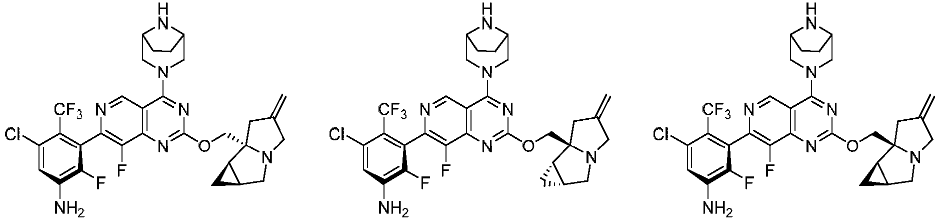 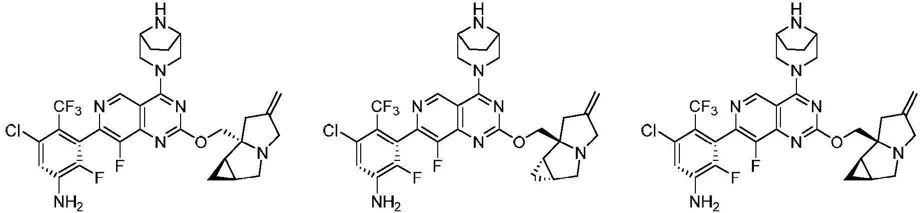 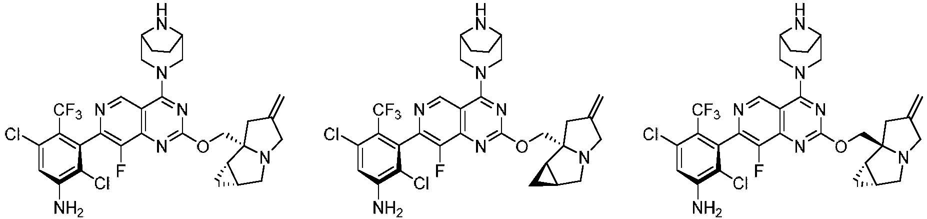 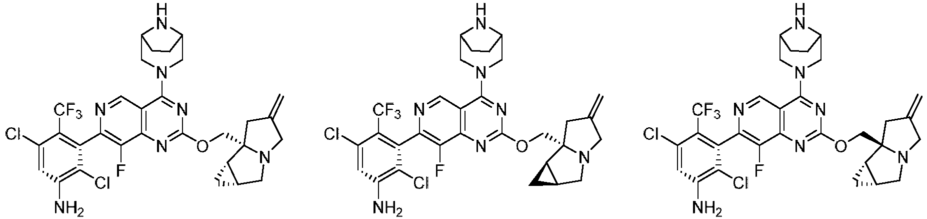 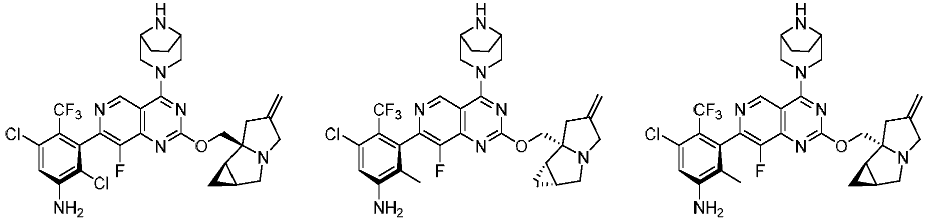 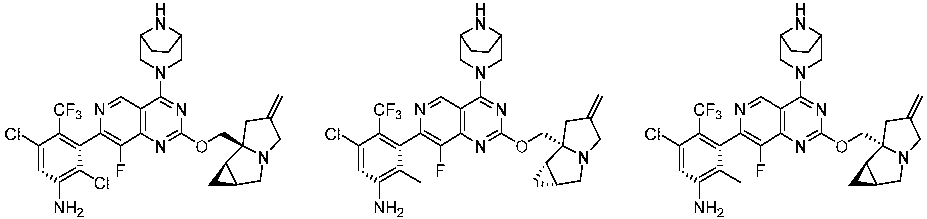 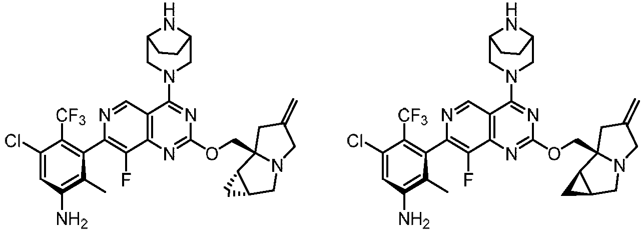 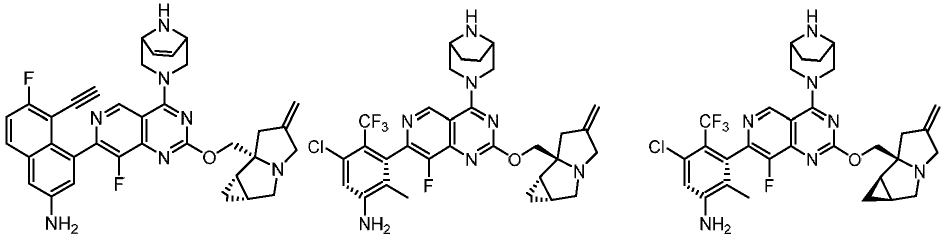 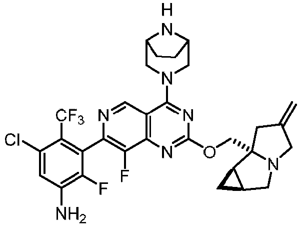 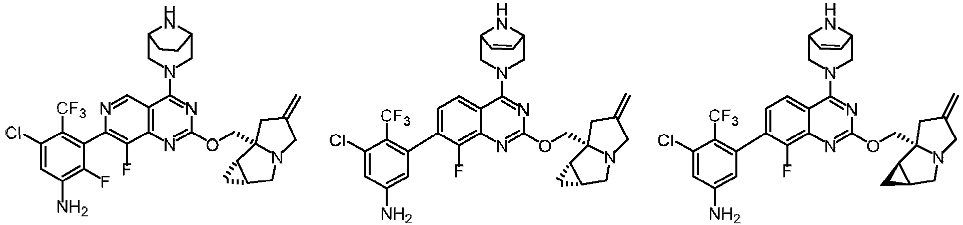 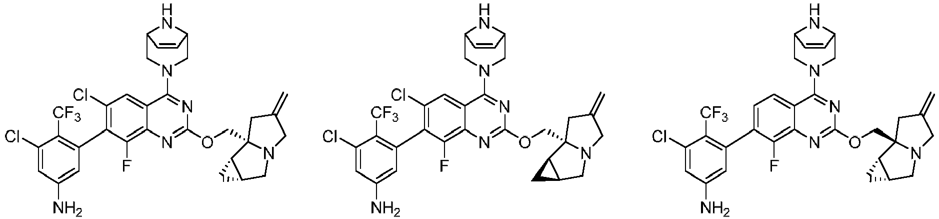 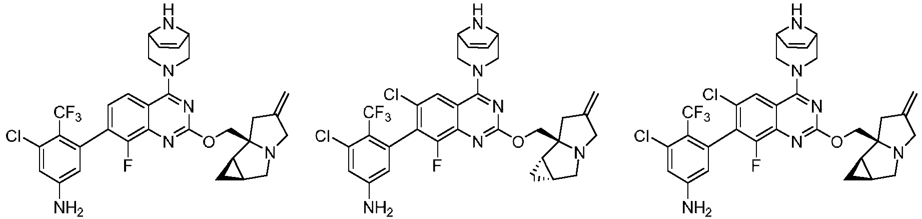 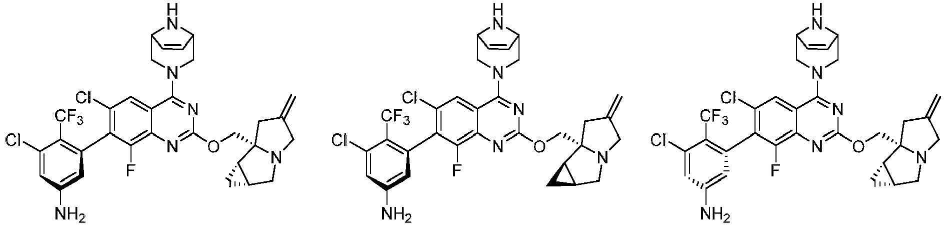 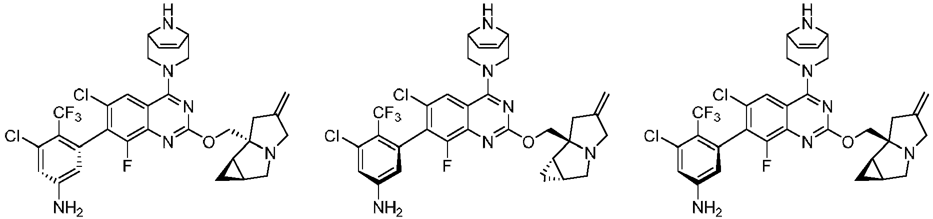 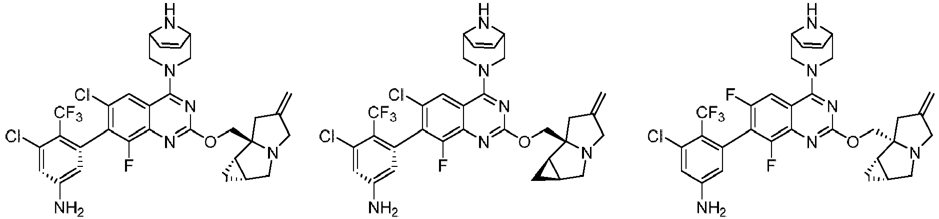 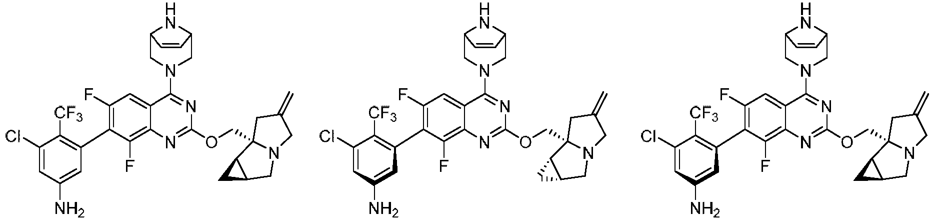 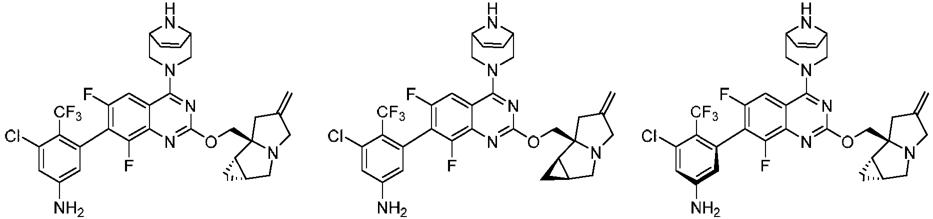 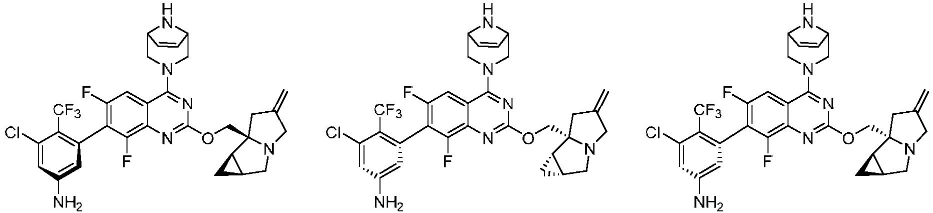 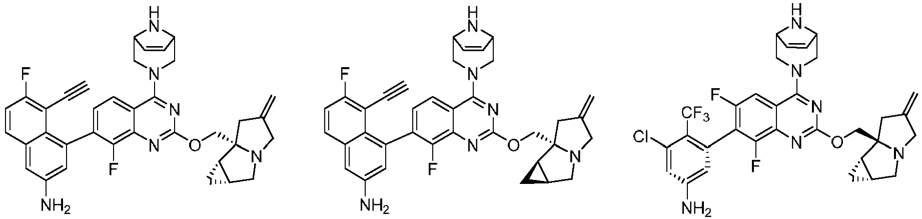 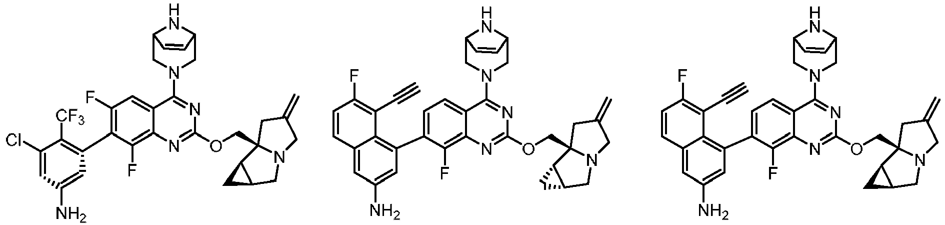 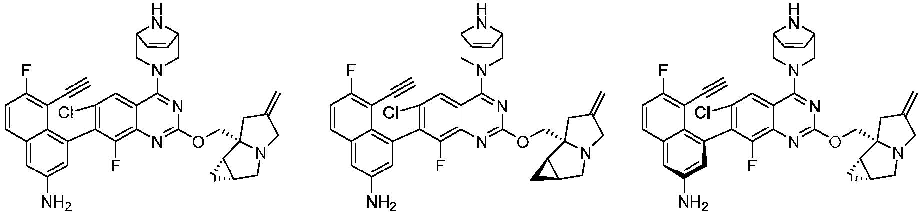 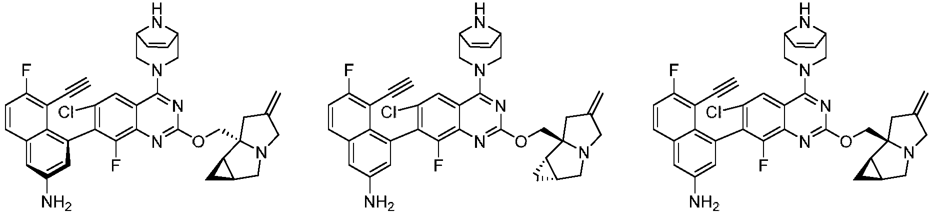 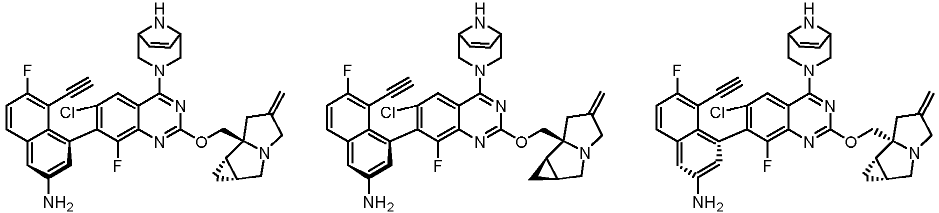 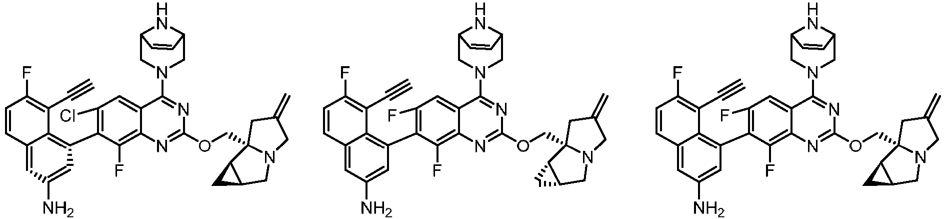 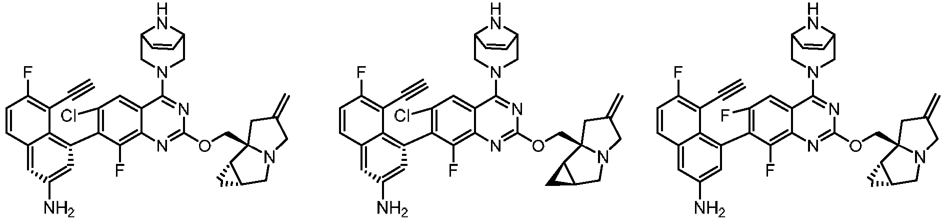 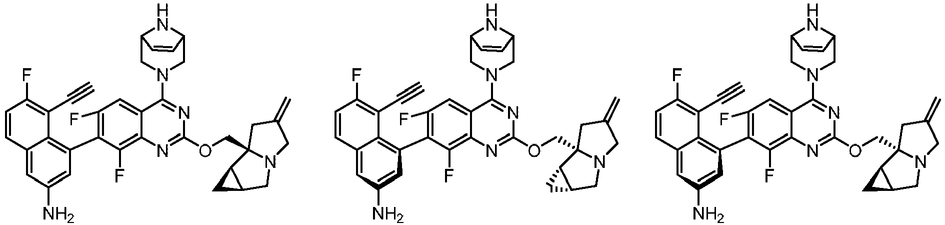 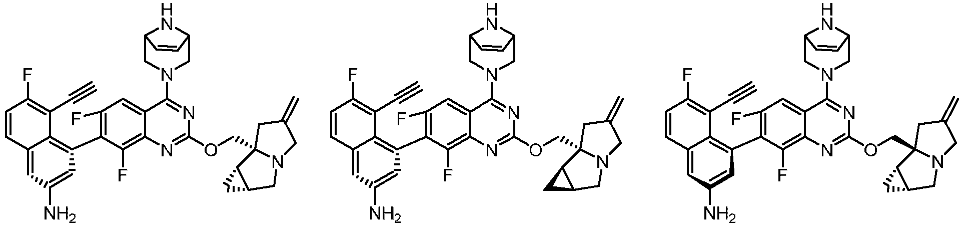 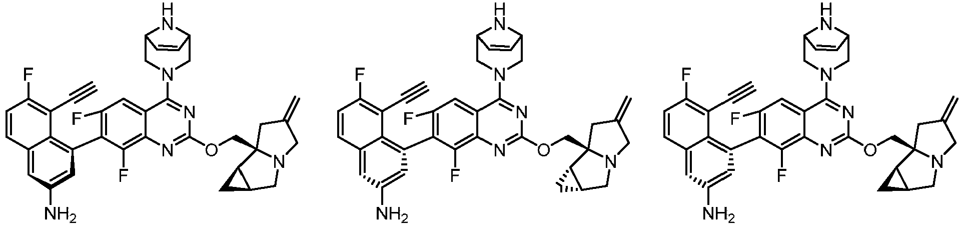 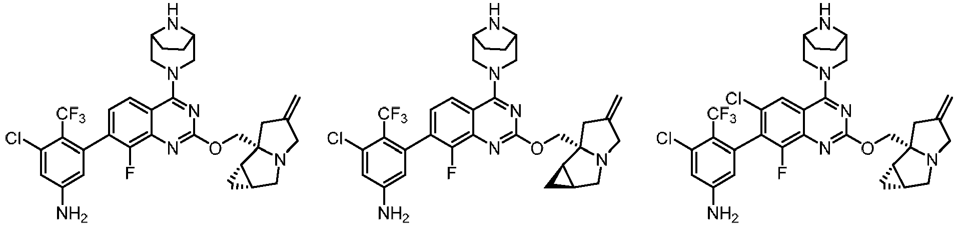 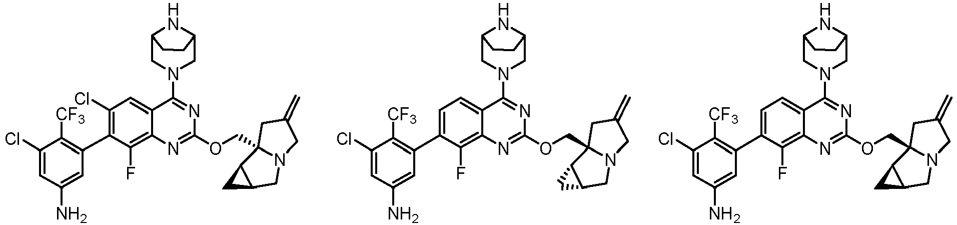 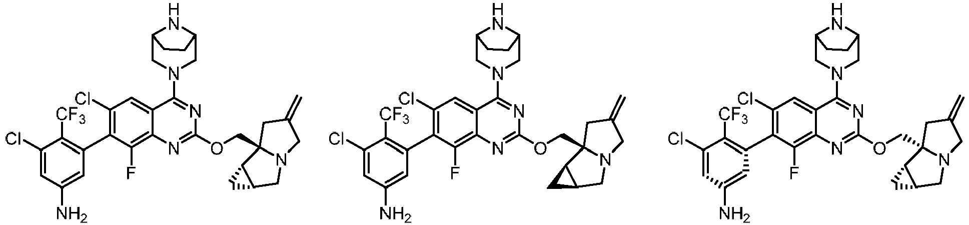 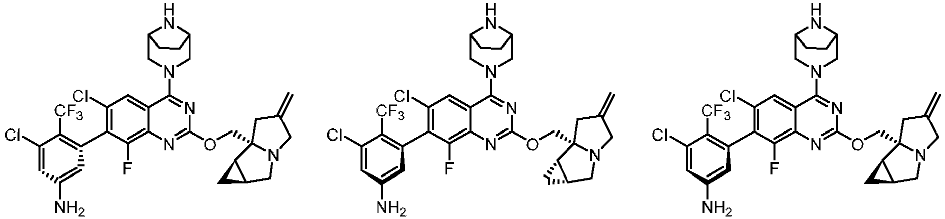 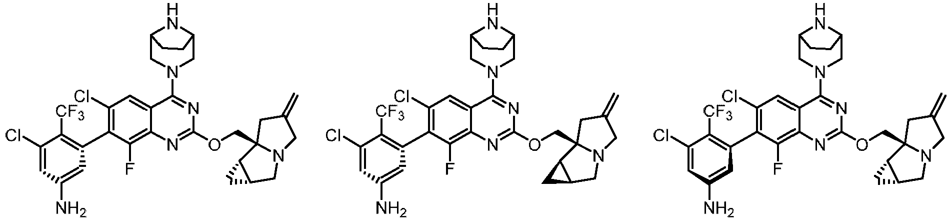 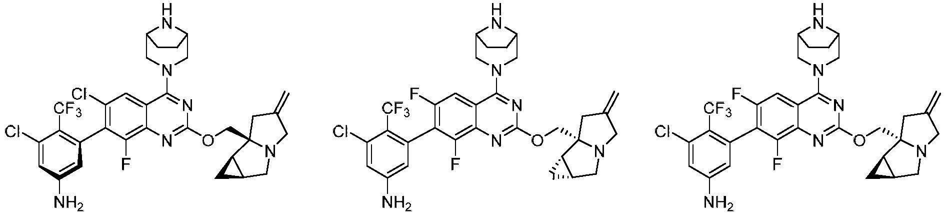 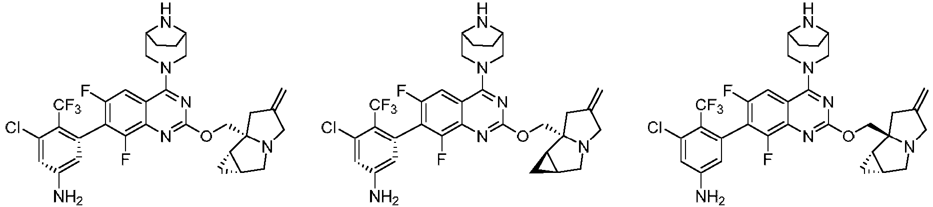 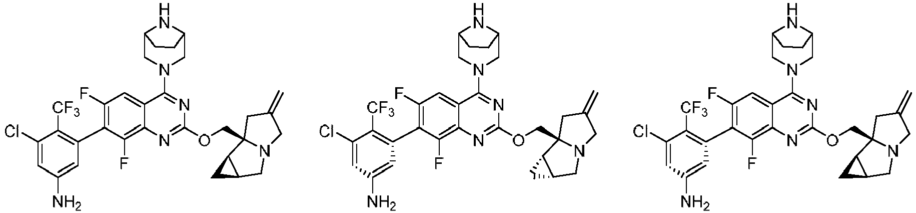 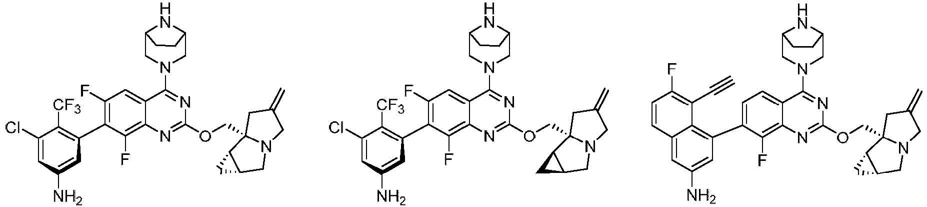 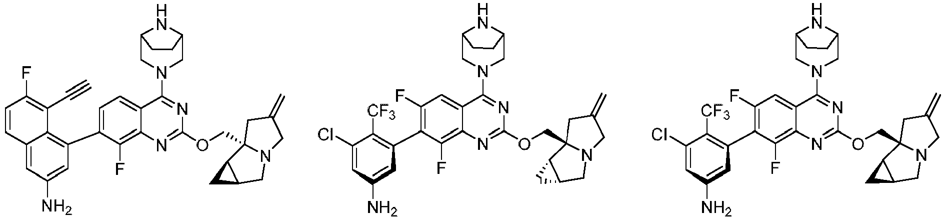 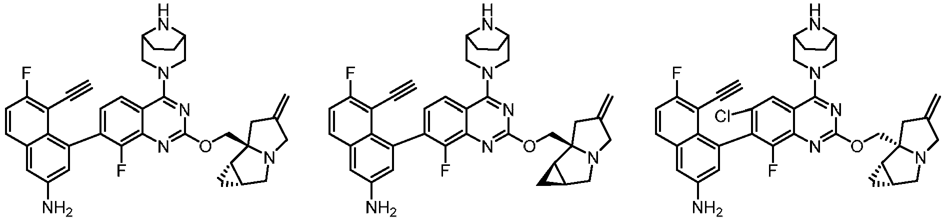 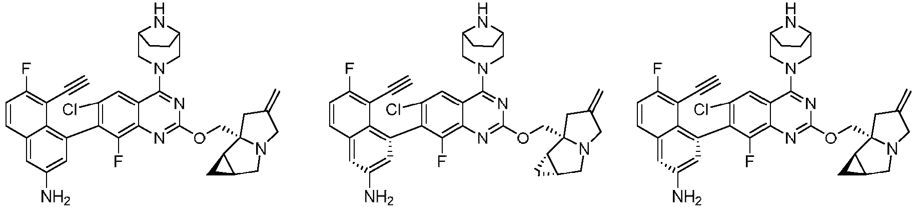 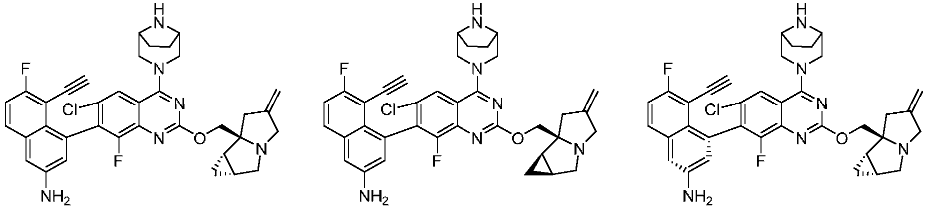 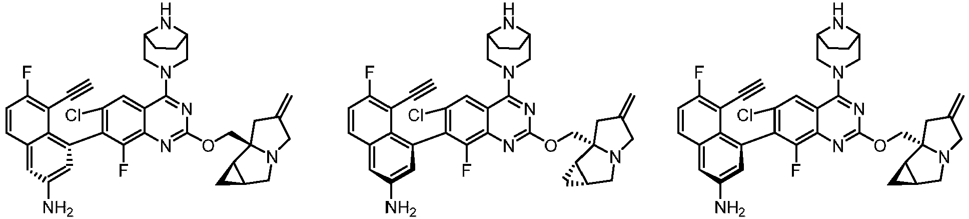 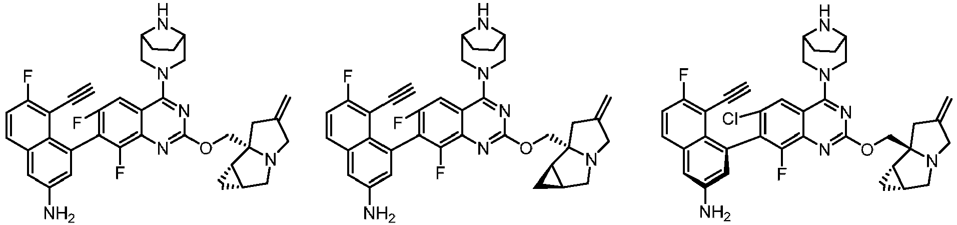 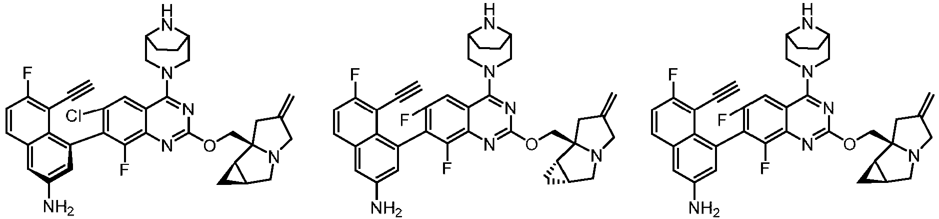 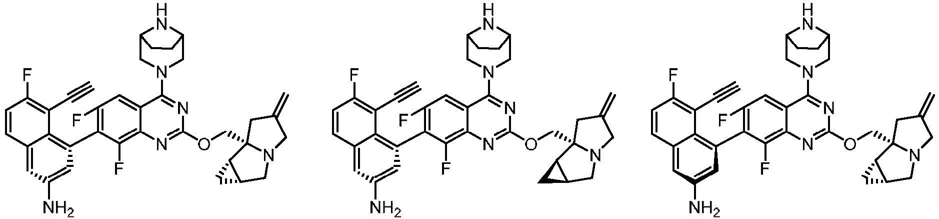 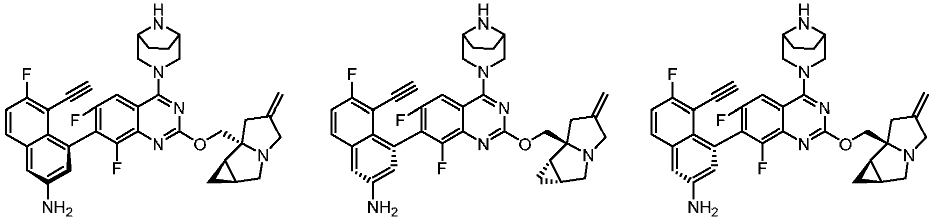 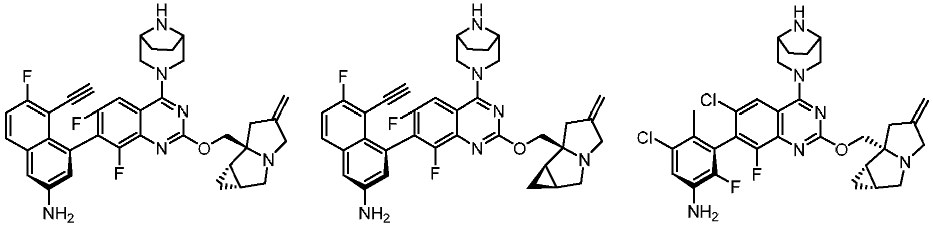 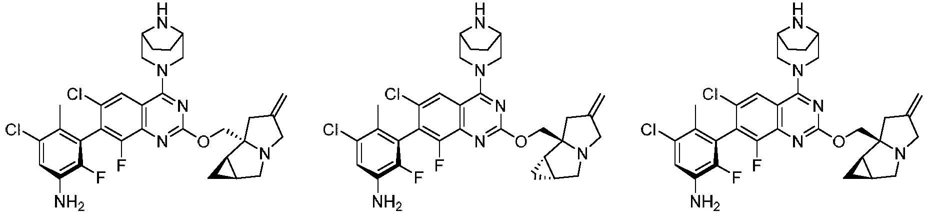 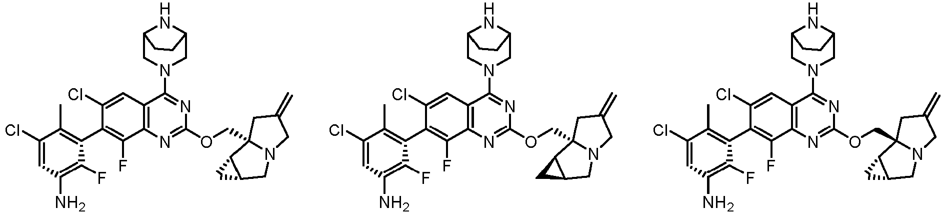 or

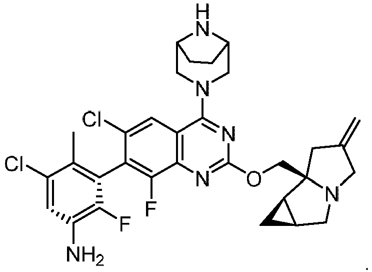

In some embodiments of the present disclosure, the compound or the pharmaceutically acceptable salt thereof is provided, and the compound is selected from: 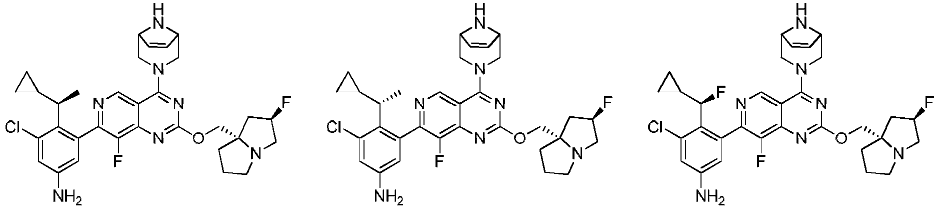 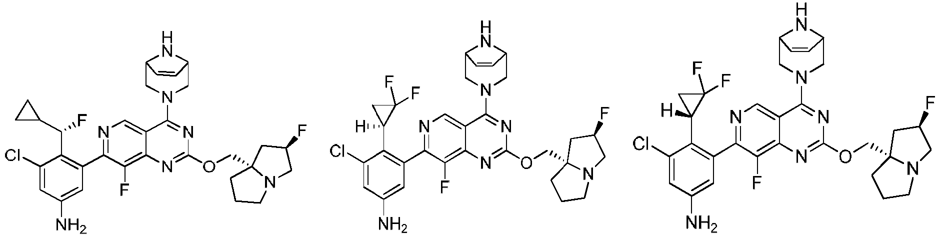 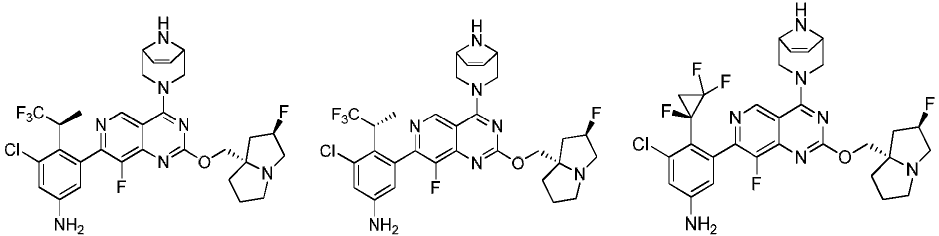 and

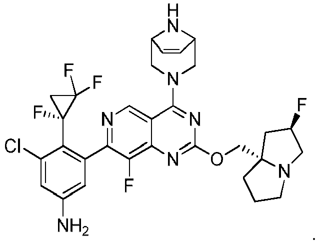

The present disclosure provides a use of the compound or the pharmaceutically acceptable salt thereof in the manufacture of a medicament for treating solid tumors with KRAS^{G12D} mutation.

The present disclosure provides a pharmaceutical composition comprising the compound represented by any one of the chemical general formulas in formula I, formula II, formula III-1, formula III-2, or the pharmaceutically acceptable salt thereof of the present disclosure, and any pharmaceutically acceptable carrier.

The present disclosure provides a use of the compound represented by any one of the chemical general formulas in formula I, formula II, formula III-1, formula III-2, or the pharmaceutically acceptable salt thereof of the present disclosure in the manufacture of a medicament for treating solid tumors with KRAS^{G12D} mutation; preferably, the solid tumors are selected from colon cancer and pancreatic cancer.

The present disclosure further provides a use of the above pharmaceutical composition in the manufacture of a medicament for treating solid tumors with KRAS^{G12D} mutation; preferably, the solid tumors are selected from colon cancer and pancreatic cancer.

The present disclosure further provides the following synthesis methods:

### Synthesis route 1:

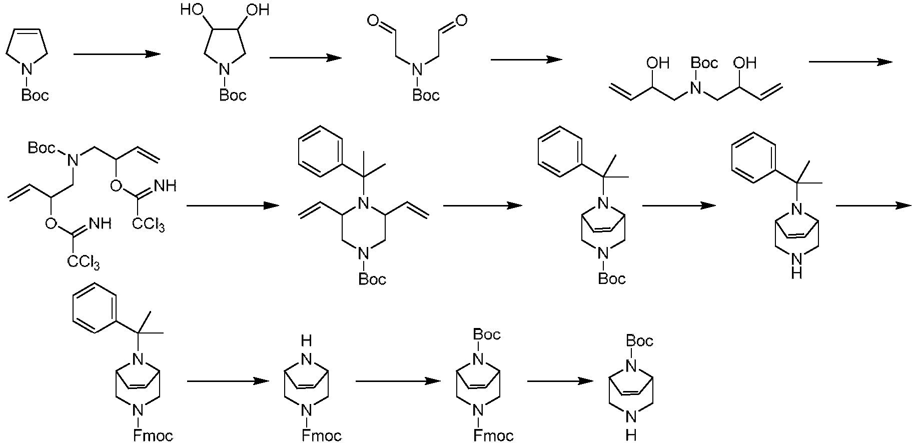

### Synthesis route 2:

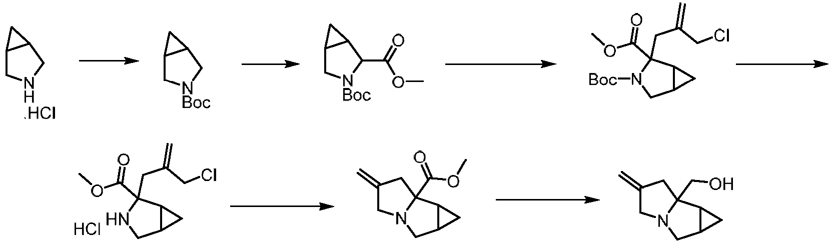

### Synthesis route 3:

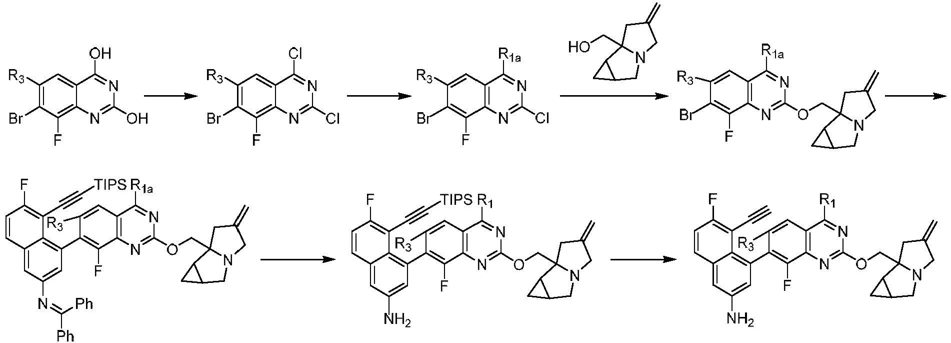

wherein R₃ is selected from H, F, and Cl; R₁ₐ is selected from 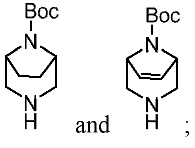 and R₁ is selected from

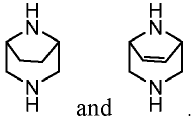

### Synthesis route 4:

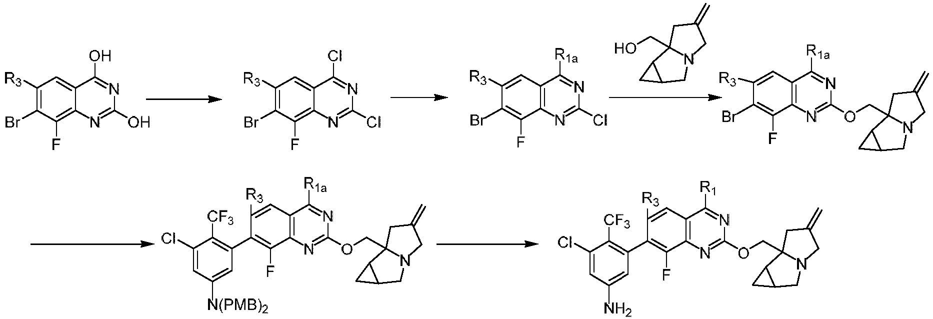

wherein R₃ is selected from H, F, and Cl; R₁ₐ is selected from 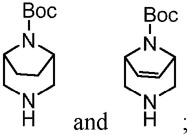 R₁ is selected from

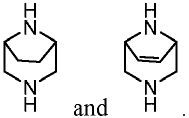

The present disclosure further provides the following test methods:

### Test method 1. KRAS^{G12D} inhibitory activity test

### 1. Experimental purpose:

Through the TR-FRET method, compounds that can effectively inhibit the binding of KRAS^{G12D} to GTP are screened.

### 2. Consumables and instruments:

**Table 1. Consumables and instruments**

| Name | Supplier | Cat. No. |
|---|---|---|
| HEPES (2-[4-(2-hydroxyethyl)piperazin-1-yl]ethanesulfonic acid) pH 7.3 | Thermo Fisher Scientific | BP299-500 |
| Sodium chloride | Promega | V4221 |
| EDTA (ethylenediaminetetraacetic acid) | EMD Millipore | 324506 |
| Tween 20 | Bio-Rad | 1706531 |
| Magnesium chloride | MP Biomedicals | 191421 |
| Bodipy GDP (guanosine 5'-diphosphate, BODIPY^{™} FL 2'-(or -3')-O-(*N*-(2-aminoethyl)urethane), bis(triethylammonium) salt) | Invitrogen | G22360 |
| GTP (guanosine 5'-triphosphate) | Sigma | G8877 |
| Tb-SA (terbium-labeled streptavidin) | Invitrogen | PV3576 |
| SOS (son of sevenless) protein | | |
| KRAS^{G12D} (Kirsten rat sarcoma viral oncogene) protein | | |
| Compound plate | Labcyte | LP-0200 |
| Assay plate | PerkinElmer | 6008269 |
| 15 mL centrifuge tube | Corning | 430791 |
| 1.5 mL centrifuge tube | Axygen | MCT-150-C |
| Dragonfly automatic sampler | TTP | |
| Bravo | Agilent | |
| Echo 550 | Labcyte | |
| Envision | PerkinElmer | |

### 3. Reagent preparation:

### a. Storing reagents:

### 1) KRAS nucleotide exchange buffer

20 mL of 1000 mM HEPES, 20 mL of 500 mM EDTA, 10 mL of 5 M sodium chloride, 0.1 mL of 100% Tween 20, and 949.9 mL of water are taken to prepare a 1 L solution, sterilized by filtration, and stored at 4°C.

### 2) KRAS assay buffer

20 mL of 1000 mM HEPES, 10 mL of 1000 mM magnesium chloride, 30 mL of 5 M sodium chloride, 0.05 mL of 100% Tween 20, and 939.95 mL of water are taken to prepare a 1 L solution, sterilized by filtration, and stored at 4°C.

### 3) KRAS/Bodipy GDP/Tb-SA mixture

9.5 µL of 95 µM KRAS^{G12D} protein is mixed with 440.5 µL of KRAS nucleotide exchange buffer, incubated at room temperature for 1 hour, and mixed with 8.4 µL of 17.9 µM Tb-SA, 1.8 µL of 5 mM Bodipy GDP, and 9539.8 µL of KRAS assay buffer to prepare a 1 L solution. After mixing, the solution is left to stand at room temperature for 6 hours and stored at -80°C.

### b. Assay reagents:

### 1) KRAS enzyme solution

73.3 µL of KRAS/Bodipy GDP/Tb-SA mixture is taken and mixed with 2126.7 µL of KRAS assay buffer to prepare a 2200 µL solution.

### 2) SOS/GTP mixture

1.59 µL of 166 µM SOS protein, 198 µL of 100 mM GTP, and 2000.41 µL of KRAS assay buffer are taken to prepare a 2200 µL solution.

### 4. Experimental process:

1) The concentration of the control compound stock solution is 1 mM, and the concentration of the test compound stock solution is 10 mM. 9 µL of control compound and test compound are transferred to a 384-LDV plate;
2) The compound on the LDV plate is diluted 3 times at 10 points using Bravo;
3) The compound on the LDV plate is transferred to the assay plate using ECHO in a volume of 9 nL;
4) Using the Dragonfly automatic sampler, 3 µL of a mixture of 3 nM Kras/0.5 nM TB-SA/30 nM BodipyGDP and 3 µL of Ras buffer are sequentially added to each well of the assay plate, and the assay plate is centrifuged at 1000 rpm/min for 1 minute;
5) The assay plate is incubated at room temperature for 1 hour;
6) Using the Dragonfly automatic sampler, 3 µL of 120 nM SOS/9 mM GTP mixture is added to each well of the assay plate, and the assay plate is centrifuged at 1000 rpm/min for 1 minute;
7) The assay plate is incubated at room temperature for 1 hour;
8) Envision is used to read the plate and record the data;
9) Data analysis is conducted using Excel and Xlfit to calculate the IC₅₀ of the test compound.

### Test method 2. p-ERK inhibition test of GP2D cells

### 1. Experimental purpose:

Through the HTRF method, compounds that can effectively inhibit p-ERK in GP2D cells are screened out.

### 2. Experimental process:

1). GP2D cells are seeded in a transparent 96-well cell culture plate, with 80 µL of cell suspension per well and 8000 cells per well, and the cell plate is incubated in a carbon dioxide incubator at 37°C overnight;
2). 2 µL of the compound is added to 78 µL of cell culture medium; after mixing well, 20 µL of the compound solution is taken and added to the corresponding cell plate wells, and the cell plate is put back into the carbon dioxide incubator to incubate for another 1 hour;
3). After the incubation is completed, the cell supernatant is discarded, and 50 µL of 1X cell lysate is added to each well, and incubated at room temperature for 30 minutes with shaking;
4). Phospho-ERK1/2 Eu Cryptate antibody and Phospho-ERK1/2 d2 antibody are 20-fold diluted using detection buffer;
5). The supernatant of the cell lysate is added to a new 384-well white microplate at 16 µL per well, then 2 µL of Phospho-ERK1/2 Eu Cryptate antibody diluent and 2 µL of Phospho-ERK1/2 d2 antibody diluent are added thereto and incubated at room temperature for at least 4 hours;
6). After the incubation is completed, a multimode microplate reader is used to read HTRF excitation: 320 nm, emission: 615 nm, 665 nm;
7). The IC₅₀ of the test compound is calculated.

### Test method 3. GP2D 3D CTG experiment

### 1. Experimental purpose:

This experiment aims to verify the inhibitory effect of the compounds of the present disclosure on the proliferation of GP2D human colon cancer cells with KRAS G12D mutation.

### 2. Experimental materials:

Cell line GP2D, DMEM medium, penicillin/streptomycin antibiotics, purchased from Wisent; fetal bovine serum, purchased from Biosera. CellTiter-Glo^{®} 3D Cell Viability Assay reagent (chemiluminescent 3D cell viability assay reagent), purchased from Promega.

### 3. Experimental methods:

GP2D cells are seeded in a 96-well U-bottom cell culture plate, and each well of 80 µL cell suspension contains 2000 GP2D cells. The cell plate is incubated overnight in a CO₂ incubator. The test compound is diluted 5-fold to the eighth concentration using a pipette, ranging from 200 µM to 2.56 nM, and tested in duplicate. Culture medium is added to an intermediate plate at 78 µL per well. Then the gradient diluted compound is transferred to the corresponding wells of the intermediate plate at 2 µL per well and mixed well. The mixture is then transferred to the cell plate at 20 µL per well. The concentration of the compound transferred to the cell plate ranges from 1 µM to 0.0128 nM. The cell plate is incubated for 5 days in a CO₂ incubator. After the incubation of the cell plate with the compound is completed, 100 µL of chemiluminescent cell viability assay reagent is added to each well of the cell plate, and incubated at room temperature for 10 minutes to stabilize the luminescent signal. A multimode microplate reader is used for reading.

### 4. Data analysis:

Using the equation (Sample - Min) / (Max - Min) * 100% to convert the raw data into inhibition rate, the IC₅₀ value can be obtained by curve fitting with four parameters (obtained by "log (inhibitor) vs. response -- Variable slope" mode in GraphPad Prism).

### Test method 4. In vivo pharmacokinetic experiment

### 1. Experimental purpose:

This experiment aims to investigate the pharmacokinetic properties of the compounds of the present disclosure administrated by oral and intravenous injection in SD mice.

### 2. Experimental methods:

The test compound is mixed with 10% dimethyl sulfoxide/60% polyethylene glycol 400/30% aqueous solution, vortexed, and sonicated to prepare a clear solution of approximately 1 mg/mL, which is filtered through a microporous membrane and set aside for later use. Male SD mice aged 7 to 10 weeks are selected and intravenously injected with a solution of the candidate compound at a dose of 3 mg/kg. The solution of the candidate compound is administered orally at a dose of approximately 30 mg/kg. Whole blood is collected for a certain period of time and prepared into plasma. The drug concentration is analyzed by the LC-MS/MS method, and the pharmacokinetic parameters are calculated using Phoenix WinNonlin software (Pharsight, USA).

### Test method 5. In vivo pharmacodynamic experiment

### 1. Experimental purpose:

*In vivo* pharmacodynamic study of GP2D cell subcutaneously transplanted tumors in Balb/c Nude mice model with human colon cancer is conducted.

### 2. Experimental methods:

Cell culture: Human colon cancer GP2D cells are cultured in monolayer *in vitro.* The culture conditions are DMEM/F12 medium + 20% fetal bovine serum + 1% penicillin-streptomycin, and cultured in a 37°C, 5% carbon dioxide incubator. Routine digestion with trypsin-EDTA is performed twice a week for passaging. When the cell saturation reaches 80% to 90% and the required quantity is reached, the cells are harvested, counted, resuspended in an appropriate amount of PBS, and mixed with matrigel at a ratio of 1:1 to obtain a cell suspension with a cell density of 25 × 10⁶ cells/mL.

Cell inoculation: 0.2 mL (5 × 10⁶ cells/mouse) of Mia PaCa-2 cells (added with matrigel with a volume ratio of 1:1) are subcutaneously inoculated into the right back of each mouse.

Experimental operation: When the average tumor volume reaches approximately 190 mm³, the mice are randomly divided into groups according to the tumor volume, with 6 animals in each group. The blank group is administered with a dose of 0, and the test group is orally administered with a dose of 30 mg/kg, 100 mg/kg, and a volume of 10 µL/g, respectively, and is administered twice a day for 22 days.

### 3. Tumor measurement and experimental indicators:

Tumor diameters are measured twice a week with a vernier caliper. The formula for calculating tumor volume is: V = 0.5a × b², wherein a and b represent the long and short diameters of the tumor, respectively.

The tumor inhibitory efficacy of compounds is evaluated by TGI (%) or relative tumor proliferation rate T/C (%). Relative tumor proliferation rate T/C (%) = TRTV / CRTV × 100% (TRTV: RTV of treatment group; CRTV: RTV of negative control group). The relative tumor volume (RTV) is calculated based on the results of tumor measurements. The calculation formula is RTV = Vₜ / V₀, where V₀ is the average tumor volume measured at the beginning of administration in groups (*i.e.*, D₀), and Vₜ is the average tumor volume measured at a certain measurement. TRTV and CRTV use the data from the same day.

TGI (%) reflects the tumor growth inhibition rate. TGI (%) = [1 - (average tumor volume at the end of administration in a treatment group - average tumor volume at the beginning of administration in this treatment group) / (average tumor volume at the end of treatment in the vehicle control group - average tumor volume at the beginning of treatment in the vehicle control group)] × 100%.

### Technical effects

The compounds of the present disclosure have a good binding effect with KRAS^{G12D} protein, and can significantly inhibit KRAS^{G12D} enzyme and p-ERK in GP2D cells. The compounds of the present disclosure have good cell proliferation inhibitory activity against cells with KRAS^{G12D} mutation, and have excellent tumor suppression effects. Furthermore, the compounds of the present disclosure have good pharmacokinetic properties.

### Related definitions

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. When the compound of the present disclosure contains a relatively basic functional group, an acid addition salt can be obtained by contacting such compounds with a sufficient amount of acid in a pure solution or a suitable inert solvent. Certain specific compounds of the present disclosure contain both basic and acidic functional groups, and thus can be converted to any base or acid addition salt.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (R)- and (S)-enantiomers, diastereoisomers, (*D*)-isomers, (L)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, in chiral HPLC (chiral high-performance liquid chromatography) analysis and SFC (supercritical fluid chromatography) analysis, retention times of compounds may differ due to factors such as the measuring instrument. There may be a measurement error in the retention time of a compound for any particular compound. Therefore, this error should be taken into account when determining each compound, and the error is also within the scope of the present disclosure.

The compound of the present disclosure may contain an unnatural proportion of atomic isotope at one or more than one atom that constitutes the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, *etc.* All isotopic variations of the compound of the present disclosure, whether radioactive or not, are encompassed within the scope of the present disclosure.

The term "optional" or "optionally" means that the subsequent event or condition may occur but is not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (*i.e., =*O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When the enumerative linking group does not indicate the direction for linking, the direction for linking is arbitrary, for example, the linking group L contained in 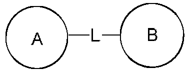 is -M-W-, then -M-W- can link ring A and ring B to form 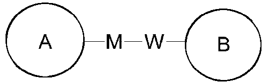 in the direction same as left-to-right reading order, and form 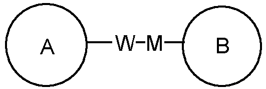 in the direction contrary to left-to-right reading order. A combination of the linking groups, substituents and/or variables thereof is allowed only when such combination can result in a stable compound.

Unless otherwise specified, when a group has one or more than one linkable site, any one or more than one site of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond ( 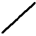 ), a straight dashed bond ( 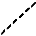 ), or a wavy line ( 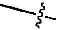 ). For example, the straight solid bond in -OCH₃ means that it is linked to other groups through the oxygen atom in the group; the straight dashed bond in 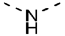 means that it is linked to other groups through the two ends of the nitrogen atom in the group; the wave lines in 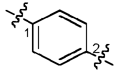 means that the phenyl group is linked to other groups through carbon atoms at position 1 and position 2; 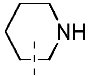 means that it can be linked to other groups through any linkable sites on the piperidinyl by one chemical bond, including at least four types of linkage, including 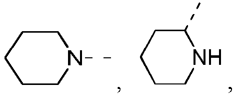 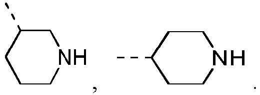 Even though the H atom is drawn on the -N-, 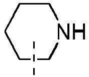 still includes the linkage of 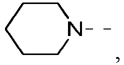 merely when one chemical bond is connected, the H of this site will be reduced by one to the corresponding monovalent piperidinyl.

Unless otherwise specified, the absolute configuration of a stereogenic center is represented by a wedged solid bond ( 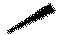 ) and a wedged dashed bond ( 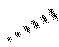 ), and the relative configuration of a stereogenic center is represented by a straight solid bond ( 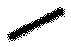 ) and a straight dashed bond ( 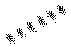 ), a wave line ( 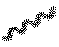 ) is used to represent a wedged solid bond ( 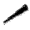 ) or a wedged dashed bond ( 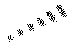 ), or the wave line ( 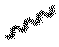 ) is used to represent a straight solid bond ( 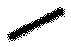 ) or a straight dashed bond ( 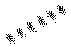 ). For example, 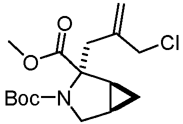 uses a straight solid bond ( 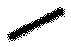 ) and a straight dashed bond ( 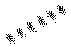 ) to represent the relative configurations of stereocenters, representing a mixture of 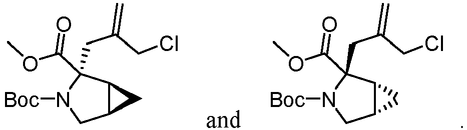 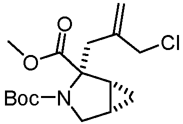 represents a mixture of 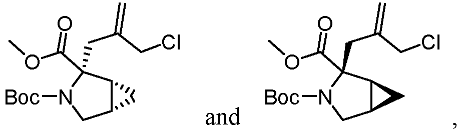 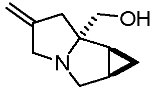 represents a mixture of

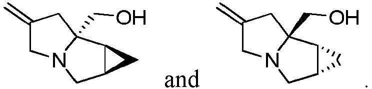

Certain compounds of the present disclosure may exist as atropisomers, which are conformational isomers occurring when rotation around a single bond in the molecule is hindered or significantly slowed due to steric interactions with other parts of the molecule. The compounds disclosed in the present disclosure include all atropisomers, which may be pure individual atropisomers, enriched in one of the atropisomers, or non-specific mixtures thereof. If the rotational potential energy around a single bond is high enough and the interconversion between conformations is slow enough, isomers are allowed to be separated. For example, 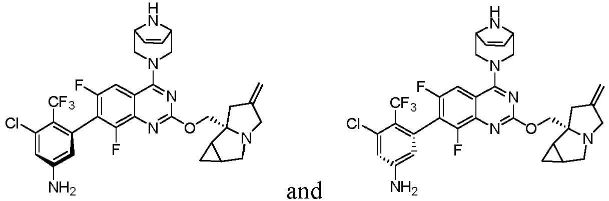 are a pair of atropisomers, wherein 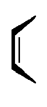 on the phenyl group indicates that the stereo orientation on this side is outward, while 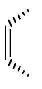 indicates that the stereo orientation on this side is inward.

Unless otherwise specified, when a double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound and its substituent are represented by 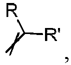 this refers to the (Z) isomer, (E) isomer, or a mixture of two isomers of the compound.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, *etc.*; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, *etc.*

Unless otherwise specified, the term "C₁₋₃ alkyl" refers to a linear or branched saturated hydrocarbon group consisting of 1 to 3 carbon atoms. The C₁₋₃ alkyl includes C₁₋₂, C₂₋₃ alkyl, *etc.*; it can be monovalent (such as methyl), divalent (such as methylene), or multivalent (such as methine). Examples of C₁₋₃ alkyl include, but are not limited to, methyl (Me), ethyl (Et), propyl (including n-propyl and isopropyl), *etc.*

Unless otherwise specified, the term "halide" or "halogen" by itself or as part of another substituent refers to fluorine (F), chlorine (Cl), bromine (Br), or iodine (I) atom.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃, C₂ alkoxy, *etc.* Examples of C₁₋₃ alkoxy include, but are not limited to, methoxy, ethoxy, propoxy (including n-propoxy and isopropoxy), *etc.*

Unless otherwise specified, "C₂₋₄ alkenyl" is used to mean a linear or branched hydrocarbon group consisting of 2 to 4 carbon atoms containing at least one carbon-carbon double bond, wherein the carbon-carbon double bond can be located at any position within the group. The C₂₋₄ alkenyl includes C₂₋₃, C₄, C₃, C₂ alkenyl, *etc.*; The C₂₋₄ alkenyl can be monovalent, divalent, or multivalent. Examples of C₂₋₄ alkenyl include, but are not limited to, vinyl, propenyl, butenyl, butadienyl, *etc.*

Unless otherwise specified, "C₂₋₄ alkynyl" is used to mean a linear or branched hydrocarbon group consisting of 2 to 4 carbon atoms containing at least one carbon-carbon triple bond, wherein the carbon-carbon triple bond can be located at any position within the group. The C₂₋₄ alkynyl includes C₂₋₃, C₄, C₃, C₂ alkynyl, *etc.* It can be monovalent, divalent, or multivalent. Examples of C₂₋₄ alkynyl include, but are not limited to, ethynyl, propynyl, butynyl, *etc.*

Unless otherwise specified, in formula III of the present disclosure, when X is N⁺-O⁻, "N⁺-O⁻" is used to represent "N(→O)", representing an oxide of N.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The solvents used in the present disclosure are commercially available. Boc represents tert-butyloxycarbonyl; Fmoc represents 9-fluorenylmethoxycarbonyl; TIPS represents triisopropylsilyl; PMB represents p-methoxybenzyl; Tf represents trifluoromethanesulfonyl; DCE represents dichloroethane; THF represents tetrahydrofuran; H₂O represents water; FA represents formic acid; ACN represents acetonitrile; PE represents petroleum ether; EA represents ethyl acetate; DEA represents diethanolamine; IPA represents isopropanol; DBU represents 1,8-diazabicyclo[5.4.0]undec-7-ene; The second-generation Grubbs catalyst represents the compound with CAS number of 246047-72-3; The ratio of eluent in column chromatography represents the volume ratio; M in the concentration represents mol/L.

The compounds of the present disclosure are named according to the conventional naming principles in the art or by ChemDraw^{®} software, and the commercially available compounds use the supplier catalog names.

### BRIEF DESCRIPTION OF THE DRAWINGS

FIG. 1. Binding mode diagram of compound A and KRAS^{G12D} protein;
FIG. 2. Binding mode diagram of compound B and KRAS^{G12D} protein;
FIG. 3. Binding mode diagram of compound C and KRAS^{G12D} protein;
FIG. 4. Binding mode diagram of compound D and KRAS^{G12D} protein;
FIG. 5. Binding mode diagram of compound E and KRAS^{G12D} protein;
FIG. 6. Binding mode diagram of compound F and KRAS^{G12D} protein;
FIG. 7. Binding mode diagram of compound G and KRAS^{G12D} protein;
FIG. 8. Binding mode diagram of compound H and KRAS^{G12D} protein;
FIG. 9. Binding mode diagram of compound I and KRAS^{G12D} protein;
FIG. 10. Binding mode diagram of compound J and KRAS^{G12D} protein;
FIG. 11. Binding mode diagram of compound K and KRAS^{G12D} protein.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the specific examples of the present disclosure without departing from the spirit and scope of the present disclosure.

### Calculation example 1

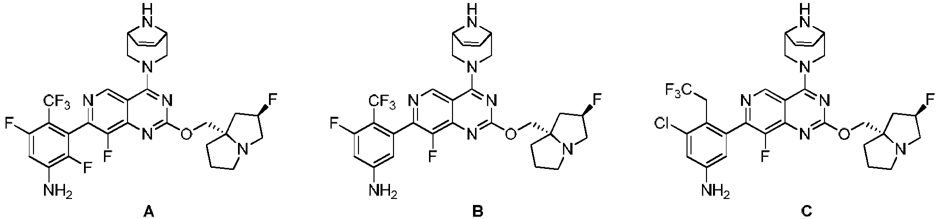

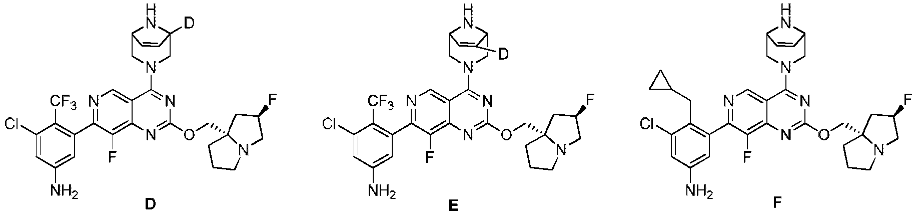

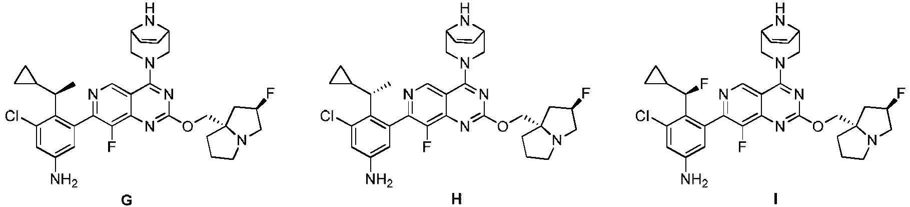

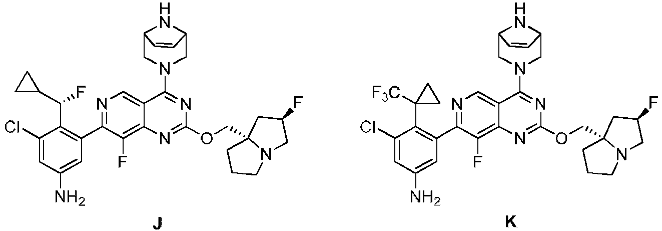

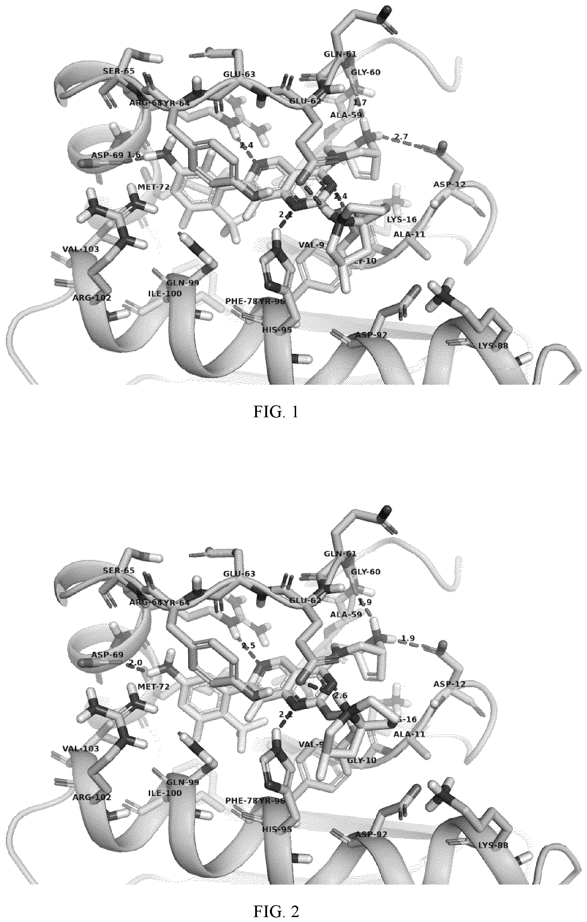

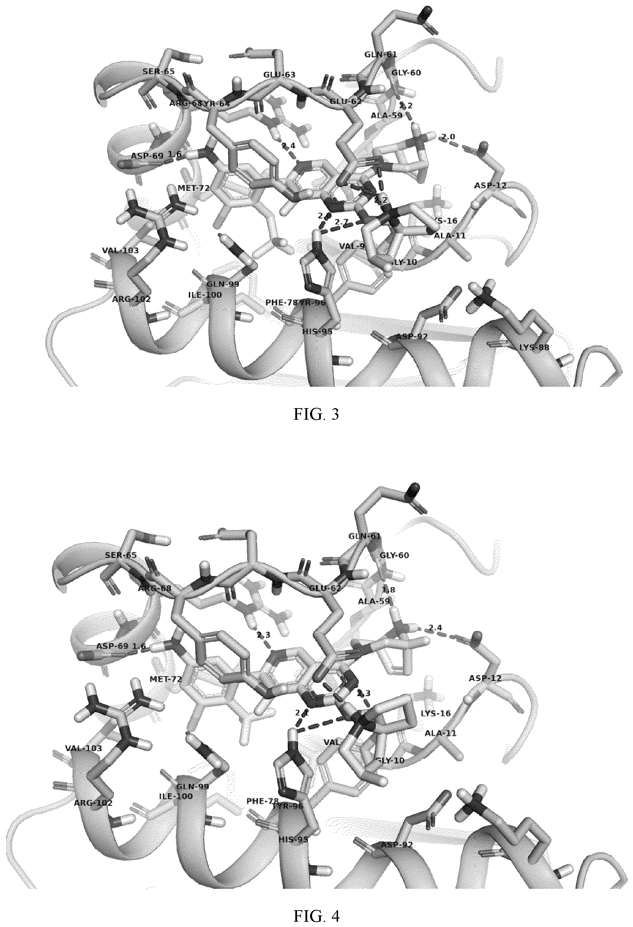

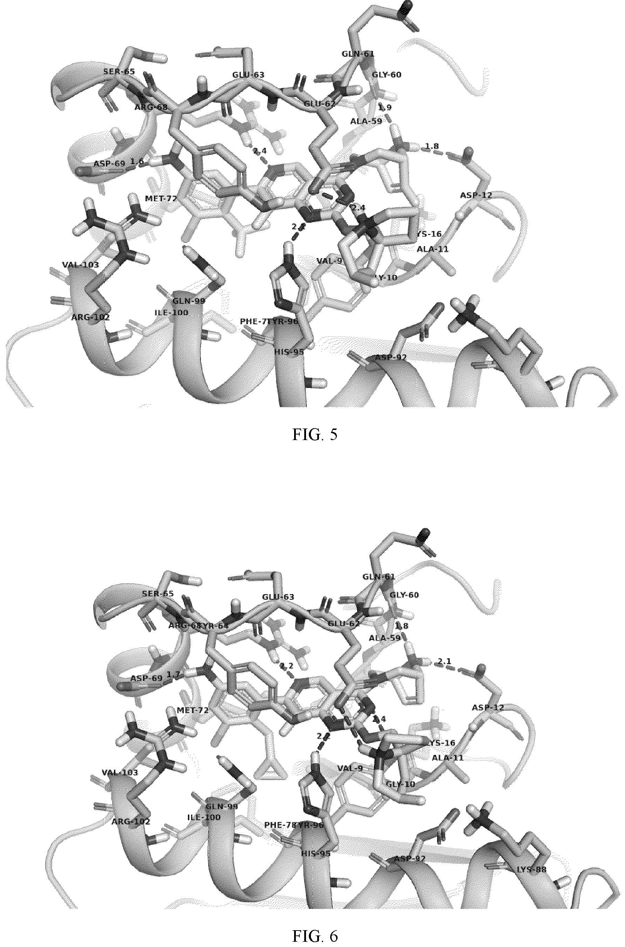

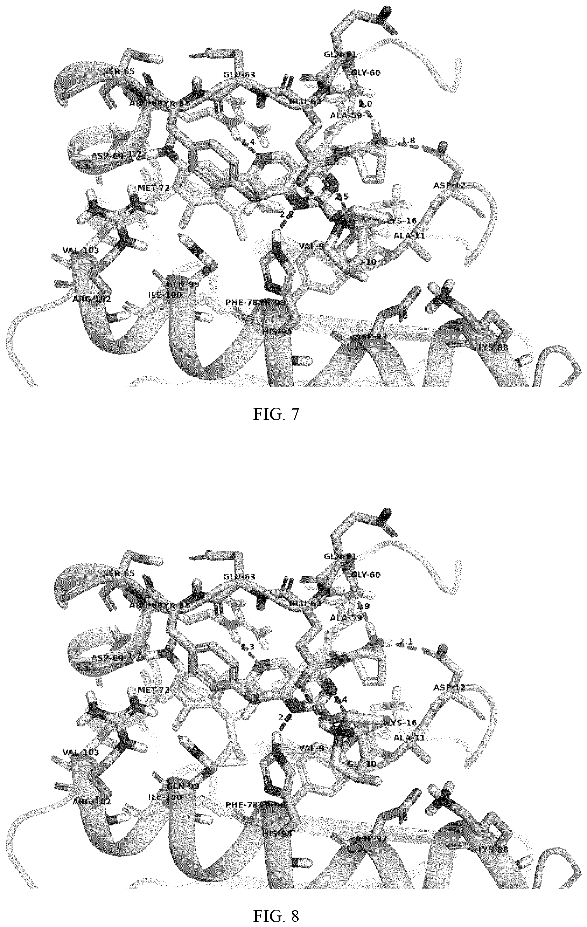

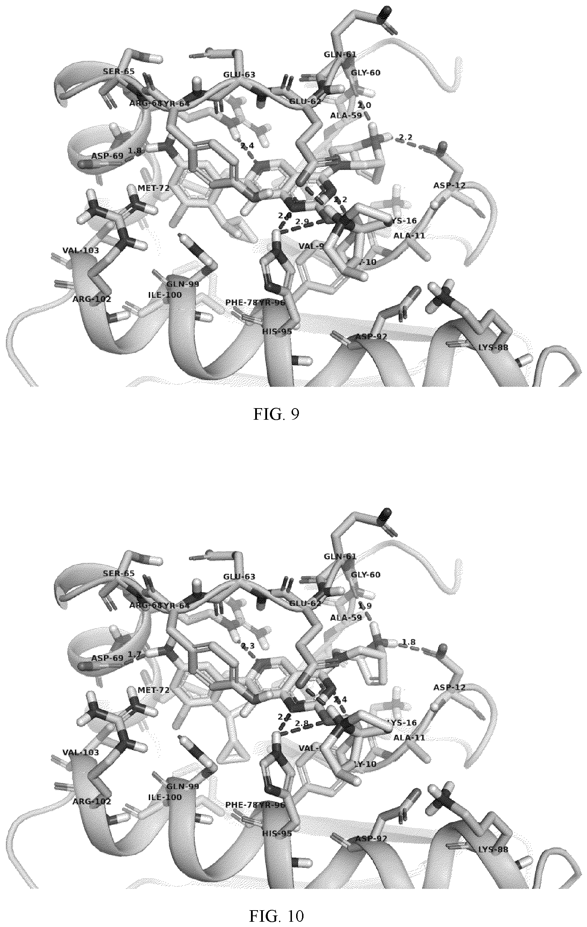

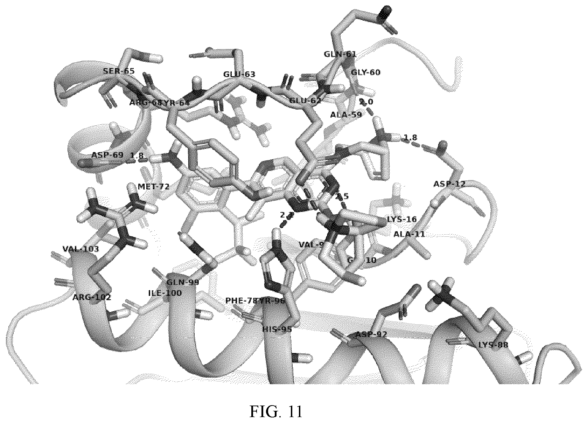

The molecular docking process was conducted by using the Glide SP^{[1]} with default settings in Maestro (Schrödinger version 2017-2). The crystal structure of KRAS_G12C in the PDB database (PDB: 6UT0) was selected, and Cys12 was simulated to be mutated to Asp12. After energy optimization, it was used as a docking template. To prepare protein, hydrogen atoms were added using the Protein Preparation Wizard module of Maestro^{[2]}, and the OPLS3 force field was utilized. For the preparation of ligands, LigPrep was used to generate the three-dimensional structure of the molecule and energy minimization was performed^{[3]}, and the confgen module was used to sample the small molecule conformation. Using the 6UT0 ligand as the center of mass, a cube docking grid with side lengths of 25 Å * 25 Å * 25 Å was generated. The reference compounds were placed during the molecular docking process. The types of protein receptor-ligand interactions were analyzed, and the types of protein receptor-ligand interactions were analyzed, and then reasonable docking conformations were selected and saved based on the calculated docking scrore as well as the binding modes, as shown in FIG. 1 to FIG. 11.
[1] Glide, Schrödinger, LLC, New York, NY, 2017.
[2] Maestro, Schrödinger, LLC, New York, NY, 2017.
[3] LigPrep, Schrödinger, LLC, New York, NY, 2017.

Conclusion: The compounds of the present disclosure have good binding with KRAS^{G12D}.

### Synthesis of intermediates Int-3A and Int-3B

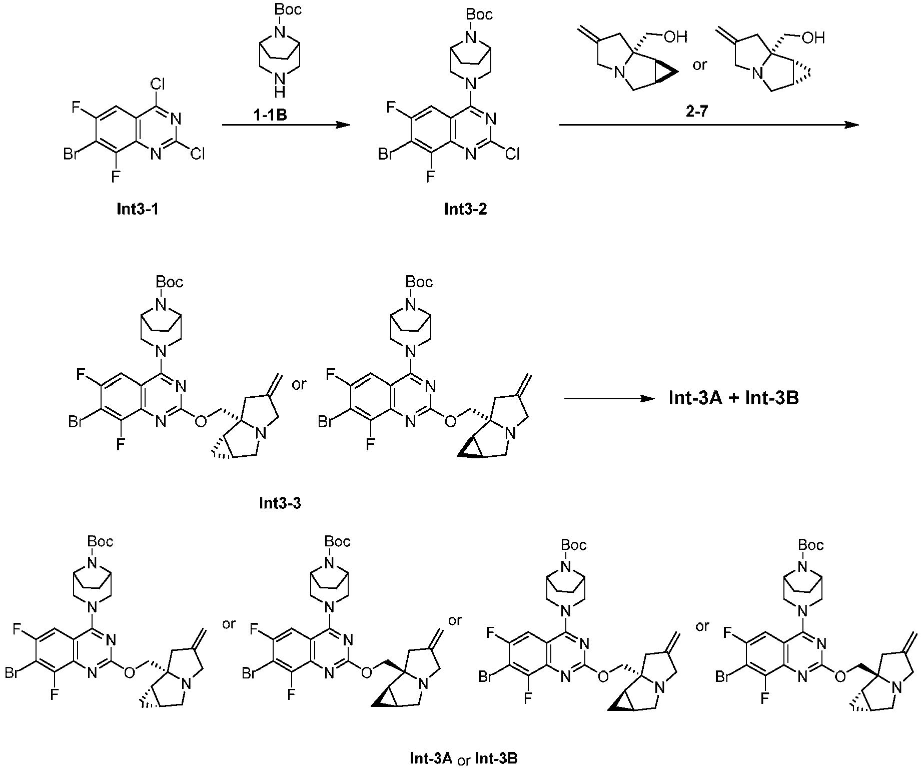

### Step 1:

Under nitrogen atmosphere, Int3-1 (3 g, 9.56 mmol) and 1-1B (1.83 g, 8.60 mmol) were dissolved in dichloromethane (30 mL), then triethylamine (2.90 g, 28.67 mmol) was added thereto at -40°C, and the reaction was carried out at -40°C for 0.5 hours. Water (20 mL) was added to the reaction system, and the aqueous phase was extracted with dichloromethane (20 mL * 4), and then the phases were separated. The organic phase was washed with saturated brine (20 mL), dried over anhydrous sodium sulfate, filtered, and concentrated under reduced pressure to obtain a crude product. The crude product was purified by column chromatography (mobile phase: petroleum ether: ethyl acetate = 20:1 to 1.5:1) to obtain intermediate **Int3-2.** MS m/z: 488.9, 490.9 [M+1]⁺.

### Step 2:

Under nitrogen atmosphere, intermediate **Int3-2** (0.8 g, 1.63 mmol) and intermediate **2-7** (269.91 mg, 1.63 mmol) were dissolved in N,N-dimethylformamide (8 mL) and tetrahydrofuran (8 mL), then cesium carbonate (1.33 g, 4.08 mmol) and triethylenediamine (54.97 mg, 490.06 µmol) were added thereto, and the reaction was carried out at 25°C for 15 hours. Water (10 mL) was added to the reaction system, and the aqueous phase was extracted with ethyl acetate (10 mL * 5), and then the phases were separated. The organic phase was washed with saturated brine (10 mL), dried over anhydrous sodium sulfate, filtered, and concentrated under reduced pressure to obtain a crude product. The crude product was purified by column chromatography (mobile phase: petroleum ether: ethyl acetate = 25:1 to 1:1) to obtain intermediate **Int3-3.** MS m/z: 618.0 [M+1]⁺.

### Step 3:

Intermediate **Int3-3** was separated by preparative SFC (chromatographic column: REGIS(S,S)WHELK-O1 (250 mm * 25 mm, 10 µm); mobile phases: A (supercritical CO₂) and B (isopropanol, containing 0.1% ammonia); gradient: B%:60% to 60%, 10 min) to obtain intermediates **Int-3A** and **Int-3B.**

Intermediate **Int-3A:** SFC analysis method: chromatographic column REGIS(S,S)WHELK-O1 (50 mm * 4.6 mm, 3.5 µm), mobile phase: A (supercritical CO₂) and B (isopropanol, containing 0.1% isopropylamine); gradient: B%: 5% to 5%; the retention time was 1.780 min, and the ee value was 95.37%. ¹H NMR (400 MHz, CDCl₃) δ = 7.32 (dd, *J* = 9.2, 2.0 Hz, 1H), 5.02 (s, 1H), 4.94 (s, 1H), 4.38 - 4.25 (m, 4H), 4.24 - 4.17 (m, 1H), 3.66 - 3.48 (m, 3H), 3.34 - 3.20 (m, 2H), 2.93 (d, *J =* 17.2 Hz, 1H), 2.75 (dd, *J =* 8.8, 4.0 Hz, 1H), 2.47 (d, *J =* 18.4 Hz, 1H), 1.95 (t, *J* = 5.6 Hz, 2H), 1.89 - 1.75 (m, 3H), 1.60 - 1.55 (m, 2H), 1.52 (s, 9H), 0.75 (d, *J =* 3.2 Hz, 1H), 0.52 - 0.45 (m, 1H). MS m/z: 618.0 [M+1]⁺.

Intermediate **Int-3B:** SFC analysis method: chromatographic column REGIS(S,S)WHELK-O1 (50 mm * 4.6 mm, 3.5 µm), mobile phase: A (supercritical CO₂) and B (isopropanol, containing 0.1% isopropylamine); gradient: B%: 5% to 5%; the retention time was 1.914 min, and the ee value was 96.62%. ¹H NMR (400 MHz, CDCl₃) δ = 7.33 (dd, *J* = 9.2, 2.0 Hz, 1H), 5.18 - 4.85 (m, 2H), 4.52 - 4.09 (m, 6H), 3.73 - 3.44 (m, 3H), 3.43 - 3.10 (m, 2H), 2.88 - 2.66 (m, 1H), 2.62 - 2.36 (m, 1H), 2.02 - 1.67 (m, 6H), 1.52 (s, 9H), 1.27 (d, *J* = 4.4 Hz, 1H), 0.89 - 0.65 (m, 1H), 0.63 - 0.40 (m, 1H). MS m/z: 618.0 [M+1]⁺.

### Synthesis of intermediate Int-4

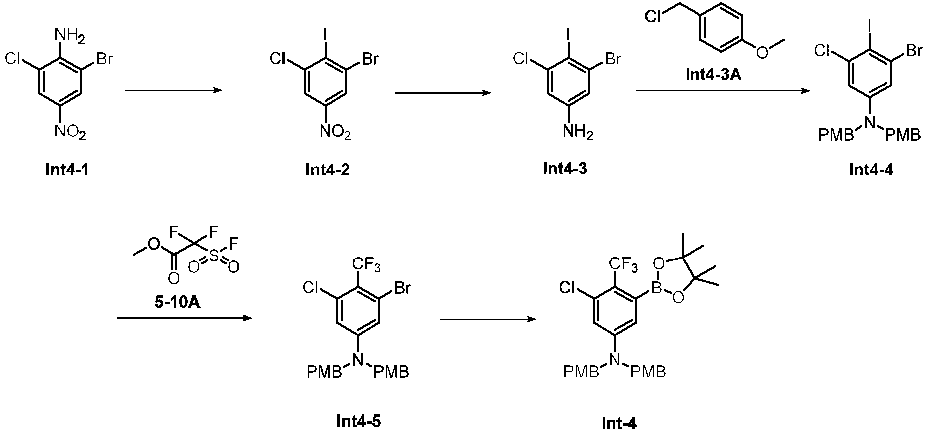

### Step 1:

**Int4-1** (100 g, 397.67 mmol) was dissolved in acetic acid (300 mL) and cooled to 0°C, then concentrated sulfuric acid (390.03 g, 3.98 mol) was added thereto, and then a solution of sodium nitrite (54.87 g, 795.34 mmol) in water (50 mL) was added dropwise thereto. The reaction was carried out at 0°C for 1 hour until the suspension became clear, then a solution of potassium iodide (132.03 g, 795.34 mmol) in water (50 mL) was added dropwise thereto, and the reaction was carried out at 0°C for 1 hour. 5000 mL of water was added thereto, and the mixture was stirred for 10 minutes, then filtrated with suction, rinsed with water for 5 times, added with 500 mL of saturated sodium thiosulfate aqueous solution, and stirred overnight. The mixture was filtrated with suction again, washed with water (500 mL * 4) for 5 times, and filtrated with suction. The filter cake was intermediate **Int4-2.** ¹H NMR (400 MHz, CDCl₃) δ = 8.37 (d, *J =* 2.0 Hz, 1H), 8.24 (d, *J =* 2.0 Hz, 1H).

### Step 2:

Intermediate **Int4-2** (126.15 g, 348.15 mmol) was dissolved in ethanol (500 mL) and water (200 mL), and then iron powder (38.88 g, 696.29 mmol) and ammonium chloride (37.25 g, 696.29 mmol) were added thereto. The reaction was carried out at 80°C for 2 hours. The reaction mixture was filtrated with suction. The filtrate was concentrated under reduced pressure, extracted with ethyl acetate (500 mL * 2), washed with saturated brine (500 mL * 6), dried over anhydrous sodium sulfate, and concentrated under reduced pressure to obtain a crude product. The crude product was subjected to flash column chromatography (eluent: ethyl acetate/petroleum ether, with an ethyl acetate ratio of 0 to 20%) to obtain intermediate **Int4-3.** MS (ESI) m/z: 331.8, 333.8 [M+1]⁺.

### Step 3:

Intermediate **Int4-3** (46 g, 138.40 mmol) was dissolved in N,N-dimethylformamide (300 mL), then sodium hydride (16.61 g, 415.21 mmol, 60%) was added thereto at 0°C, and the mixture was stirred for 0.5 hours at 0°C. Then, intermediate **Int4-3A** (65.03 g, 415.21 mmol) was added thereto, and the reaction mixture was gradually warmed to 25°C and reacted for 2 hours. The reaction mixture was slowly added with 1000 mL of water to precipitate completely and filtrated with suction. The filter cake was rinsed with water (250 mL * 4) and dried under vacuum at 50°C to obtain intermediate **Int4-4.** ¹H NMR (400 MHz, CDCl₃) δ = 7.09 (d, J=8.53 Hz, 4H), 6.94-6.97 (m, 1H), 6.88 (d, J=8.53 Hz, 4H), 6.80 (d, J=2.76 Hz, 1H), 4.50 (s, 4H), 3.81 (s, 6H).

### Step 4:

Intermediate **Int4-4** (78.66 g, 137.36 mmol) was dissolved in *N,N-*dimethylformamide (280 mL), and cuprous iodide (130.80 g, 686.80 mmol) was added thereto. After replacing with nitrogen, the mixture was heated to 100°C, and then intermediate **5-10A** (211.11 g, 1.10 mol) was added thereto, and the reaction was carried out for 0.5 hours. 500 mL of ethyl acetate and 500 mL of water were added thereto, and the phases were separated. The organic phase was washed with water (1 L * 5) for 5 times, then washed with saturated brine (1 L * 3). The aqueous phase was extracted again with 1 L of ethyl acetate, and the phases were separated again. The organic phase was washed with water (1 L * 5) and then washed with saturated sodium chloride solution (1 L * 3). All organic phases were concentrated under reduced pressure to obtain a crude product. 300 mL of methanol was added to the obtained crude product, stirred, and filtered with suction. The filter cake was intermediate **Int4-5.** ¹H NMR (400 MHz, CDCl₃) δ = 7.05-7.13 (m, 4H), 6.96 (d, J=3.01 Hz, 1H), 6.84-6.92 (m, 4H), 6.80 (d, J=2.76 Hz, 1H), 4.50 (s, 4H), 3.81 (s, 6H).

### Step 5:

Intermediate **Int4-5** (0.098 g, 190.38 µmol), bis(pinacolato)diboron (483.44 mg, 1.90 mmol), and potassium acetate (56.05 mg, 571.14 µmol) were added to anhydrous dioxane (2.5 mL), followed by three nitrogen replacements. [1,1'-Bis(diphenylphosphino)ferrocene]dichloropalladium(II) (41.79 mg, 57.11 µmol) was added thereto, and the reaction mixture was replaced with nitrogen again for three times, and then heated to 110°C, and reacted for 20 hours. The reaction mixture was added with 50 mL of water, extracted with 150 mL of ethyl acetate, and the phases were separated. The organic phase was washed with saturated brine (50 mL * 3), and concentrated under reduced pressure to obtain a crude product. The crude product was separated by flash column chromatography (eluent: ethyl acetate: petroleum ether: 0 to 10%) to obtain intermediate **Int-4.** MS (ESI) m/z: 562.2 [M+1]⁺.

### Synthesis of intermediate Int-5

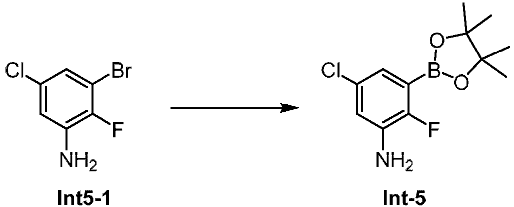

**Int5-1** (3 g, 13.37 mmol), bis(pinacolato)diboron (5.09 g, 20.05 mmol), and potassium acetate (2.62 g, 26.73 mmol) were dissolved in *N,N-*dimethylformamide (30 mL), followed by three nitrogen replacements. 1,1'-Bis(diphenylphosphino)ferrocene-palladium(II)dichloride dichloromethane complex (1.09 g, 1.34 mmol) was added thereto, and the reaction mixture was heated to 100°C and stirred for 6 hours. The reaction mixture was poured into 150 mL of water and extracted with ethyl acetate (20 mL * 3). The organic phases were combined, washed with saturated brine (15 mL), dried over anhydrous sodium sulfate, filtered, and concentrated under reduced pressure. The crude product was purified by column chromatography (eluent: petroleum ether: ethyl acetate = 1:0 to 3:1) to obtain **Int-5.** ¹H NMR (400 MHz, CDCl₃) δ 7.05 (dd, J = 4.0, 2.8 Hz, 1H), 6.87 (dd, J = 7.6, 2.4 Hz, 1H), 3.16 - 4.46 (m, 2H), 1.38 (s, 12H).

### Example 1

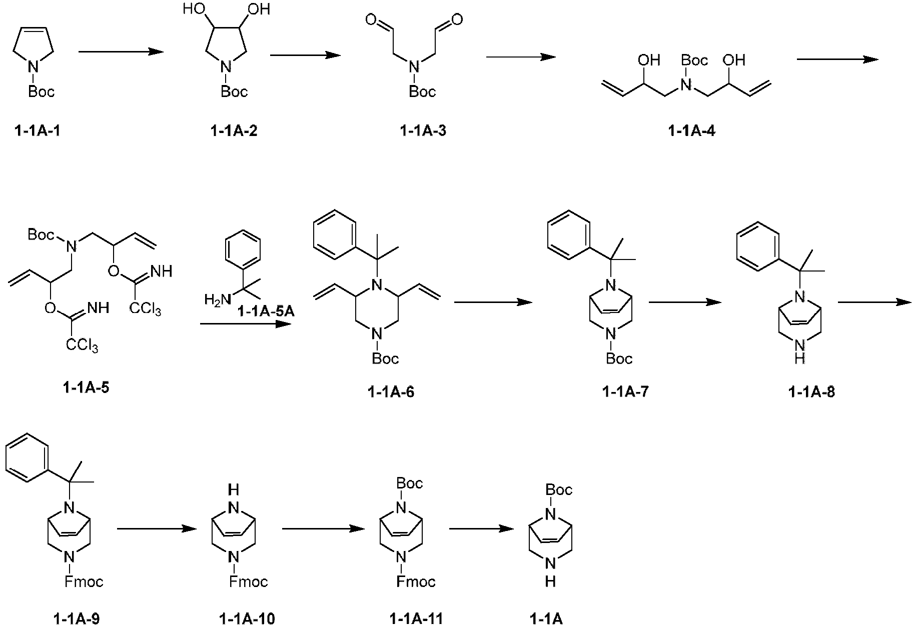

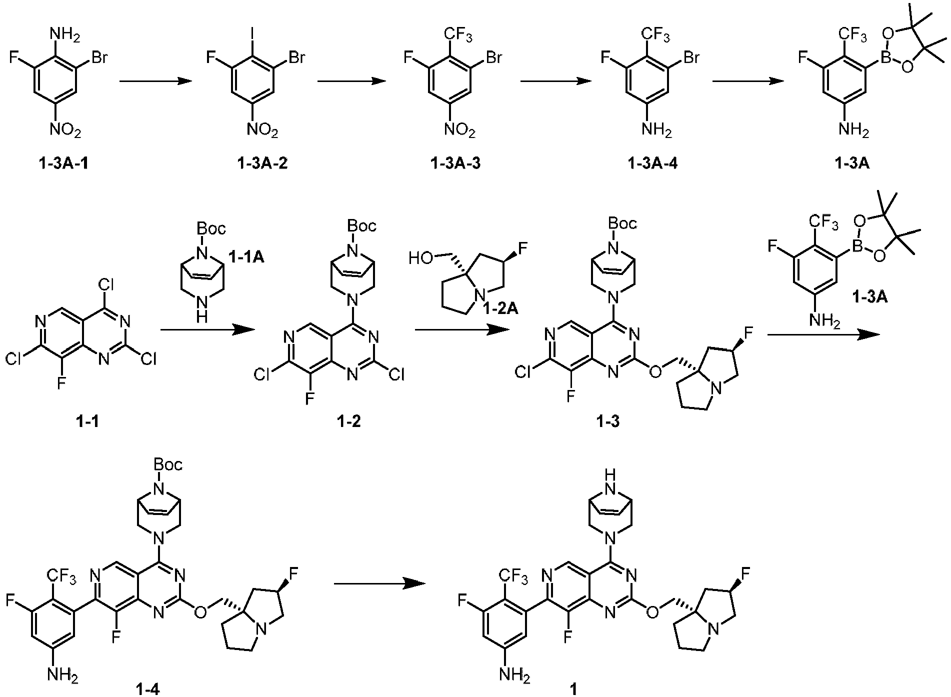

### Step 1: Preparation of intermediate 1-1A-2

Intermediate **1-1A-1** (120 g, 709 mmol) was dissolved in tert-butanol (1200 mL) and water (1200 mL), and then potassium osmate(VI) dihydrate (10.4 g, 28.3 mmol) and 4-methylmorpholine N-oxide (249 g, 2.13 mol) were added thereto. The reaction mixture was stirred at 45°C for 16 hours. The reaction mixture was concentrated under reduced pressure to remove excess solvent, extracted with ethyl acetate (500 mL * 2), and washed with saturated sulfurous acid solution (1000 mL). The combined organic layers were washed with saturated brine (500 mL * 3), dried over anhydrous sodium sulfate, filtered, and concentrated under reduced pressure to obtain a crude product. The crude product was purified by column chromatography (petroleum ether/ethyl acetate = 1/0 to 0/1) to obtain **1-1A-2.** ¹H NMR (400 MHz, CDCl₃) *δ* 4.23 (t, *J* = 3.6 Hz, 2H), 3.55-3.58 (m, 2H), 3.35-3.32 (m, 2H), 2.87-2.83 (m, 2H), 1.45 (s, 9H).

### Step 2: Preparation of intermediate 1-1A-3

Intermediate **1-1A-2** (107 g, 526 mmol) was dissolved in dichloromethane (1700 mL), cooled to 0°C, and then iodobenzene diacetate (254 g, 789 mmol) was added thereto. The reaction system was transferred to 25°C and stirred for 3 hours. Saturated sodium bicarbonate solution (500 mL) was added to quench the reaction system, and dichloromethane (100 mL) was added and stirred for 0.5 hours. The organic phase was then dried over anhydrous sodium sulfate, filtered, and concentrated under reduced pressure to obtain a crude product. The crude product was added with methyl tert-butyl ether (200 mL) at 25°C, stirred for 10 minutes, filtered, and concentrated under reduced pressure to obtain crude intermediate **1-1A-3.**

### Step 3: Preparation of intermediate 1-1A-4

Intermediate **1-1A-3** (200 g) was dissolved in tetrahydrofuran (600 mL), cooled to - 78°C, and then vinylmagnesium bromide (1 M, 1.79 L) was added to the reaction system. The reaction system was then warmed to 25°C and stirred for 16 hours. The reaction system was quenched by adding saturated ammonium chloride solution (1000 mL) at 10°C and extracted with ethyl acetate (500 mL). The organic phase was washed with saturated brine (500 mL * 3), dried over anhydrous sodium sulfate, filtered, and concentrated under reduced pressure to obtain a crude product. The crude product was purified by column chromatography (petroleum ether/ethyl acetate = 1/0 to 0/1) to obtain intermediate **1-1A-4.** ¹H NMR (400 MHz, CDCl₃) *δ* 5.82-5.89 (m, 2H), 5.32 (t, *J* = 11.6 Hz, 2H), 5.16-5.19 (m, 2H), 4.45 (s, 2H), 3.60-3.70 (m, 1H), 3.37 (s, 2H), 3.25 (s, 1H), 2.95 (d, *J =* 8.8 Hz, 1H), 1.48 (s, 9H).

### Step 4: Preparation of intermediate 1-1A-5

Intermediate **1-1A-4** (80.0 g, 310 mmol) was dissolved in dichloromethane (1000 mL), then the reaction system was transferred to 0°C, and DBU (23.6 g, 155 mmol) and 2,2,2-trichloroacetonitrile (269 g, 1.87 mol) were added thereto. The reaction system was transferred to 25°C and stirred for 16 hours. The reaction mixture was concentrated under reduced pressure. The residue was extracted with ethyl acetate (500 mL * 2), washed with water (100 mL * 2), washed with saturated brine (100 mL * 3), dried over anhydrous sodium sulfate, filtered, and concentrated under reduced pressure to obtain a crude product. The crude product was purified by column chromatography (petroleum ether/ethyl acetate = 1/0 to 0/1) to obtain intermediate **1-1A-5.** ¹H NMR (400 MHz, CDCl₃) δ 8.37 (s, 2H), 5.81-5.87 (m, 2H), 5.45 (s, 2H), 5.39-5.43 (m, 2H), 5.25-5.30 (m, 2H), 3.61-3.81 (m, 4H), 1.48 (s, 9H).

### Step 5: Preparation of intermediate 1-1A-6

Intermediate **1-1A-5A** (32.1 g, 238 mmol) was dissolved in DCE (850 mL), and then chloro(1,5-cyclooctadiene)iridium(I) dimer (12.3 g, 18.3 mmol) was added thereto. The reaction system was cooled to 0°C, then intermediate **1-1A-5** (100 g, 183.1 mmol) was dissolved in DCE (1.00 L) and transferred to the above reaction system, and the reaction was warmed to 25°C and stirred for 16 hours. The excess solvent was removed by concentration under reduced pressure to obtain a crude product. The crude product was purified by column chromatography (petroleum ether/ethyl acetate = 1/0 to 0/1, petroleum ether/ethyl acetate = 10:1) to obtain intermediate **1-1A-6.** ¹H NMR (400 MHz, CDCl₃) *δ* 7.52-7.55 (m, 2H), 7.30 (t, *J* = 7.2 Hz, 2H), 7.22 (t, *J =* 7.2 Hz, 1H), 5.94-6.03 (m, 2H), 5.10 (t, *J* = 19.2 Hz, 2H), 4.99 (d, *J* = 10.4 Hz, 2H), 3.51-3.61 (m, 4H), 3.33 (t, *J* = 13.6 Hz, 2H), 1.48 (s, 15H).

### Step 6: Preparation of intermediate 1-1A-7

**1-1A-6** (36.0 g, 50.4 mmol) was dissolved in toluene (900 mL), and then second-generation Grubbs catalyst (2.14 g, 2.52 mmol) was added thereto. The reaction system was heated to 125°C and stirred for 16 hours. The reaction mixture was filtered, and the filter cake was discarded. The filtrate was concentrated under reduced pressure to obtain a crude product. The crude product was purified by column chromatography (petroleum ether/ethyl acetate = 1/0 to 0/1, petroleum ether/ethyl acetate = 10:1) to obtain intermediate **1-1A-7.** MS: m/z = 329.2, [M+1]⁺. ¹H NMR (400 MHz, CDCl₃) δ 7.60 (t, *J* = 1.2 Hz, 2H), 7.31 (t, *J* = 7.2 Hz, 2H), 7.22 (s, 1H), 5.96 (t, *J* = 9.2 Hz, 2H), 3.60-3.65 (m, 2H), 3.46-3.53 (m, 2H), 3.09-3.14 (m, 2H), 1.42 (s, 9H), 1.25 (d, *J* = 6.0 Hz, 6H).

### Step 7: Preparation of intermediate 1-1A-8

Intermediate **1-1A-7** (26.8 g, 81.6 mmol) was dissolved in methanol (201 mL), and then hydrogen chloride/methanol (4 M, 67.3 mL) was added thereto. The reaction system was heated to 35°C and stirred for 16 hours. The pH value of the reaction mixture was adjusted to 12. The reaction mixture was extracted with ethyl acetate (30.0 mL), dried over anhydrous sodium sulfate, filtered, and concentrated under reduced pressure to obtain **1-1A-8.** MS: m/z = 229.2, [M+1]⁺. ¹H NMR (400 MHz, CDCl₃) δ 7.62 (t, *J* = 7.2 Hz, 2H), 7.31 (t, J = 7.6 Hz, 2H), 7.21 (s, 1H), 6.01 (s, 2H), 3.42 (s, 2H), 2.89-2.93 (m, 2H), 2.30-2.34 (m, 2H), 1.23 (s, 6H).

### Step 8: Preparation of intermediate 1-1A-9

Intermediate **1-1A-8** (18.6 g, 79.4 mmol) was dissolved in THF (190 mL), and then 9-fluorenylmethyl chloroformate (20.5 g, 79.4 mmol) and sodium carbonate (25.2 g, 238.2 mmol) were added thereto. The reaction mixture was stirred at 0°C for 1 hour. Then the reaction mixture was extracted with ethyl acetate (50.0 mL * 2) and washed with water (200.0 mL). The organic phases were combined, washed with saturated brine (150.0 mL), dried over anhydrous sodium sulfate, filtered, and concentrated under reduced pressure to obtain intermediate **1-1A-9.** MS: m/z = 451.3, [M+1]⁺. ¹H NMR (400 MHz, CDCl₃) *δ* 7.76 (d, *J* = 13.6 Hz, 2H), 7.54-7.61 (m, 4H), 7.24-7.40 (m, 7H), 5.93-6.01 (m, 2H), 4.34-4.40 (m, 2H), 4.21 (s, 1H), 3.70 (t, *J* = 2 Hz, 2H), 3.55-3.59 (m, 2H), 3.15-3.23 (m, 2H), 1.27 (d, *J* = 2.4 Hz, 6H).

### Step 9: Preparation of intermediate 1-1A-10

Intermediate **1-1A-9** (9.52 g, 21.1 mmol) was dissolved in trifluoroacetic acid (192 mL), heated to 75°C, and stirred for 16 hours. The reaction mixture was added with water (20.0 mL), adjusted to pH = 9, and extracted with dichloromethane (20.0 mL). The organic phase was dried over anhydrous sodium sulfate, filtered, and concentrated under reduced pressure to obtain a crude product. The crude product was stirred with n-heptane (6 mL) at 25°C for 2 hours to obtain the trifluoroacetate of **1-1A-10.** MS: m/z = 333.1, [M+1]⁺. ¹H NMR (400 MHz, CDCl₃) δ 7.77 (d, *J =* 7.6 Hz, 2H), 7.56 (d, *J =* 7.2 Hz, 2H), 7.41 (t, *J =* 7.6 Hz, 2H), 7.33 (t, *J* = 6 Hz, 2H), 6.18-6.27 (m, 2H), 4.38-4.42 (m, 2H), 4.23 (s, 1H), 3.88 (d, *J* = 2.0 Hz, 2H), 3.82 (d, *J=* 2.4 Hz, 1H), 3.72 (d, *J =* 2.0 Hz, 1H), 3.21-3.60 (m, 2H).

### Step 10: Preparation of intermediate 1-1A-11

The trifluoroacetate of intermediate 1-1A-10 (1.00 g, 2.92 mmol) was dissolved in tetrahydrofuran (10.0 mL), and then di-tert-butyl dicarbonate (764 mg, 3.50 mmol) and triethylamine (885 mg, 8.75 mmol) were successively added thereto, and stirred at 25°C for 1 hour. The reaction mixture was extracted by adding ethyl acetate (10.0 mL * 2) and water (10.0 mL). The organic phases were combined, washed with saturated brine (15.0 mL), dried over anhydrous sodium sulfate, filtered, and concentrated under reduced pressure to obtain a crude product. The crude product was purified by column chromatography (petroleum ether/ethyl acetate = 1/0 to 0/1, petroleum ether: ethyl acetate = 3:1) to obtain intermediate 1-**1A-11.** MS:m/z = 433.2, [M+1]⁺.

### Step 11: Preparation of intermediate 1-1A

Intermediate **1-1A-11** (5.69 g, 12.59 mmol) was dissolved in ethanol (60.0 mL), and then dimethylamine (34.4 g, 251.8 mmol) was added thereto. The reaction system was stirred at 25°C for 3 hours. The reaction mixture was concentrated directly under reduced pressure. The residue was extracted with ethyl acetate (40.0 mL) and 10% citric acid (40.0 mL). The pH value of the aqueous phase was adjusted to 9. The aqueous phase was filtered and extracted with ethyl acetate (40.0 mL * 2). The organic phases were combined, dried over anhydrous sodium sulfate, filtered, and concentrated under reduced pressure to obtain intermediate **1-1A.** MS: m/z = 211.2, [M+1]⁺. ¹H NMR (400 MHz, CDCl₃) δ 6.22 (d, *J =* 10 Hz, 2H), 4.40 (d, *J =* 38.8 Hz, 2H), 2.89-3.01 (m, 2H), 2.40 (d, *J =* 13.2 Hz, 2H), 1.49 (s, 9H).

### Step 12: Preparation of intermediate 1-3A-2

Intermediate **1-3A-1** (4 g, 17.02 mmol) was dissolved in AcOH (30 mL), and cooled to 0°C in an ice bath, then concentrated sulfuric acid (17.03 g, 170.21 mmol) was added thereto, and then an aqueous solution (5 mL) of sodium nitrite (1.76 g, 25.53 mmol) was added dropwise thereto, and the mixture was stirred for 0.25 hours. An aqueous solution (5 mL) of potassium iodide (4.24 g, 25.53 mmol) was then added dropwise thereto, and the reaction mixture was transferred to 25°C and reacted for 0.5 hours. The reaction mixture was added with water (50 mL) and filtered. The solid was washed with saturated sodium thiosulfate solution (2 * 40 mL) and water (40 mL). The solid was dissolved with dichloromethane (50 mL), dried over anhydrous sodium sulfate, filtered, and concentrated to obtain intermediate 1-**3A-2.**

### Step 13: Preparation of intermediate 1-3A-3

Intermediate **1-3A-2** (2.85 g, 8.24 mmol) and cuprous iodide (3.14 g, 16.48 mmol) were dissolved in *N,N*-dimethylformamide (45 mL), and then methyl 2,2-difluoro-2-(fluorosulfonyl)acetate (6.33 g, 32.96 mmol) was added thereto, and the reaction was carried out at 80°C for 1 hour. The reaction was carried out for another 0.75 hours. The reaction mixture was cooled to 25°C. The insoluble substances were filtered off and rinsed with ethyl acetate (100 mL). The filtrate was washed with water (3 * 200 mL), dried over anhydrous sodium sulfate, and concentrated. The crude product was purified by column chromatography (petroleum ether/ethyl acetate = 30/1 to 20/1) to obtain intermediate **1-3A-3.**

### Step 14: Preparation of intermediate 1-3A-4

Intermediate **1-3A-3** (1.66 g, 5.76 mmol), iron powder (1.13 g, 20.17 mmol), and ammonium chloride (1.54 g, 28.82 mmol) were dissolved in ethanol (20 mL) and water (10 mL), heated to 60°C, and reacted for 2.5 hours. Ethyl acetate (80 mL) was added to the reaction mixture for dilution. The insoluble substances were filtered off. The filtrate was washed with water (2 * 50 mL), dried over anhydrous sodium sulfate, filtered, and concentrated to obtain intermediate **1-3A-4.** MS: m/z =257.9, [M+1]⁺.

### Step 15: Preparation of intermediate 1-3A

Intermediate **1-3A-4** (1.4 g, 5.43 mmol), bis(pinacolato)diboron (2.07 g, 8.14 mmol, 1.5 eq), 1,1'-bis(diphenylphosphino)ferrocene-palladium(II)dichloride dichloromethane complex (443.12 mg, 542.61 µmol), and potassium acetate (1.60 g, 16.28 mmol) were dissolved in dioxane (30 mL), and reacted at 85°C for 16 hours under nitrogen atmosphere. The reaction mixture was cooled to 25°C. The insoluble substances were filtered off and rinsed with ethyl acetate (50 mL). The filtrate was concentrated. The crude product was purified by column chromatography (petroleum ether/ethyl acetate = 20/1 to 10/1) to obtain intermediate **1-3A.** MS: m/z =306.0, [M+1]⁺.

### Step 16: Preparation of intermediate 1-2

Intermediate **1-1** (900 mg, 3.56 mmol) was dissolved in dichloromethane (10 mL), cooled to 0°C, and then *N,N*-diisopropylethylamine (1.38 g, 10.69 mmol) and **1-1A** (749.60 mg, 3.56 mmol) were successively added thereto, and the reaction was carried out at 0°C for 1 hour. The reaction mixture was directly concentrated under reduced pressure to obtain crude product **1-2.** MS: m/z = 426.0, [M+1]⁺.

### Step 17: Preparation of intermediate 1-3

Intermediates **1-2** (220 mg, 516.10 µmol) and **1-2A** were dissolved in acetonitrile (10 mL), then *N,N-*diisopropylethylamine (200.10 mg, 1.55 mmol) was added thereto, and the reaction system was heated to 80°C and stirred for 16 hours. The reaction mixture was concentrated under reduced pressure to remove most of the solvent, and then added with ethyl acetate (10 mL) and washed with water (5 mL) for extraction, dried over anhydrous sodium sulfate, and concentrated. The crude product was separated by silica gel column chromatography (PE/EA= 1/1 to DCM/MeOH = 20/1) to obtain intermediate **1-3.** MS: m/z = 549.1, [M+1]⁺.

### Step 18: Preparation of intermediate 1-4

Intermediate **1-3A** (0.2 g, 364.29 µmol), intermediate **1-3** (222.27 mg, 728.58 µmol), 1,1'-bis(diphenylphosphino)ferrocene-palladium(II)dichloride dichloromethane complex (29.75 mg, 36.43 µmol), and cesium carbonate (356.08 mg, 1.09 mmol) were dissolved in dioxane (5 mL) and water (1.25 mL), and reacted at 90°C for 17 hours under nitrogen atmosphere. The reaction mixture was cooled to 25°C. The insoluble substances were filtered off and rinsed with ethyl acetate (50 mL). The filtrate was dried over anhydrous sodium sulfate and concentrated. The crude product was separated by silica gel column chromatography (DCM/MeOH = 50/1 to 20/1) to obtain intermediate **1-4.** MS: m/z = 692.2, [M+1]⁺.

### Step 19: Preparation of compound 1

Intermediate **1-4** (92 mg, 133.01 µmol) was dissolved in dichloromethane (4 mL), then trifluoroacetic acid (1.54 g, 13.51 mmol, 1 mL) was added thereto at 20°C, and the reaction was carried out for 0.5 hours. The reaction mixture was concentrated to dryness, dissolved with dichloromethane (2 mL), and stirred thoroughly with sodium bicarbonate solid (0.5 g). Ethyl acetate (5 mL) was added thereto, and the reaction mixture was stirred for another 5 minutes. The insoluble substances were filtered off, and the filtrate was concentrated. The crude product was separated by preparative HPLC (chromatographic column: Boston Green ODS 150 * 30 mm * 5 µm; mobile phase: [water (formic acid)-acetonitrile]; acetonitrile%: 10% to 40%, 6 min) to obtain compound 1. MS (ESI) m/z: 592.3 [M+1]⁺. ¹H NMR (400 MHz, CD₃OD) δ 9.01 (s, 1H), 6.58 (d, *J=* 14 Hz, 1H), 6.41 (s, 1H), 6.34 (s, 2H), 5.56 (d, *J=* 51 Hz, 1H), 4.79 - 4.75 (m, 2H), 4.62 - 4.61 (m, 2H), 4.72 (s, 2H), 4.03 - 3.82 (m, 5H), 3.31 - 3.30 (m, 1H), 2.59 - 2.54 (m, 2H), 2.38 - 2.29 (m, 4H).

### Example 2

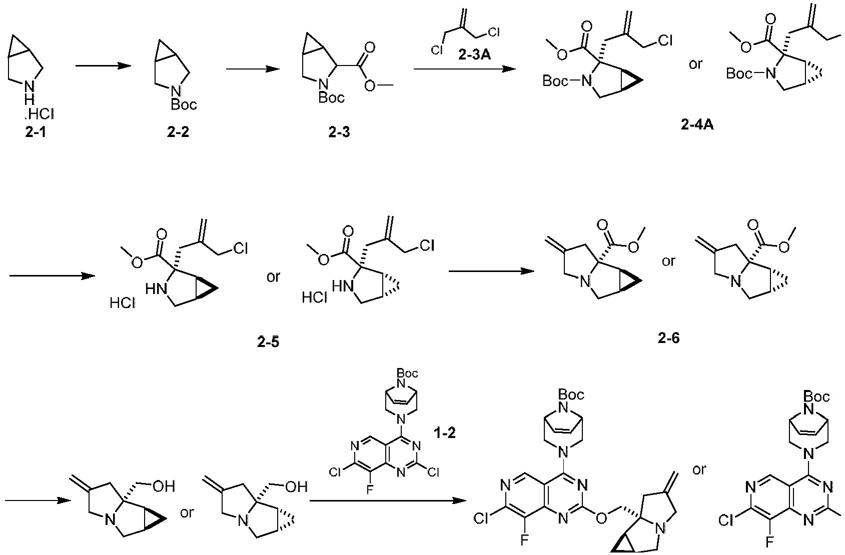

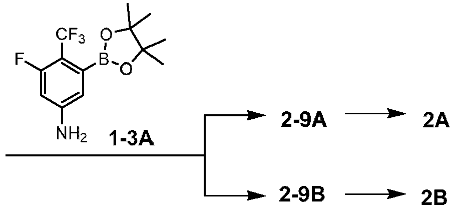

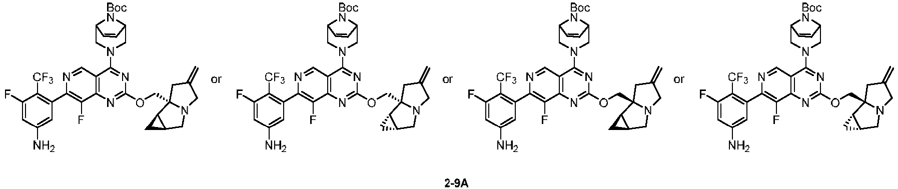

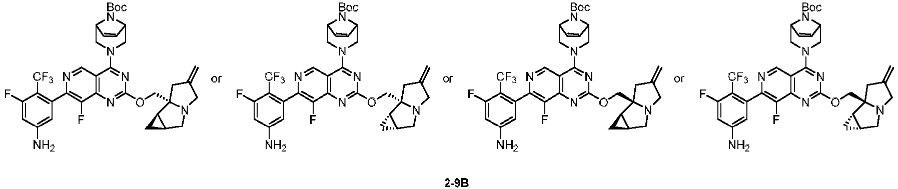

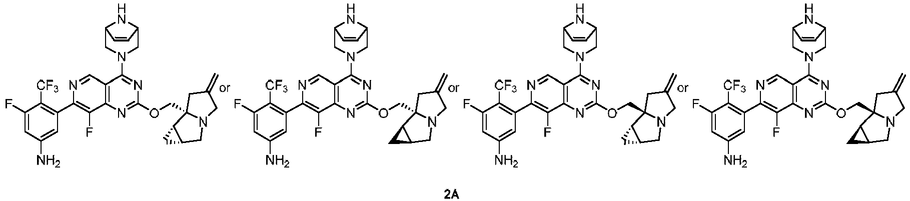

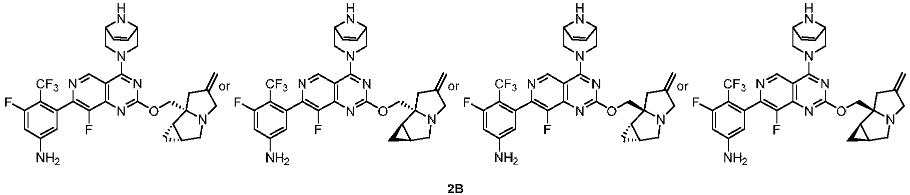

### Step 1: Preparation of intermediate 2-2

Intermediate **2-1** (50 g, 418.09 mmol) was dissolved in dichloromethane (500 mL), and triethylamine (84.61 g, 836.17 mmol) was added thereto, then di-tert-butyl dicarbonate (100.37 g, 459.90 mmol) was added thereto, and the reaction was carried out at 25°C for 16 hours. 100 mL of water was added to the reaction mixture, and the phases were separated. The organic phase was dried over anhydrous sodium sulfate, filtered, and concentrated. The crude product was separated by silica gel column chromatography (PE/EA = 100/1 to 50/1) to obtain intermediate **2-2.** MS: m/z = 206.1, [M+Na]⁺.

### Step 2: Preparation of intermediate 2-3

Intermediate **2-2** (25 g, 136.43 mmol) was dissolved in anhydrous tetrahydrofuran (350 mL), and 3,7-dipropyl-3,7-diazabicyclo[3.3.1]nonane (37.31 g, 177.36 mmol) was added thereto, and cooled to -65°C. Then sec-butyllithium (1.3 M, 157.42 mL) was slowly added dropwise thereto. After reacting for 1 hour, methyl chloroformate (15.73 g, 166.44 mmol) was added dropwise thereto, and the reaction was carried out at -65°C for 2 hours. The reaction mixture was quenched by adding saturated ammonium chloride (20 mL) dropwise, and extracted with ethyl acetate (300 mL * 2). The organic phases were combined, washed with saturated brine (150 mL), dried over anhydrous sodium sulfate, filtered, and concentrated. The crude product was separated by silica gel column chromatography (PE/EA = 50/1 to 25/1) to obtain intermediate **2-3.** MS: m/z = 186.0 [M-tBu+H]⁺.

### Step 3: Preparation of intermediate 2-4A

Intermediate **2-3** (12 g, 49.73 mmol) was dissolved in THF (120 mL), then 2-chloromethyl-3-chloropropene (24.87 g, 198.94 mmol) was added thereto, and cooled to -40°C. Lithium bis(trimethylsilyl)amide (1 M, 99.47 mL) was slowly added dropwise thereto, and the reaction mixture was gradually warmed to 20°C and reacted for 2 hours. The reaction mixture was quenched by adding saturated ammonium chloride (20 mL) dropwise, and extracted with ethyl acetate (150 mL * 2). The organic phases were combined, washed with saturated brine (80 mL), dried over anhydrous sodium sulfate, filtered, and concentrated. The crude product was separated by silica gel column chromatography (PE/EA = 20/1 to 10/1) to obtain intermediate **2-4A.** After developing twice by TLC thin-layer chromatography (petroleum ether: acetone = 5:1), the Rf value of compound **2-4A** was 0.5, and the Rf value of its isomer was 0.55. The retention time of **2-4A** on LCMS (chromatographic column: Agilent Poroshell 120 EC-C18 2.7 µm, 3.0 * 30 mm, mobile phase: A: H₂O (0.037% FA)-B: ACN (0.0187% FA); B: 5% to 95%) was 0.776 min, and the retention time of its isomer was 0.801 min. MS: m/z = 274.0 [M-tBu+H]⁺.

### Step 4: Preparation of intermediate 2-5

Intermediate **2-4A** (3.6 g, 10.92 mmol) was dissolved in hydrogen chloride/ethyl acetate (4 M, 27.29 mL) and reacted at 20°C for 2 hours. The reaction mixture was concentrated under reduced pressure to obtain crude intermediate **2-5,** which was directly used to the next step without purification. MS: m/z = 230.1, [M+H]⁺.

### Step 5: Preparation of intermediate 2-6

Intermediate **2-5** (2.9 g, 10.90 mmol) was dissolved in methanol (100 mL), then potassium carbonate (4.52 g, 32.69 mmol) was added thereto, and the reaction was carried out at 20°C for 2 hours. The reaction mixture was added with dichloromethane (80 mL), filtered, and concentrated. The crude product was separated by silica gel column chromatography (PE/EA= 10/1 to 5/1) to obtain intermediate **2-6.** MS: m/z = 194.1, [M+H]⁺.

### Step 6: Preparation of intermediate 2-7

Intermediate **2-6** (1.6 g, 8.28 mmol) was dissolved in anhydrous tetrahydrofuran (20 mL), then lithium aluminum hydride (628.51 mg, 16.56 mmol) was added thereto, and the reaction was carried out at 0°C for 2 hours. The reaction mixture was diluted by adding ethyl acetate (10 mL) dropwise, added with water (0.63 mL) dropwise, dried over anhydrous sodium sulfate, filtered, and concentrated to obtain crude intermediate **2-7,** which was directly used to the next step without purification. MS: m/z = 166.1 [M+H]⁺.

### Step 7: Preparation of intermediate 2-8

Intermediate **1-2** (700 mg, 1.64 mmol) and intermediate **2-7** (407.00 mg, 2.46 mmol) were dissolved in anhydrous toluene (20 mL), and then sodium tert-butoxide (473.45 mg, 4.93 mmol) was added thereto. The reaction was carried out at 0°C for 2 hours, and then at 20°C for 2 hours. The reaction mixture was added with ethyl acetate (50 mL), quenched by adding 10 mL of water, and the phases were separated. The organic phase was washed with saturated brine (10 mL), dried over anhydrous sodium sulfate, filtered, and concentrated. The crude product was separated by silica gel column chromatography (PE/EA = 5/1 to 2/1) to obtain intermediate **2-8.** MS: m/z = 555.1, [M+H]⁺.

### Step 8: Preparation of intermediates 2-9A and 2-9B

Intermediate **2-8** (0.1 g, 180.17 µmol, 1 eq) and intermediate **1-3A** (82.45 mg, 270.26 µmol) were dissolved in dioxane (2 mL) and water (0.5 mL), and then methanesulfonato(diadamantyl-*n*-butylphosphino)-2'-amino-1,1'-biphenyl-2-yl)palladium(II) (13.12 mg , 18.02 µmol) and cesium carbonate (146.75 mg, 450.42 µmol) were added thereto, and the reaction was carried out at 90°C for 16 hours. The reaction mixture was diluted with ethyl acetate (30 mL), washed with saturated brine (10 mL), dried over anhydrous sodium sulfate, filtered, and concentrated. The crude product was subjected to silica gel column chromatography (PE/EA = 1/50 to 1/100) and then separated by preparative SFC (chromatographic column: REGIS(S,S)WHELK-O1 (250 mm * 25 mm, 10 µm); mobile phase: A: CO₂, B: [0.1% NH₃H₂O IPA]; B%: 45% to 45%) to obtain intermediates **2-9A** and **2-9B.** The retention time of intermediate **2-9A** in analytical SFC (chromatographic column: (S,S)Whelk-01 100 × 4.6 mm I.D., 5.0 µm, mobile phase: A: supercritical carbon dioxide, B: IPA (0.05% DEA), gradient: 40% B, flow rate: 2.5mL/min) was 3.276 min, and the ee value was 98.3%. The retention time of compound **2-9B** in analytical SFC (chromatographic column: (S,S)Whelk-01 100 × 4.6 mm I.D., 5.0 µm, mobile phase: A: supercritical carbon dioxide, B: IPA (0.05% DEA), gradient: 40% B, flow rate: 2.5 mL/min) was 3.879 min, and the ee value was 96.4%.

### Step 9: Preparation of compound 2A

Intermediate **2-9A** (30 mg, 43.00 µmol) was dissolved in dichloromethane (1.5 mL), then trifluoroacetic acid (147.08 mg, 1.29 mmol) was added thereto, and the reaction was carried out at 20°C for 2 hours. The reaction mixture was diluted with dichloromethane (20 mL), added with saturated sodium bicarbonate (10 mL), and the phases were separated. The aqueous phase was extracted with dichloromethane (10 mL), and the organic phases were combined, dried over anhydrous sodium sulfate, filtered, and concentrated. The crude product was purified by preparative HPLC (chromatographic column: O-Welch C18 150 * 30 mm * 5 µm; mobile phase: [H₂O(FA)-ACN]; B%: 1% to 41%, 10 min) to obtain the formate of compound **2A.** LCMS: MS (ESI) m/z: 598.2 [M+H]⁺. ¹H NMR (400 MHz, CD₃OD) δ 9.07 (s, 1H), 8.52 (br s, 1H), 6.59 (br d, J=14.05 Hz, 1H), 6.38-6.48 (m, 1H), 6.33 (s, 2H), 5.10-5.26 (m, 2H), 4.59-4.72 (m, 3H), 4.49 (d, J=11.04 Hz, 1H), 4.27 (br s, 2H), 4.08 (br d, J=15.31 Hz, 1H), 3.94 (br d, J=11.04 Hz, 2H), 3.62 (br d, J=15.81 Hz, 1H), 3.53 (d, J=10.54 Hz, 1H), 3.00-3.14 (m, 2H), 2.75 (br d, J=16.31 Hz, 1H), 1.86-1.98 (m, 1H), 1.79 (td, J=3.67, 6.96 Hz, 1H), 0.69-0.85 (m, 2H).

### Step 10: Preparation of compound 2B

Intermediate **2-9B** (31 mg, 44.43 µmol) was dissolved in dichloromethane (1.5 mL), then trifluoroacetic acid (42.31 mg, 371.05 µmol) was added thereto, and the reaction was carried out at 20°C for 1 hour. The reaction mixture was diluted with dichloromethane (20 mL), added with saturated sodium bicarbonate (10 mL), and the phases were separated. The aqueous phase was extracted with dichloromethane (10 mL), and the organic phases were combined, dried over anhydrous sodium sulfate, filtered, and the filtrate was concentrated. The crude product was purified by preparative HPLC (chromatographic column: O-Welch C18 150 * 30 mm * 5 µm; mobile phase: [H₂O(FA)-ACN]; B%: 1% to 41%, 12 min) to obtain the formate of compound **2B.** LCMS: MS (ESI) m/z: 598.2 [M+H]⁺. ¹H NMR (400 MHz, CD₃OD) δ 9.06 (s, 1H), 8.52 (br s, 1H), 6.59 (br d, J=13.80 Hz, 1H), 6.43 (s, 1H), 6.33 (s, 2H), 5.16 (br d, J=10.29 Hz, 2H), 4.58-4.72 (m, 3H), 4.48 (d, J=11.29 Hz, 1H), 4.25 (br s, 2H), 4.07 (br d, J=15.56 Hz, 1H), 3.94 (br d, J=11.04 Hz, 2H), 3.61 (br d, J=15.81 Hz, 1H), 3.51 (d, J=10.54 Hz, 1H), 2.99-3.12 (m, 2H), 2.74 (br d, J=16.06 Hz, 1H), 1.88-1.95 (m, 1H), 1.72-1.85 (m, 1H), 0.69-0.81 (m, 2H).

### Example 3

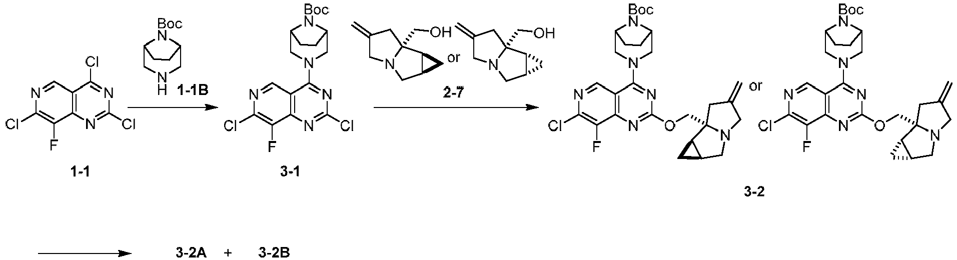

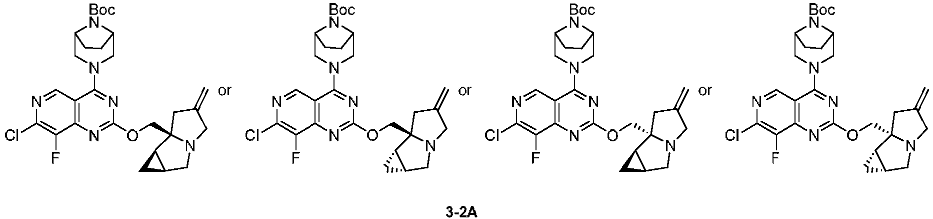

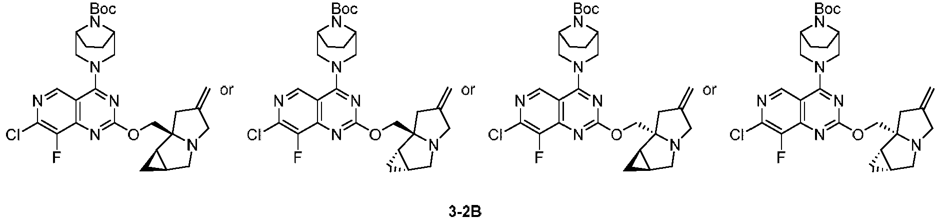

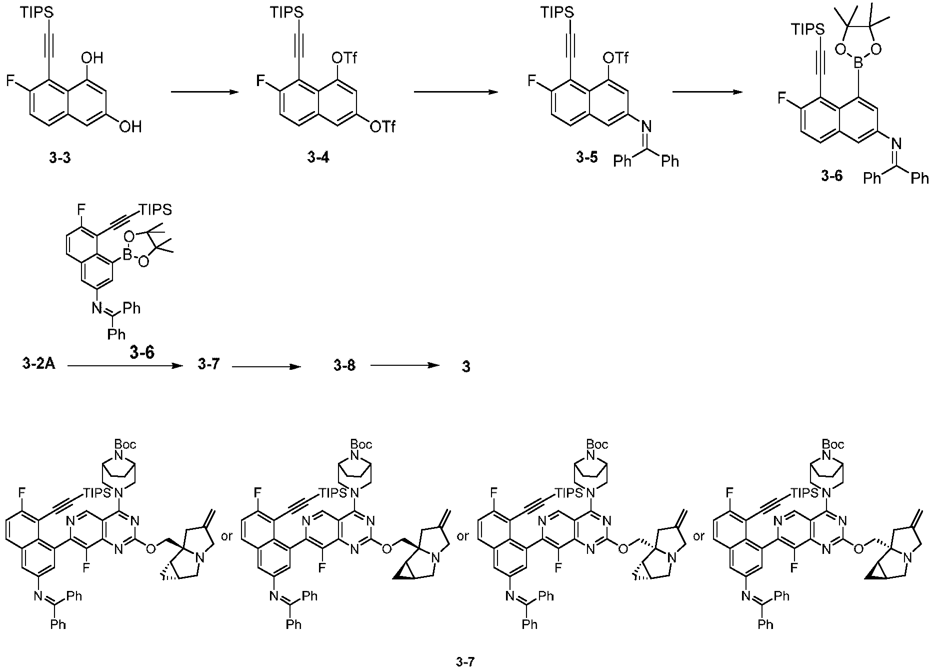

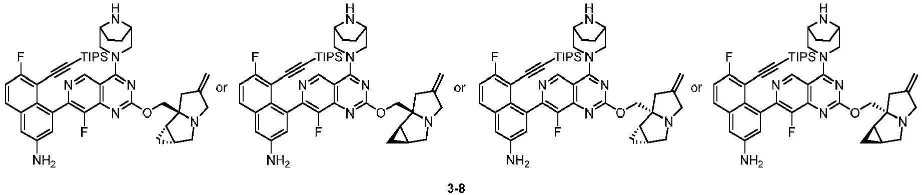

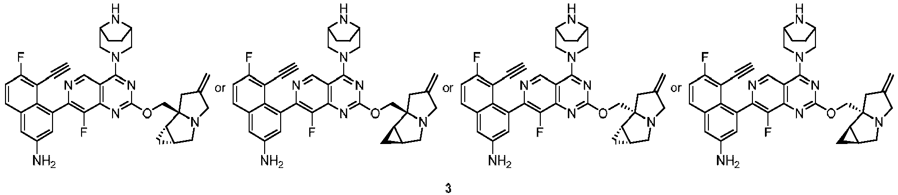

### Step 1: Preparation of intermediate 3-1

Intermediate **1-1** (25 g, 99.03 mmol) was dissolved in dichloromethane (200 mL), cooled to -30°C, and then *N,N-*diisopropylethylamine (38.40 g, 297.08 mmol) and **1-1B** (21.02 g, 99.03 mmol) were successively added thereto, and the reaction was carried out at -30°C for 2 hours. The reaction mixture was directly concentrated under reduced pressure to obtain crude product 3-1. MS: m/z = 428.1 [M+1]⁺.

### Step 2: Preparation of intermediate 3-2A

Intermediate **3-1** (23 g, 53.70 mmol) and intermediate **2-7** (9.76 g, 59.07 mmol) were dissolved in anhydrous toluene (300 mL), and then sodium *tert*-butoxide (13.93 g, 145.00 mmol) was slowly added thereto at 0°C. The reaction was carried out at 0°C for 0.5 hours, and then at 20°C for 0.5 hours. The reaction mixture was diluted by adding 200 mL of ethyl acetate, washed with saturated brine (100 mL), dried over anhydrous sodium sulfate, filtered, and the filtrate was concentrated under reduced pressure to obtain crude product **3-2.** The crude product was purified by preparative SFC (chromatographic column: DAICEL CHIRALCEL OD (250 mm * 50 mm, 10 µm); mobile phase: [A (supercritical carbon dioxide), B (ethanol containing 0.1% ammonia)]; B: 40%) to obtain products **3-2A** and **3-2B.** The retention time of **3-2A** under the conditions of analytical SFC (chromatographic column: Cellulose 2 100 mm * 4.6 mm, 3 µm), mobile phase: [A (supercritical carbon dioxide), B (MeOH (0.05% DEA))]; B: 40%) was 4.964 min, and the ee value was 95.7%. ¹H NMR (400MHz, CD₃OD) δ 8.76 (s, 1H), 4.98 - 4.87 (m, 2H), 4.51 - 4.48 (m, 3H), 4.34 - 4.29 (m, 1H), 4.27 (br s, 2H), 4.23 - 4.18 (m, 1H), 3.61 - 3.58 (m, 3H), 3.12 (d, J=9.5 Hz, 1H), 2.78 (br d, J=16.8 Hz, 1H), 2.71 (dd, J=4.0, 9.6 Hz, 1H), 2.45 (br d, J=16.8 Hz, 1H), 1.83 - 1.72 (m, 2H), 1.75 - 1.62 (m, 3H), 1.55 - 1.51 (m, 1H), 1.42 (s, 9H), 0.60 (q, J=4.2 Hz, 1H), 0.47 -0.42 (m, 1H). MS: m/z = 557.2 [M+1]⁺. The retention time of **3-2B** under the same conditions was 8.382 min, and the ee value was 96.5%.

### Step 3: Synthesis of intermediate 3-4

The raw material **3-3** (2 g, 5.58 mmol) was dissolved in dichloromethane (40 mL), and *N,N-*diisopropylethylamine (4.33 g, 33.47 mmol) was added thereto under an ice bath at 0 to 10°C, and then trifluoromethanesulfonic anhydride (6.30 g, 22.31 mmol) was added dropwise thereto, and the reaction was carried out for 1.5 hours. Water (20 mL) was added to the reaction mixture and stirred thoroughly, then the aqueous phase was separated. The organic phase was dried over anhydrous sodium sulfate and concentrated. The crude product was separated by column chromatography (mobile phase: petroleum ether: ethyl acetate = 10:1 to 2:1) to obtain intermediate **3-4.**

### Step 4: Synthesis of intermediate 3-5

Intermediate **3-4** (3 g, 4.82 mmol), benzophenone imine (1.75 g, 9.64 mmol), 4,5-bis(diphenylphosphino)-9,9-dimethylxanthene (557.57 mg, 963.63 µmol), and cesium carbonate (4.71 g, 14.45 mmol) were dissolved in toluene (60 mL), then tris(dibenzylideneacetone)dipalladium (441.21 mg, 481.82 µmol) was added thereto, and the reaction was carried out at 100°C for 2 hours under nitrogen atmosphere. The reaction mixture was cooled to 25°C. The insoluble substances were filtered off. The filtrate was concentrated to remove most of the toluene, and the residue was diluted with ethyl acetate (30 mL), washed with water (20 mL), dried over anhydrous sodium sulfate, and concentrated to obtain a crude product. The crude product was separated by column chromatography (mobile phase: petroleum ether: ethyl acetate = 10:1 to 1:1) to obtain intermediate **3-5.**

### Step 5: Synthesis of intermediate 3-6

Intermediate **3-5** (3.1 g, 4.74 mmol), bis(pinacolato)diboron (2.41 g, 9.48 mmol), [1,1'-bis(diphenylphosphino)ferrocene]dichloropalladium(II) (693.88 mg, 948.30 µmol), and potassium acetate (1.40 g, 14.22 mmol) were dissolved in toluene (60 mL), and reacted at 110°C for 18 hours under nitrogen atmosphere. The reaction mixture was cooled to 25°C. The insoluble substances were filtered off and extracted with ethyl acetate (50 mL). The filtrate was washed with water (50 mL), dried over anhydrous sodium sulfate, and concentrated. The crude product was separated by column chromatography (mobile phase: petroleum ether: ethyl acetate = 10:1 to 2:1) to obtain intermediate **3-6.** MS (ESI) m/z: 468.2 [M+H₂O-Ph₂CO+1]⁺.

### Step 6: Synthesis of intermediate 3-7

Intermediate **3-2A** (11.3 g, 20.29 mmol) was added to water (60 mL) and anhydrous dioxane (240 mL), and then methanesulfonato(diadamantyl-*n*-butylphosphino)-2'-amino-1,1'-biphenyl-2-yl)palladium(II) (1.48 g, 2.03 mmol), intermediate 3-6 (15.38 g, 24.34 mmol), and cesium carbonate (13.22 g, 40.57 mmol) were added thereto, and the reaction was carried out at 87°C for 2 hours. The reaction mixture was diluted by adding ethyl acetate (200 mL), washed with saturated brine (40 mL x 2), dried over anhydrous sodium sulfate, and filtered. The filtrate was concentrated under reduced pressure to obtain a crude product. The crude product was separated by column chromatography (mobile phase: dichloromethane: methanol = 50:1 to 20:1) to obtain intermediate **3-7.** MS (ESI) m/z: 862.4 [M+H₂O-Ph₂CO+1]⁺.

### Step 7: Synthesis of intermediate 3-8

Intermediate **3-7** (8.4 g, 8.18 mmol) was added to ethyl acetate (30 mL) and water (10 mL), then hydrochloric acid/ethyl acetate (4 M, 62.37 mL) was added thereto, and the reaction was carried out for 2 hours at 20°C. Water (30 mL) was added to the reaction mixture. The organic phase was washed with water (30 mL x 2). The aqueous phases were combined, adjusted to pH = 7 to 8 with saturated sodium bicarbonate, and extracted with ethyl acetate (80 mL x 3). The organic phases were combined, dried over anhydrous sodium sulfate, filtered, and the filtrate was concentrated under reduced pressure to obtain crude product **3-8,** which was used directly in the next step. MS (ESI) m/z: 762.3 [M+1]⁺.

### Step 8: Synthesis of compound 3

Intermediate **3-8** (6.2 g, 8.14 mmol) was added to acetonitrile (70 mL), then tetramethylammonium fluoride (2.29 g, 13.83 mmol) was added thereto, and the reaction was carried out at 60°C for 1 hour. The reaction mixture was diluted by adding 100 mL of ethyl acetate, washed with 30 mL of saturated sodium bicarbonate, dried over anhydrous sodium sulfate, and filtered. The filtrate was concentrated under reduced pressure to obtain a crude product. The crude product was purified by column chromatography (mobile phase: dichloromethane (10% ammonia in methanol solution): methanol = 20:1 to 10:1) to obtain compound 3. SFC analysis (chromatographic column: Chiralcel OJ-3 100 * 4.6 mm, 3 µm; mobile phase: A (supercritical carbon dioxide) and B (ethanol, containing 0.05% diethylamine); gradient: B%: 40% to 40%) showed a retention time of 3.761 min and an ee value of 97.34%. Chiral HPLC analysis (chromatographic column: FLM Chiral NQ, 150 * 4.6 mm, 3 µm; mobile phase: A: (*n*-hexane) and B: (ethanol, containing 0.2% diethylamine, v/v); gradient: B%: isocratic 30%; elution time: 60 min; column temperature: 35°C) showed a retention time of 17.387 min. MS (ESI) m/z: 606.3 [M+1]⁺,¹H NMR (400 MHz, CD₃OD) δ = 9.01 (s, 1H), 7.76 (dd, J = 5.6, 9.2 Hz, 1H), 7.28 - 7.19 (m, 2H), 7.15 (d, J= 2.3 Hz, 1H), 5.08 (br s, 1H), 5.01 (br s, 1H), 4.70 - 4.54 (m, 3H), 4.43 (dd, J = 7.3, 10.0 Hz, 1H), 4.32 (dd, J = 6.3,10.3 Hz, 1H), 3.78 - 3.65 (m, 5H), 3.39 - 3.34 (m, 1H), 3.23 (d, J = 9.5 Hz, 1H), 2.93 (br d, J = 17.3 Hz, 1H), 2.82(dd, J = 4.0, 9.3 Hz, 1H), 2.56 (br d, J = 17.1 Hz, 1H), 1.91 - 1.76 (m, 5H), 1.64 (td, J = 3.6, 6.9 Hz, 1H), 0.72 (q, J =4.0 Hz, 1H), 0.61 - 0.53 (m, 1H).

### Example 4

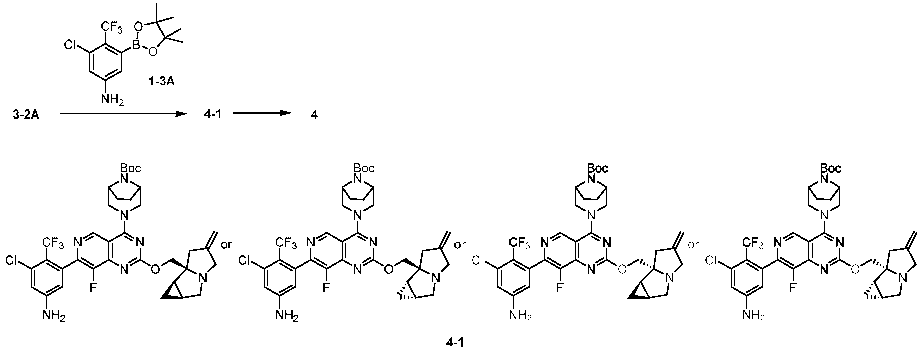

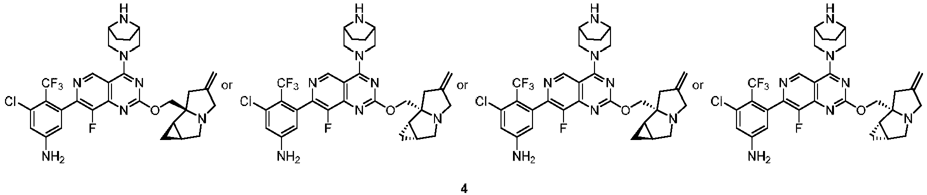

### Step 1: Synthesis of intermediate 4-1

Intermediate **3-2A** (150 mg, 269.27 µmol), intermediate **1-3A** (86.58 mg, 269.27 µmol), methanesulfonato(diadamantyl-n-butylphosphino)-2'-amino-1,1'-biphenyl-2-yl)palladium(II) (98.05 mg, 134.64 µmol), and cesium carbonate (263.20 mg, 807.82 µmol) were dissolved in dioxane (8 mL). The reaction mixture was replaced with nitrogen for three times, then added with water (0.5 mL), and reacted at 80°C for 6 hours under nitrogen atmosphere. The reaction mixture was filtered to remove insoluble substances, washed with water (10 mL), extracted with ethyl acetate (30 mL * 2), washed with saturated brine (10 mL), dried over anhydrous sodium sulfate, and concentrated under reduced pressure to remove the organic solvent to obtain a crude product, which was purified by column chromatography (mobile phase: dichloromethane: methanol = 100:1 to 50:1) to obtain intermediate **4-1.** MS (ESI) m/z: 716.3 [M+1]⁺.

### Step 2: Synthesis of compound 4

Intermediate **4-1** (22 mg, 30.1 µmol) was dissolved in dichloromethane (5 mL), then trifluoroacetic acid (1.5 mL) was added thereto, and the reaction was carried out at 25°C for 3 hours. The pH of the system was adjusted to 8 by adding saturated sodium bicarbonate solution, then extracted by adding dichloromethane (5 mL * 2), dried over anhydrous sodium sulfate, and concentrated under reduced pressure to remove the organic solvent to obtain a crude product. The crude product was separated by prep-HPLC (chromatographic column: O-Welch C18 150 * 30 mm * 5 µm; mobile phase: [A: water (0.5% formic acid)-B: acetonitrile]; B%: 8% to 48%, 8 min) to obtain the formate of compound **4.** MS (ESI) m/z: 616.2 [M+1]⁺. ¹H NMR (400 MHz, CD₃OD) δ = 9.06 (s, 1H), 8.51 (br s, 1H), 6.94 (s, 1H), 6.52 (s, 1H), 5.16 (br d, J=10.79 Hz, 2H), 4.71-4.80 (m, 2H), 4.67 (br d, J=10.79 Hz, 1H), 4.53 (br d, J=11.04 Hz, 1H), 4.02-4.10 (m, 2H), 3.84-3.90 (m, 2H), 3.59-3.63 (m, 1H), 3.50-3.53 (m, 1H), 3.03-3.09 (m, 2H), 2.73-2.78 (m, 1H), 1.96-2.09 (m, 4H), 1.90-1.93 (m, 1H), 1.75-1.81 (m, 1H), 0.87-0.97 (m, 1H), 0.68-0.83 (m, 2H).

### Example 5

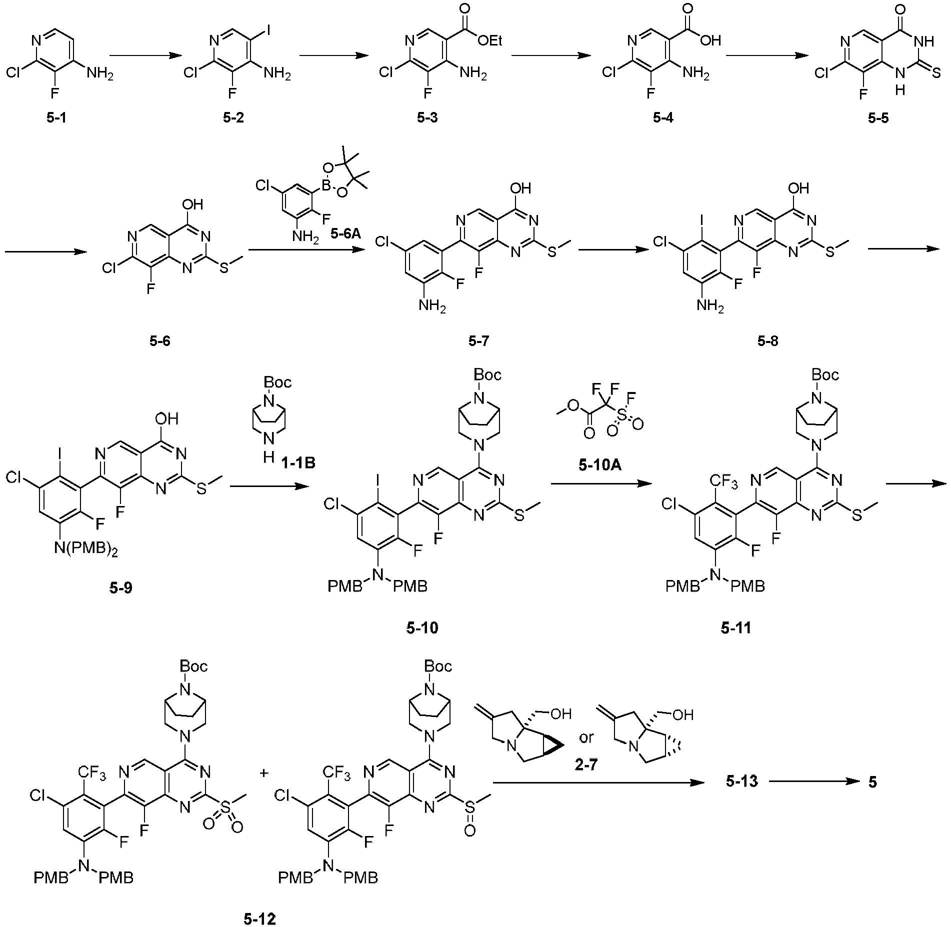

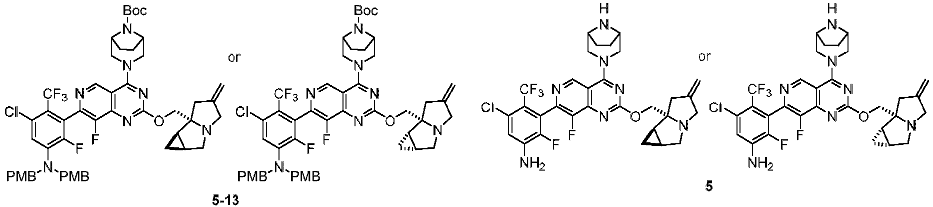

### Step 1: Synthesis of compound 5-2

Under nitrogen atmosphere, compound **5-1** (21 g, 143.30 mmol) was dissolved in acetonitrile (210 mL), then *N*-iodosuccinimide (38.69 g, 171.96 mmol) and*p*-toluenesulfonic acid monohydrate (1.36 g, 7.16 mmol) were added thereto, and the reaction was carried out at 75°C for 5 hours. Water (150 mL) was slowly added thereto, and the aqueous phase was extracted with ethyl acetate (150 mL * 3), and the phases were separated. The organic phases were combined, successively washed with saturated sodium sulfite solution (15 mL) and saturated brine (100 mL), dried over anhydrous sodium sulfate, filtered, and concentrated under reduced pressure to obtain crude product **5-2.** MS (ESI) m/z: 272.9 [M+1]⁺.

### Step 2: Synthesis of compound 5-3

Compound **5-2** (39.3 g) was dissolved in ethanol (435 mL), then triethylamine (43.79 g, 432.75 mmol) and bis(triphenylphosphine)palladium(II) chloride (10.12 g, 14.42 mmol) were added thereto. The reaction mixture was replaced with CO for three times, then reacted at 80°C for 47 hours under CO atmosphere (50 Psi). The reaction mixture was concentrated under reduced pressure, added with ethyl acetate (300 mL) and 0.3 M hydrochloric acid (300 mL) to precipitate solids, and filtered to obtain a filter cake. The phases of the filtrate were separated, and the aqueous phase was extracted with ethyl acetate (200 mL * 2), and the phases were separated. The organic phases were combined, washed with saturated brine (200 mL), dried over anhydrous sodium sulfate, filtered, and the filter cake was combined with the organic phase and concentrated under reduced pressure to obtain crude product **5-3.** ¹H NMR (400 MHz, CDCl₃) δ 8.54 (s, 1H), 4.39 (q, J = 7.1 Hz, 2H), 1.41 (t, J = 7.1 Hz, 3H).

### Step 3: Synthesis of compound 5-4

Under nitrogen atmosphere, compound **5-3** (41.3 g, 188.92 mmol) was dissolved in tetrahydrofuran (240 mL), then methanol (80 mL) and water (80 mL) were added thereto, and lithium hydroxide monohydrate (23.78 g, 566.76 mmol) was added thereto in batches, and then the reaction was carried out at 20°C for 5 hours. The reaction system was concentrated under reduced pressure. Water (100 mL) was added to the reaction system, and the pH of the aqueous phase was adjusted to 2 with 4 M hydrochloric acid to precipitate solids, and the mixture was filtered. The filter cake was dissolved in ethanol (200 mL), stirred at 20°C for 16 hours, filtered, and the filter cake was dried under reduced pressure to obtain a crude product. The crude product was dissolved in petroleum ether (20 mL) and ethyl acetate (30 mL), stirred at 20°C for 1 hour, filtered, and the filter cake was dried under reduced pressure to obtain compound **5-4.** ¹H NMR (400 MHz, DMSO-*d*₆) δ 13.95 - 13.08 (m, 1H), 8.35 (s, 1H), 7.62 (br s, 2H).

### Step 4: Synthesis of compound 5-5

Under nitrogen atmosphere, compound **5-4** (17.8 g, 93.41 mmol) was dissolved in phosphorus oxychloride (234.25 g, 1.53 mol), and then the reaction was carried out at 95°C for 3 hours. The reaction system was concentrated under reduced pressure. Tetrahydrofuran (360 mL) was added thereto, and then ammonium thiocyanate (21.33 g, 280.23 mmol) was added thereto in batches at 20°C, and the reaction was carried out at 40°C for 16 hours. Water (300 mL) was added to the reaction system, and the aqueous phase was extracted with ethyl acetate (300 mL * 3), and then the phases were separated. The organic phases were combined, washed with saturated brine (300 mL), dried over anhydrous sodium sulfate, filtered, and concentrated under reduced pressure to obtain a crude product. Ethyl acetate (40 mL) and petroleum ether (20 mL) were added to the crude product, stirred at 20°C for 0.5 hours, and filtered. The filter cake was dried under reduced pressure to obtain compound **5-5.** ¹H NMR (400 MHz, DMSO-*d*₆) δ 13.33 (br s, 1H), 12.91 (s, 1H), 8.64 (s, 1H).

### Step 5: Synthesis of compound 5-6

Under nitrogen atmosphere, compound 5-5 (8.55 g, 36.91 mmol) was dissolved in N,N-dimethylformamide (140 mL), and sodium methoxide (2.09 g, 38.76 mmol) was added thereto at 20°C, then iodomethane (4.72 g, 33.22 mmol) was slowly added dropwise thereto at 20°C, and the reaction was carried out at 20°C for 5 hours. Ice water (300 mL) was added to the reaction system to precipitate solids. The mixture was filtered, and the filter cake was washed with ice water (100 mL). The filter cake was dried under reduced pressure to obtain compound **5-6.** ¹H NMR (400 MHz, DMSO-*d*₆) δ 13.27 (br s, 1H), 8.81 (s, 1H), 2.61 (s, 3H).

### Step 6: Synthesis of compound 5-7

Under nitrogen atmosphere, compound **5-6** (3.5 g, 14.25 mmol) and compound **5-6A** (3.98 g, 14.67 mmol) were dissolved in dioxane (70 mL), water (7 mL), and ethanol (14 mL). Potassium phosphate (9.07 g, 42.74 mmol) and chloro(2-dicyclohexylphosphino-2',4',6'-triisopropyl-1,1'-biphenyl)[2-(2'-amino-1,1'-biphenyl)]palladium(II) (1.68 g, 2.14 mmol) were added thereto, and then the reaction was carried out at 105°C for 1.5 hours. Water (200 mL) was added to the reaction system, and the aqueous phase was extracted with ethyl acetate (200 mL * 2), and then the phases were separated. Ethyl acetate (200 mL) was added to the aqueous phase to make it turbid, filtered through diatomite, and the phases of the filtrate were separated. The aqueous phase was extracted with ethyl acetate (200 mL * 2), and the phases were separated. All organic phases were combined, and the organic phase was washed with saturated brine (100 mL), dried over anhydrous sodium sulfate, filtered, and concentrated under reduced pressure to obtain a crude product. The crude product was separated by column chromatography (developing solvent: petroleum ether: ethyl acetate = 20:1 to 1:5, dichloromethane: methanol = 10:1) to obtain compound 5-7. ¹H NMR (400 MHz, DMSO-*d*₆) δ 13.19 (br s, 1H), 9.03 (s, 1H), 6.92 (dd, J = 2.6, 7.3 Hz, 1H), 6.70 (dd, J = 2.7, 4.9 Hz, 1H), 5.71 (s, 2H), 2.62 (s, 3H).

### Step 7: Synthesis of compound 5-8

Under nitrogen atmosphere, compound **5-7** (2.1 g, 5.92 mmol) was dissolved in ethanol (63 mL), and silver sulfate (2.21 g, 7.10 mmol) and iodine (1.58 g, 6.22 mmol) were added thereto at 0°C, and then the reaction was slowly warmed to 10°C and reacted for 2 hours. Saturated sodium sulfite solution (70 mL) and ethyl acetate (100 mL) were added to the reaction system, filtered, and the phases were separated. The aqueous phase was extracted with ethyl acetate (100 mL * 3), and the phases were separated. The organic phases were combined, dried over anhydrous sodium sulfate, filtered, and concentrated under reduced pressure to obtain a crude product. The crude product was separated by column chromatography (developing solvent: petroleum ether: ethyl acetate = 20:1 to 1:5) to obtain compound **5-8.** ¹H NMR (400 MHz, CDCl₃) δ 10.47 (br s, 1H), 9.34 (s, 1H), 7.10 (d, J = 8.1 Hz, 1H), 2.78 (s, 3H).

### Step 8: Synthesis of compound 5-9

Sodium hydride (96.11 mg, 2.40 mmol) was added to a solution of compound **5-8** (0.33 g, 686.56 µmol) in *N*,*N*-dimethylformamide (5 mL) under nitrogen atmosphere at 0°C in an ice-water bath. After stirring for 30 minutes, 4-methoxybenzylchloride (236.55 mg, 1.51 mmol) was added dropwise thereto, and the resulting mixture was naturally warmed to 25°C and stirred for 14 hours. 20 mL of saturated ammonium chloride aqueous solution and 2 * 20 mL of ethyl acetate were added to the reaction mixture, and stirred for 10 minutes. The aqueous phase was removed, and the organic phase was concentrated under reduced pressure to obtain crude compound **5-9.**

### Step 9: Synthesis of compound 5-10

Under nitrogen atmosphere, compound **5-9** (0.11 g) and *N,N*-diisopropylethylamine (59.16 mg, 457.73 µmol) were dissolved in tetrahydrofuran (2.7 mL), and 1*H*-benzotriazol-1-yloxytri(1-pyrrolidinyl)phosphonium hexafluorophosphate (95.28 mg, 183.09 µmol) was added thereto at 10°C, and the reaction was carried out at 10°C for 1 hour. Compound **1-1B** (38.87 mg, 183.09 µmol) was then added thereto, and the reaction was carried out at 10°C for 17 hours. Water (10 mL) was added to the reaction system, and the phases were separated. The aqueous phase was extracted with dichloromethane (10 mL * 3), and the phases were separated. The organic phases were combined, washed with saturated brine (10 mL), dried over anhydrous sodium sulfate, filtered, and the filtrate was concentrated under reduced pressure to obtain a crude product. The crude product was separated by column chromatography (developing solvent: ethyl acetate/petroleum ether = 4.0% to 20.0%) to obtain compound **5-10.** MS (ESI) m/z: 915.0 [M+1]⁺.

### Step 10: Synthesis of compound 5-11

Under nitrogen atmosphere, compound **5-10** (0.15 g, 163.89 µmol) and cuprous iodide (156.07 mg, 819.47 µmol) were dissolved in *N*,*N*-dimethylformamide (3.75 mL), then compound **5-10A** (314.86 mg, 1.64 mmol) was added thereto, and the reaction was carried out at 80°C for 2 hours. Water (10 mL) and ethyl acetate (20 mL) were slowly added to precipitate solids, and the mixture was filtered. The phases of the filtrate were separated, and the aqueous phase was extracted with ethyl acetate (10 mL * 3), and the phases were separated. The organic phases were combined, washed with saturated brine (5 mL), dried over anhydrous sodium sulfate, filtered, and concentrated under reduced pressure to obtain a crude product. The crude product was separated by column chromatography (developing solvent: ethyl acetate/petroleum ether = 4.0% to 20.0%) to obtain compound **5-11.** MS (ESI) m/z: 857.1 [M+1]⁺.

### Step 11: Synthesis of compound 5-12

Under nitrogen atmosphere, compound **5-11** (0.06 g, 69.98 µmol) was dissolved in tetrahydrofuran (1.2 mL) and water (0.4 mL), then potassium peroxymonosulfate (82.38 mg, 489.89 µmol) was added thereto at 0°C, and the reaction mixture was slowly warmed to 10°C and reacted for 4 hours. Additional potassium peroxymonosulfate (35.31 mg, 209.95 µmol) was added thereto, and the reaction was carried out at 10°C for 2 hours. Saturated sodium sulfite solution (5 mL) was slowly added thereto, and the aqueous phase was extracted with ethyl acetate (10 mL * 3), and then the phases were separated. The organic phases were combined, washed with saturated brine (5 mL), dried over anhydrous sodium sulfate, filtered, and concentrated under reduced pressure to obtain compound **5-12.** MS (ESI) m/z: 889.2 [M+1]⁺; MS (ESI) m/z: 873.2 [M+1]⁺.

### Step 12: Synthesis of compound 5-13

Under nitrogen atmosphere, compound **5-12** (0.05 g, 56.22 µmol) and compound **2-7** (13.93 mg, 84.33 µmol) were dissolved in tetrahydrofuran (1.5 mL), and sodium *tert*-butoxide (10.81 mg, 112.44 µmol) was added thereto at 0°C. The reaction mixture was slowly warmed to 10°C and reacted for 3 hours. Water (5 mL) and ethyl acetate (5 mL) were added to the reaction system, filtered, and the phases of the filtrate were separated. The aqueous phase was extracted with ethyl acetate (5 mL * 3), and the phases were separated. The organic phases were combined, washed with saturated brine (5 mL), dried over anhydrous sodium sulfate, filtered, and concentrated under reduced pressure to obtain compound **5-13.** MS (ESI) m/z: 974.3 [M+1]⁺.

### Step 13: Synthesis of compound 5

Under nitrogen atmosphere, compound **5-13** (0.1 g, 102.62 µmol) was dissolved in dichloromethane (2.5 mL), then trifluoroacetic acid (468.04 mg, 4.10 mmol) was added thereto, and the reaction was carried out at 10°C for 6 hours. The reaction system was concentrated under reduced pressure to obtain a crude product. The crude product was separated by high-performance liquid chromatography twice to obtain compound **5.** HPLC preparation method 1: chromatographic column: Phenomenex Luna 75 * 30 mm * 3 µm; mobile phase A: water (0.04% hydrochloric acid), mobile phase B: acetonitrile; running gradient: B%: 1% to 43%, run for 8 min. HPLC preparation method 2: chromatographic column: Waters Xbridge BEH 100 * 30 mm * 10 µm; mobile phase A: water (10 mM ammonium bicarbonate), mobile phase B: acetonitrile; running gradient: B%: 30% to 60%, run for 8 min. MS (ESI) m/z: 634.2 [M+1]⁺.

### Example 6

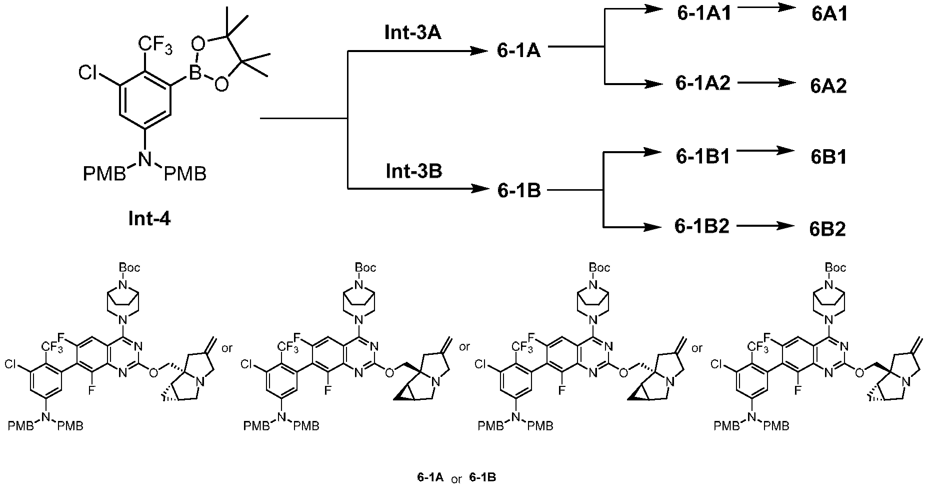

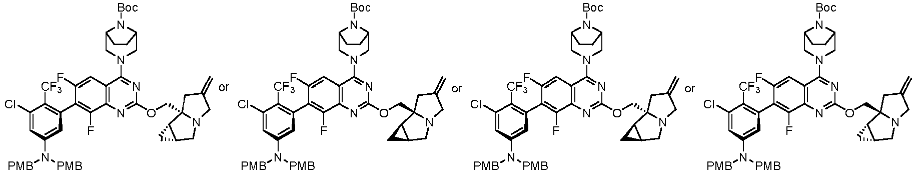

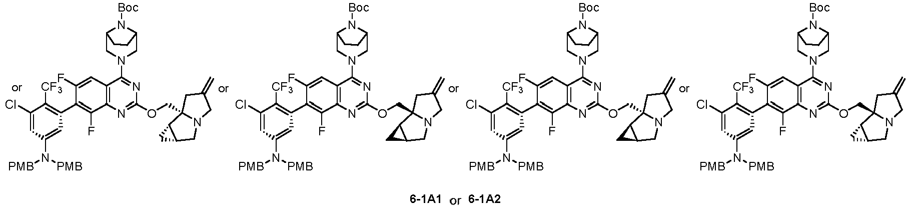

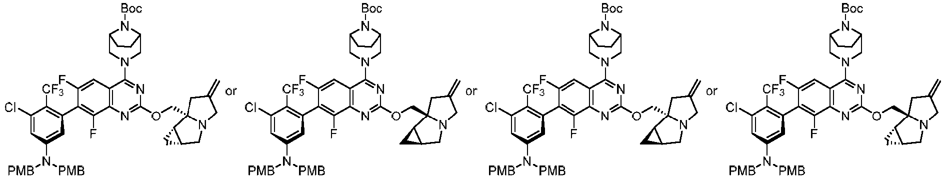

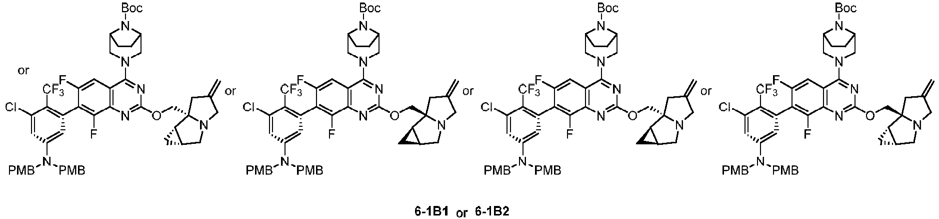

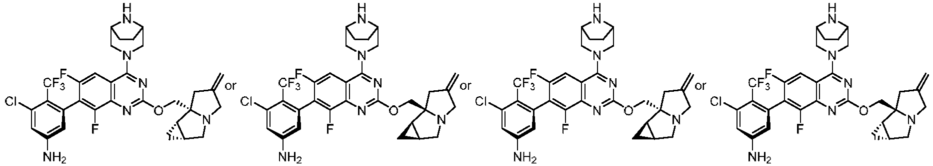

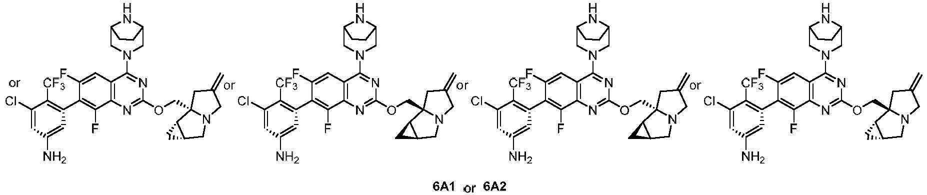

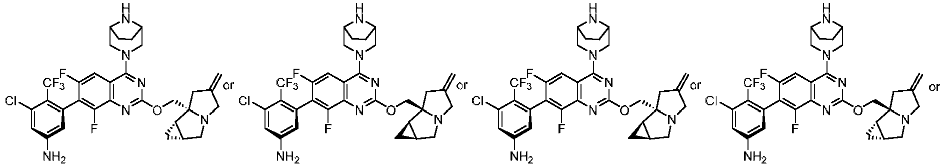

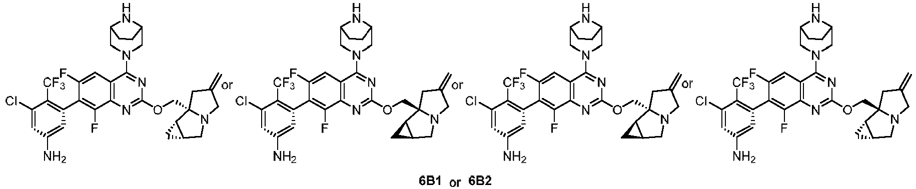

### Step 1:

Under nitrogen atmosphere, intermediates **Int-3A** (0.2 g, 323.36 µmol) and **Int-4** (181.67 mg, 323.36 µmol) were dissolved in tetrahydrofuran (8 mL) and water (2 mL), and potassium phosphate (137.28 mg, 646.71 µmol) and chloro(2-dicyclohexylphosphino-2',6'-dimethoxy-1,1'-biphenyl)[2-(2'-amino-1,1'-biphenyl)]palladium(II) (23.30 mg, 32.34 µmol) were added thereto, and then the reaction was carried out at 50°C for 2 hours. Water (5 mL) was added to the reaction system, and the aqueous phase was extracted with ethyl acetate (8 mL * 3), and then the phases were separated. The organic phase was washed with saturated brine (10 mL), dried over anhydrous sodium sulfate, filtered, and concentrated under reduced pressure to obtain a crude product. The crude product was purified by high-performance liquid chromatography (chromatographic column: Phenomenex luna C18 100 * 40 mm * 5 µm; mobile phase: A (acetonitrile) and B (water, containing 0.04% hydrochloric acid); gradient: B%: 50% to 80%, 8 min) to obtain compound **6-1A.** MS m/z: 973.1 [M+1]⁺.

Referring to step 1, intermediate **Int-3B** was used as the raw material to obtain compound **6-1B.** MS m/z: 973.1 [M+1]⁺.

### Step 2:

Compound **6-1A** (0.2 g, 205.45 µmol) was purified by preparative SFC (chromatographic column: ChiralPak IH, 250 * 30 mm, 10 µm; mobile phase: A (supercritical CO₂) and B (isopropanol, containing 0.1% ammonia); gradient: B%: 60% to 60%, 10 min) to obtain compounds **6-1A1** and **6-1A2,** respectively.

Compound **6-1A1:** The retention time under analytical SFC (column: ChiralPak IH-3, 50 * 4.6 mm, 3 µm; mobile phases: A (supercritical CO₂) and B (isopropanol, containing 0.1% isopropylamine); gradient: B%:5% to 5%, 3 min) was 1.463 min with an ee value of 97.49%. ¹H NMR (400 MHz, CDCl₃) δ = 7.30 (s, 1H), 7.10 (d, *J* = 8.8 Hz, 4H), 6.94 (d, *J* = 2.0 Hz, 1H), 6.88 (d, *J* = 8.8 Hz, 4H), 6.52 (d, *J* = 2.0 Hz, 1H), 5.25 (s, 2H), 5.04 (d, *J* = 11.2 Hz, 1H), 4.69 (d, *J* = 10.8 Hz, 1H), 4.55 (s, 4H), 4.52 - 4.44 (m, 2H), 4.44 - 4.32 (m, 3H), 4.12 - 4.02 (m, 1H), 3.81 (s, 6H), 3.63 (d, *J =* 15.6 Hz, 2H), 3.56 - 3.43 (m, 1H), 3.36 (d, *J =* 17.6 Hz, 1H), 3.19 - 3.08 (m, 1H), 2.77 (d, *J =* 17.6 Hz, 1H), 2.07 - 1.87 (m, 4H), 1.85 - 1.77 (m, 2H), 1.51 (s, 9H), 1.26 (s, 1H), 0.91 - 0.84 (m, 1H). MS m/z: 973.1 [M+1]⁺.

Compound **6-1A2:** The retention time under analytical SFC (column: ChiralPak IH-3, 50 * 4.6 mm, 3 µm; mobile phases: A (supercritical CO₂) and B (isopropanol, containing 0.1% isopropylamine); gradient: B%:5% to 5%, 3 min) was 1.983 min with an ee value of 95.66%. ¹H NMR (400 MHz, CDCl₃) δ = 7.30 (s, 1H), 7.10 (d, *J* = 8.8 Hz, 4H), 6.94 (d, *J=* 2.4 Hz, 1H), 6.88 (d, *J* = 8.4 Hz, 4H), 6.51 (d, *J* = 2.4 Hz, 1H), 5.22 (s, 2H), 5.06 - 4.83 (m, 1H), 4.77 - 4.62 (m, 1H), 4.54 (s, 4H), 4.49 - 4.24 (m, 5H), 4.13 - 3.94 (m, 1H), 3.81 (s, 6H), 3.70 - 3.46 (m, 3H), 3.39 - 3.23 (m, 1H), 3.18 - 3.02 (m, 1H), 2.80 - 2.64 (m, 1H), 2.09 - 1.89 (m, 4H), 1.85 - 1.76 (m, 2H), 1.52 (s, 9H), 1.32 - 1.25 (m, 1H), 0.90 - 0.75 (m, 1H). MS m/z: 973.1 [M+1]⁺.

### Step 3:

Compound **6-1B** (0.2 g, 205.45 µmol) was purified by preparative SFC (column: ChiralPak IH, 250 * 30 mm, 10 µm; mobile phase: A (supercritical CO₂) and B (methanol, containing 0.1% ammonia); gradient: B%: 40% to 40%, 11 min) to obtain compound **6-1B1** and compound **6-1B2,** respectively.

Compound **6-1B1:** The retention time under analytical SFC (column: ChiralPak IH-3, 50 * 4.6 mm, 3 µm; mobile phases: A (supercritical CO₂) and B (isopropanol, containing 0.1% isopropylamine); gradient: B%:5% to 5%, 3 min) was 1.330 min with an ee value of 94.45%. ¹H NMR (400 MHz, CDCl₃) δ = 7.26 (s, 1H), 7.10 (d, *J* = 8.4 Hz, 4H), 6.94 (d, *J =* 2.0 Hz, 1H), 6.88 (d, *J =* 8.4 Hz, 4H), 6.51 (d, *J =* 2.4 Hz, 1H), 5.01 (s, 1H), 4.93 (s, 1H), 4.54 (s, 4H), 4.42 - 4.16 (m, 6H), 3.81 (s, 6H), 3.65 - 3.50 (m, 3H), 3.33 - 3.19 (m, 2H), 2.94 (d, *J=* 16.0 Hz, 1H), 2.75 (d, *J =* 6.4 Hz, 1H), 2.46 (dd, *J =* 15.6, 2.0 Hz, 1H), 2.00 - 1.92 (m, 2H), 1.92 - 1.72 (m, 4H), 1.52 (s, 9H), 0.78 - 0.69 (m, 1H), 0.54 - 0.42 (m, 1H). MS m/z: 973.1 [M+1]⁺.

Compound **6-1B2:** The retention time under analytical SFC (column: ChiralPak IH-3, 50 * 4.6 mm, 3 µm; mobile phases: A (supercritical CO₂) and B (isopropanol, containing 0.1% isopropylamine); gradient: B%:5% to 5%, 3 min) was 1.495 min with an ee value of 98.01%. ¹H NMR (400 MHz, CDCl₃) δ = 7.25 (s, 1H), 7.10 (d, *J* = 8.4 Hz, 4H), 6.93 (s, 1H), 6.88 (d, *J* = 8.4 Hz, 4H), 6.51 (s, 1H), 5.01 (s, 1H), 4.92 (s, 1H), 4.53 (s, 4H), 4.39 - 4.24 (m, 5H), 4.17 (d, *J =* 8.8 Hz, 1H), 3.81 (s, 6H), 3.64 - 3.49 (m, 3H), 3.33 - 3.19 (m, 2H), 2.95 (d, *J =* 18.8 Hz, 1H), 2.75 (d, *J =* 4.8 Hz, 1H), 2.46 (d, *J =* 16.4 Hz, 1H), 1.99 - 1.89 (m, 2H), 1.88 - 1.72 (m, 4H), 1.52 (s, 9H), 0.74 (d, *J =* 1.6 Hz, 1H), 0.53 - 0.43 (m, 1H). MS m/z: 973.1 [M+1]⁺.

### Step 4:

### Compound 6A1:

Under nitrogen atmosphere, compound **6-1A1** (0.07 g, 71.91 µmol) was dissolved in dichloromethane (3.5 mL), and trifluoroacetic acid (1.07 g, 9.42 mmol) was added thereto, and then the reaction was carried out at 20°C for 3 hours. The reaction mixture was concentrated under reduced pressure to obtain a crude product. The crude product was purified by high-performance liquid chromatography (chromatographic column: Waters Xbridge BEH C18 100 * 30 mm * 10 µm; mobile phase: A (acetonitrile) and B (water, containing 10 mM ammonium bicarbonate); gradient: B%: 35% to 70%) to obtain compound **6A1.** ¹H NMR (400 MHz, CDCl₃) δ = 7.31 (d, *J =* 9.6 Hz, 1H), 6.87 (s, 1H), 6.42 (s, 1H), 5.03 (s, 1H), 4.94 (s, 1H), 4.44 - 4.26 (m, 3H), 4.26 - 4.11 (m, 3H), 3.66 (s, 2H), 3.53 (t, *J =* 11.6 Hz, 2H), 3.37 - 3.23 (m, 2H), 2.98 (d, *J =* 16.8 Hz, 1H), 2.77 (dd, *J =* 8.4, 2.8 Hz, 1H), 2.47 (d, *J =* 16.4 Hz, 1H), 2.23 - 2.04 (m, 2H), 1.91 - 1.81 (m, 4H), 1.56 (s, 1H), 1.28 (d, *J =* 10.8 Hz, 1H), 0.89 (t, *J =* 7.2 Hz, 1H), 0.57 - 0.44 (m, 1H). MS m/z: 633.1 [M+1]⁺.

### Compound 6A2:

Under nitrogen atmosphere, compound **6-1A2** (0.07 g, 71.91 µmol) was dissolved in dichloromethane (3.5 mL), and trifluoroacetic acid (1.07 g, 9.42 mmol) was added thereto, and then the reaction was carried out at 20°C for 3 hours. The reaction mixture was concentrated under reduced pressure to obtain a crude product. The crude product was purified by high-performance liquid chromatography (chromatographic column: Waters Xbridge BEH C18 100 * 30 mm * 10 µm; mobile phase: A (acetonitrile) and B (water, containing 10 mM ammonium bicarbonate); gradient: B%: 35% to 70%) to obtain compound **6A2.** ¹H NMR (400 MHz, CDCl₃) δ = 7.30 (d, *J =* 10.0 Hz, 1H), 6.87 (s, 1H), 6.42 (s, 1H), 5.02 (s, 1H), 4.93 (s, 1H), 4.42 (d, *J =* 12.0 Hz, 1H), 4.36 - 4.26 (m, 2H), 4.21 (d, *J =* 9.2 Hz, 3H), 3.65 (s, 2H), 3.61 - 3.54 (m, 1H), 3.47 (d, *J =* 12.4 Hz, 1H), 3.35 - 3.23 (m, 2H), 2.95 (d, *J =* 16.8 Hz, 1H), 2.84 - 2.72 (m, 1H), 2.46 (d, *J =* 16.4 Hz, 1H), 2.11 (br s, 2H), 1.92 - 1.80 (m, 4H), 1.63 - 1.51 (m, 1H), 1.40 - 1.19 (m, 1H), 0.85 - 0.70 (m, 1H), 0.60 - 0.42 (m, 1H). MS m/z: 633.2 [M+1]⁺.

### Step 5:

### Compound 6B1:

Under nitrogen atmosphere, compound **6-1B1** (0.06 g, 61.64 µmol) was dissolved in dichloromethane (3 mL), and trifluoroacetic acid (921.00 mg, 8.08 mmol) was added thereto, and then the reaction was carried out at 20°C for 2 hours. The reaction mixture was concentrated under reduced pressure to obtain a crude product. The crude product was purified by high-performance liquid chromatography (chromatographic column: Waters Xbridge Prep OBD C18 150 * 40 mm * 10 µm; mobile phase: A (acetonitrile) and B (water, containing 0.05% ammonia + 10 mM ammonium bicarbonate); gradient: B%: 30% to 70%) to obtain compound **6B1.** ¹H NMR (400 MHz, CDCl₃) δ = 7.31 (d, *J =* 9.6 Hz, 1H), 6.87 (s, 1H), 6.42 (s, 1H), 5.01 (s, 1H), 4.92 (s, 1H), 4.42 (d, *J =* 12.8 Hz, 1H), 4.33 - 4.24 (m, 2H), 4.22 - 4.17 (m, 1H), 4.14 (s, 2H), 3.63 (s, 2H), 3.58 (s, 1H), 3.54 (d, *J =* 12.4 Hz, 1H), 3.45 (d, *J =* 12.4 Hz, 1H), 3.32 - 3.21 (m, 2H), 2.95 (d, *J =* 16.8 Hz, 1H), 2.75 (dd, *J* = 8.8, 3.6 Hz, 1H), 2.46 (d, *J =* 17.2 Hz, 1H), 1.92 - 1.86 (m, 2H), 1.85 - 1.78 (m, 3H), 1.58 - 1.52 (m, 1H), 1.35 - 1.20 (m, 1H), 0.74 (q, *J =* 4.0 Hz, 1H), 0.53 - 0.44 (m, 1H). MS m/z: 633.2 [M+1]⁺.

### Compound 6B2:

Under nitrogen atmosphere, compound **6-1B2** (0.06 g, 61.64 µmol) was dissolved in dichloromethane (3 mL), and trifluoroacetic acid (921.00 mg, 8.08 mmol) was added thereto, and then the reaction was carried out at 20°C for 2 hours. The reaction mixture was concentrated under reduced pressure to obtain a crude product. The crude product was purified by high-performance liquid chromatography (chromatographic column: Waters Xbridge Prep OBD C18 150 * 40 mm * 10 µm; mobile phase: A (acetonitrile) and B (water, containing 0.05% ammonia + 10 mM ammonium bicarbonate); gradient: B%: 25% to 65%) to obtain compound **6B2.** ¹H NMR (400 MHz, CDCl₃) δ = 7.35 - 7.27 (m, 1H), 6.88 (s, 1H), 6.43 (s, 1H), 5.03 (s, 1H), 4.95 (s, 1H), 4.46 - 4.26 (m, 3H), 4.26 - 4.06 (m, 3H), 3.66 (s, 3H), 3.53 (t, *J =* 10.8 Hz, 2H), 3.29 (d, *J* = 11.2 Hz, 2H), 2.98 (d, *J* = 15.6 Hz, 1H), 2.78 (s, 1H), 2.48 *(d, J =* 14.8 Hz, 1H), 1.85 (s, 5H), 1.57 (s, 1H), 1.38 - 1.22 (m, 1H), 0.79 (s, 1H), 0.51 (s, 1H). MS m/z: 633.2 [M+1]⁺.

### Example 7

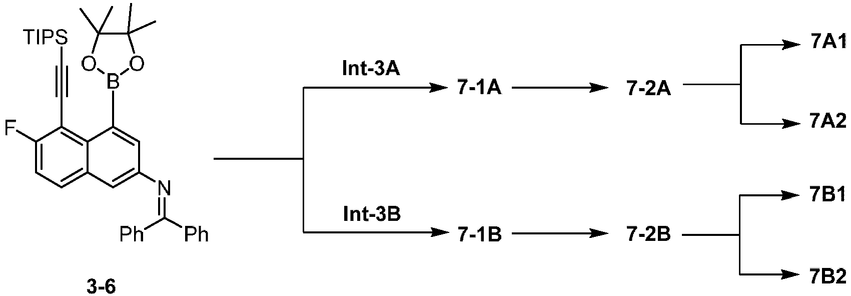

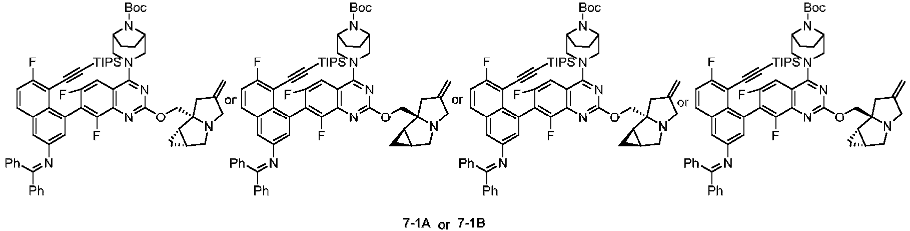

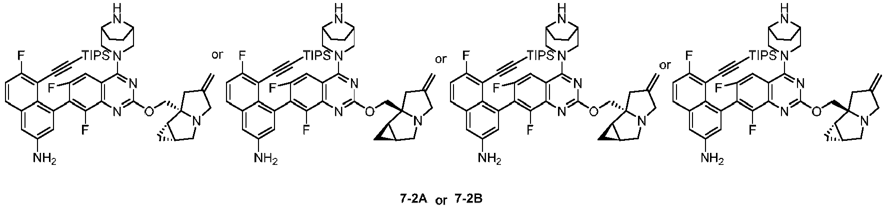

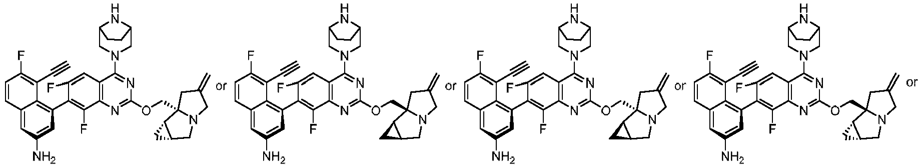

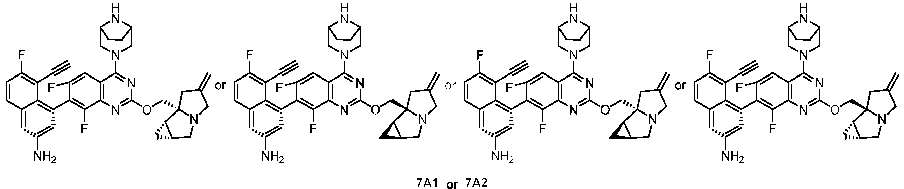

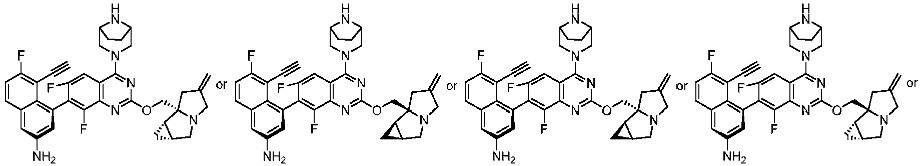

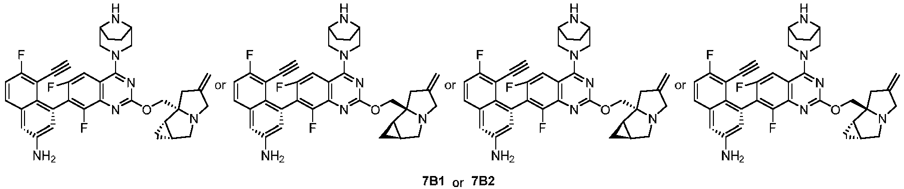

### Step 1:

Under nitrogen atmosphere, intermediate **Int-3A** (0.2 g, 323.36 µmol) and intermediate **3-6** (265.54 mg, 420.36 µmol) were dissolved in toluene (2 mL) and water (0.4 mL), and potassium phosphate (137.28 mg, 646.71 µmol) and chloro[(di(1-adamantyl)-N-butylphosphine)-2-(2-aminobiphenyl)]palladium(II) (32.43 mg, 48.50 µmol) were added thereto, and then the reaction was carried out at 90°C for 6 hours. Water (7 mL) was added to the reaction system, and the aqueous phase was extracted with ethyl acetate (7 mL * 3), and then the phases were separated. The organic phase was washed with saturated brine (10 mL), dried over anhydrous sodium sulfate, filtered, and concentrated under reduced pressure to obtain a crude product. The crude product was purified by high-performance liquid chromatography (chromatographic column: Phenomenex luna C18 250 * 50 mm * 10 µm; mobile phase: A (acetonitrile) and B (water, containing 0.04% hydrochloric acid); gradient: B%: 60% to 80%, 10 min) to obtain compound **7-1A.** MS m/z: 879.3 [M-C₁₃H₁₀O]⁺.

### Step 2:

Under nitrogen atmosphere, intermediate **Int-3B** (0.2 g, 323.36 µmol) and intermediate 3-6 (265.54 mg, 420.36 µmol) were dissolved in toluene (2 mL) and water (0.4 mL), and potassium phosphate (137.28 mg, 646.71 µmol) and chloro[(di(1-adamantyl)-N-butylphosphine)-2-(2-aminobiphenyl)]palladium(II) (32.43 mg, 48.50 µmol) were added thereto, and then the reaction was carried out at 90°C for 4 hours. Water (7 mL) was added to the reaction system, and the aqueous phase was extracted with ethyl acetate (7 mL * 3), and then the phases were separated. The organic phases were combined, washed with saturated brine (10 mL), dried over anhydrous sodium sulfate, filtered, and concentrated under reduced pressure to obtain a crude product. The crude product was purified by column chromatography (mobile phase: petroleum ether: ethyl acetate = 50:1 to 1:1) to obtain compound **7-1B.** MS m/z: 522.4 [M/2+1]⁺.

### Step 3:

Under nitrogen atmosphere, **7-1A** (0.19 g, 216.12 µmol) was dissolved in dichloromethane (7.5 mL), and trifluoroacetic acid (3.46 g, 30.36 mmol) was added thereto, and then the reaction was carried out at 20°C for 2 hours. The reaction mixture was concentrated under reduced pressure to obtain crude compound **7-2A.** MS m/z: 779.6 [M+1]⁺.

Referring to step 3, **7-1B** was used as the raw material to obtain compound **7-2B.** MS m/z: 779.6 [M+1]⁺.

### Step 4:

Under nitrogen atmosphere, compound **7-2A** (0.4 g, 143.58 µmol) was dissolved in N,N-dimethylformamide (4 mL), and cesium fluoride (2.18 g, 14.36 mmol) and sodium carbonate (91.31 mg, 861.50 µmol) were added thereto, and then the reaction was carried out at 20°C for 15 hours. Water (6 mL) was added to the reaction system, and the aqueous phase was extracted with ethyl acetate (7 mL * 3), and then the phases were separated. The organic phases were combined, washed with saturated brine (5 mL), dried over anhydrous sodium sulfate, filtered, and concentrated under reduced pressure to obtain a crude product. The crude product was separated by high-performance liquid chromatography (chromatographic column: Phenomenex Luna C18 75 * 30 mm * 3 µm; mobile phase: A (acetonitrile) and B (water, containing 0.04% formic acid); gradient: B%: 15% to 55%), and further purified by preparative SFC (column: DAICEL CHIRALPAK IG (250 mm * 30 mm, 10 µm); mobile phase: A (supercritical CO₂) and B (ethanol, containing 0.1% ammonia); gradient: B%: 50% to 50%, 11 min) to obtain compound **7A1** and compound **7A2,** respectively.

Compound **7A1:** The retention time under analytical SFC (column: ChiralPak IG-3, 50 * 4.6 mm, 3 µm; mobile phases: A (supercritical CO₂) and B (isopropanol, containing 0.1% isopropylamine); gradient: B%:5% to 5%, 3 min) was 1.693 min with an ee value of 97.92%. ¹H NMR (400 MHz, CDCl₃) δ = 7.67 (dd, *J* = 8.8, 5.6 Hz, 1H), 7.28 (s, 1H), 7.22 (t, *J* = 8.8 Hz, 1H), 7.12 (d, *J =* 2.0 Hz, 1H), 6.97 (d, *J =* 2. 0 Hz, 1H), 5.03 (s, 1H), 4.95 (s, 1H), 4.41 (d, *J = 12.4* Hz, 1H), 4.35 (d, *J =* 10.4 Hz, 2H), 4.23 (d, *J =* 10.0 Hz, 1H), 4.04 - 3.80 (m, 2H), 3.66 (s, 3H), 3.55 (d, *J* = 12.4 Hz, 1H), 3.51 (d, *J* = 12.0 Hz, 1H), 3.31 (d, *J =* 4.4 Hz, 1H), 3.28 (s, 1H), 3.01 (d, *J =* 16.8 Hz, 1H), 2.82 - 2.74 (m, 2H), 2.48 (d, *J =* 16.8 Hz, 1H), 1.90 - 1.86 (m, 5H), 1.60 - 1.53 (m, 1H), 1.32 - 1.19 (m, 1H), 0.80 (q, *J =* 4.4 Hz, 1H), 0.56 - 0.48 (m, 1H). MS m/z: 623.2 [M+1]⁺.

Compound **7A2:** The retention time under analytical SFC (column: ChiralPak IG-3, 50 * 4.6 mm, 3 µm; mobile phases: A (supercritical CO₂) and B (isopropanol, containing 0.1% isopropylamine); gradient: B%:5% to 5%, 3 min) was 2.236 min with an ee value of 98.26%. ¹H NMR (400 MHz, CDCl₃) δ = 7.67 (dd, *J* = 8.8, 6.0 Hz, 1H), 7.28 (s, 1H), 7.22 (t, *J* = 8.8 Hz, 1H), 7.25 - 7.19 (m, 1H), 7.12 (d, *J =* 2.0 Hz, 1H), 5.03 (s, 1H), 4.95 (s, 1H), 4.44 - 4.30 (m, 3H), 4.29 - 4.23 (m, 1H), 4.07 - 3.83 (m, 2H), 3.77 - 3.65 (m, 3H), 3.57 (t, *J =* 10.8 Hz, 2H), 3.32 (s, 1H), 3.29 (d, *J =* 5.2 Hz, 1H), 2.99 (d, *J =* 16.4 Hz, 1H), 2.83 - 2.73 (m, 2H), 2.49 (d, *J =* 16.8 Hz, 1H), 1.94 - 1.86 (m, 5H), 1.63 - 1.53 (m, 1H), 1.30 - 1.11 (m, 1H), 0.82 (d, *J =* 4.0 Hz, 1H), 0.53 (q, *J* = 8.0 Hz, 1H). MS m/z: 623.2 [M+1]⁺.

### Step 5:

Under nitrogen atmosphere, compound **7-2B** (0.55 g, 198.24 µmol) was dissolved in *N*,*N*-dimethylformamide (5 mL), then cesium fluoride (3.01 g, 19.82 mmol) and sodium carbonate (126.07 mg, 1.19 mmol) were added thereto, and the reaction was carried out for 15 hours at 20°C. Water (6 mL) was added to the reaction system, and the aqueous phase was extracted with ethyl acetate (7 mL * 3), and then the phases were separated. The organic phases were combined, washed with saturated brine (5 mL), dried over anhydrous sodium sulfate, filtered, and concentrated under reduced pressure to obtain a crude product. The crude product was separated by high-performance liquid chromatography (chromatographic column: Phenomenex Luna C18 75 * 30 mm * 3 µm; mobile phase: A (acetonitrile) and B (water, containing 0.04% formic acid); gradient: B%: 15% to 55%), and further purified by preparative SFC (column: DAICEL CHIRALPAK IG (250 mm * 30 mm, 10 µm); mobile phase: A (supercritical CO₂) and B (ethanol, containing 0.1% ammonia); gradient: B%: 55% to 55%, 7 min) to obtain compound **7B1** and compound **7B2,** respectively.

Compound **7B1:** The retention time under analytical SFC (column: ChiralPak IG-3, 50 * 4.6 mm, 3 µm; mobile phases: A (supercritical CO₂) and B (isopropanol, containing 0.1% isopropylamine); gradient: B%:5% to 5%, 3 min) was 1.745 min with an ee value of 98.56%. ¹H NMR (400 MHz, CDCl₃) δ = 7.67 (dd, *J* = 9.2, 6.0 Hz, 1H), 7.30 - 7.25 (m, 1H), 7.22 (t, *J* = 8.8 Hz, 1H), 7.11 (d, *J* = 1.6 Hz, 1H), 6.97 (d, *J* = 1.6 Hz, 1H), 5.03 (s, 1H), 4.94 (s, 1H), 4.38 (t, *J =* 14.0 Hz, 2H), 4.32 - 4.27 (m, 1H), 4.27 - 4.19 (m, 1H), 4.11 - 3.76 (m, 2H), 3.73 - 3.61 (m, 3H), 3.53 (t, *J =* 11.6 Hz, 2H), 3.34 - 3.23 (m, 2H), 2.98 (d, *J =* 16.8 Hz, 1H), 2.81 - 2.73 (m, 2H), 2.48 (d, *J =* 16.8 Hz, 1H), 1.92 - 1.79 (m, 5H), 1.60 - 1.52 (m, 1H), 1.26 (s, 1H), 0.79 (q, J = 3.6 Hz, 1H), 0.51 (q, J = 7.6 Hz, 1H). MS m/z: 623.2 [M+1]⁺.

Compound **7B2:** The retention time under analytical SFC (column: ChiralPak IG-3, 50 * 4.6 mm, 3 µm; mobile phases: A (supercritical CO₂) and B (isopropanol, containing 0.1% isopropylamine); gradient: B%:5% to 5%, 3 min) was 2.343 min with an ee value of 98.62%. ¹H NMR (400 MHz, CDCl₃) δ = 7.67 (dd, J = 8.8, 5.6 Hz, 1H), 7.29 - 7.25 (m, 1H), 7.22 (t, J = 8.8 Hz, 1H), 7.11 (d, J = 2.4 Hz, 1H), 6.97 (d, J = 2.0 Hz, 1H), 5.03 (s, 1H), 4.95 (s, 1H), 4.41 (d, J = 12.0 Hz, 1H), 4.36 (d, J = 10.4 Hz, 2H), 4.23 (d, J = 10.0 Hz, 1H), 4.07 - 3.78 (m, 2H), 3.75 - 3.63 (m, 3H), 3.59 (d, J = 12.0 Hz, 1H), 3.54 (d, J = 12.4 Hz, 1H), 3.31 (d, J = 3.2 Hz, 1H), 3.28 (s, 1H), 3.00 (d, J = 16.8 Hz, 1H), 2.82 - 2.73 (m, 2H), 2.48 (d, J = 16.8 Hz, 1H), 1.93 - 1.80 (m, 5H), 1.61 - 1.51 (m, 1H), 1.26 (s, 1H), 0.81 (q, J = 4.0 Hz, 1H), 0.55 - 0.47 (m, 1H). MS m/z: 623.2 [M+1]⁺.

### Example 8

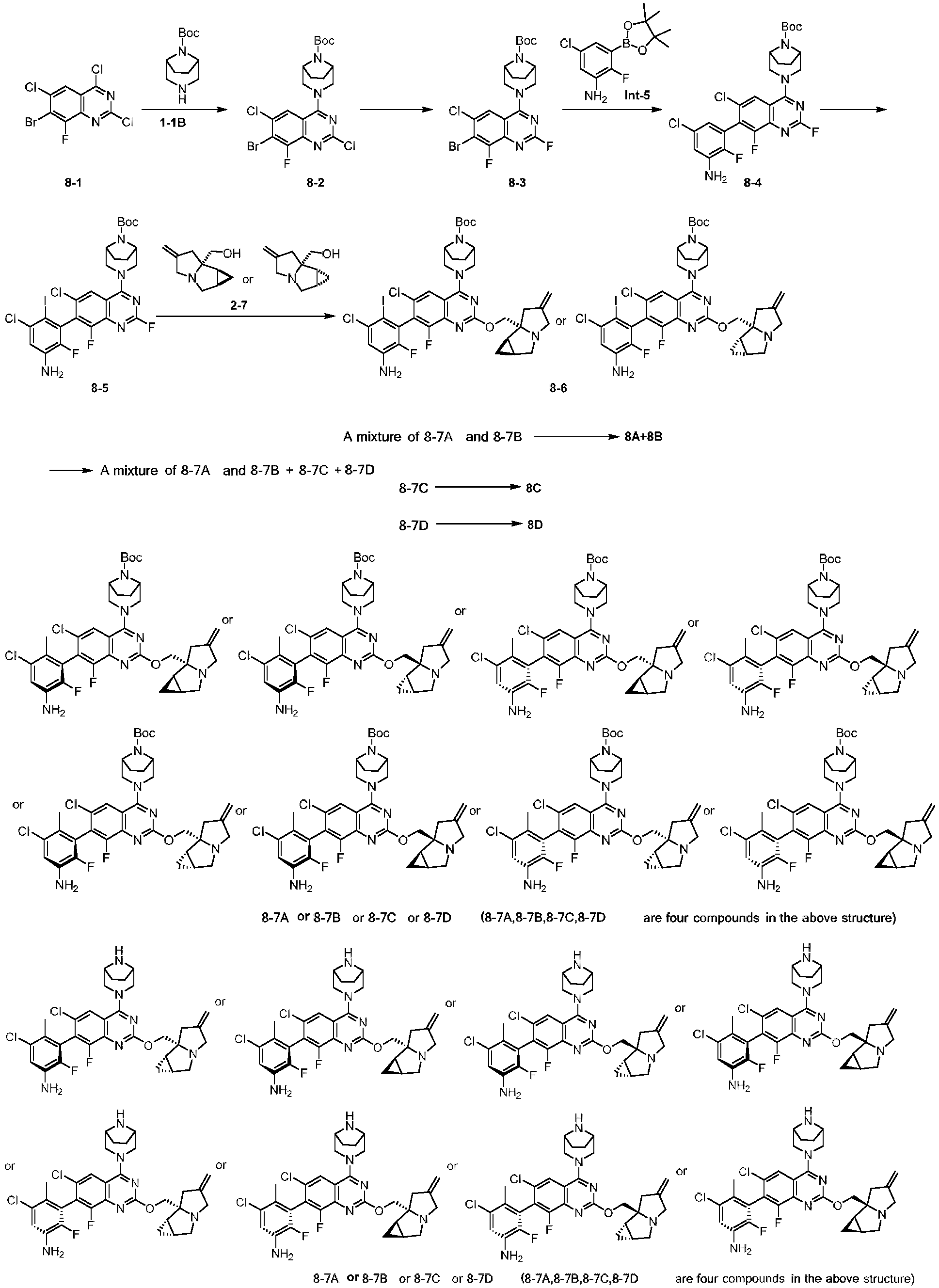

### Step 1:

Compounds **8-1** (2 g, 6.05 mmol) and **1-1B** (1.35 g, 6.36 mmol) were dissolved in dichloromethane (20 mL), and the mixture was replaced with nitrogen for three times, and then triethylamine (1.84 g, 18.16 mmol) was added dropwise thereto at -40°C. After the dropwise addition was completed, the reaction mixture was warmed to 25°C and reacted for 3 hours. The reaction mixture was poured into 50 mL of water, and the phases were separated. The aqueous phase was extracted with dichloromethane (10 mL * 3). The organic phases were combined, dried over anhydrous sodium sulfate, filtered, and concentrated under reduced pressure. The crude product was purified by column chromatography (eluent: petroleum ether: ethyl acetate = 1:0 to 3:1) to obtain compound **8-2.** MS m/z: 505.0, 507.0 [M+1]⁺.

### Step 2:

**8-2** (646 mg, 1.28 mmol) and potassium fluoride (1.48 g, 25.52 mmol) were dissolved in dimethyl sulfoxide (12 mL), and the mixture was replaced with nitrogen for three times, heated to 120°C, and stirred for 1 hour. The reaction mixture was poured into 60 mL of water and extracted with ethyl acetate (10 mL * 3). The organic phases were combined, dried over anhydrous sodium sulfate, filtered, and concentrated under reduced pressure. The crude product was purified by column chromatography (eluent: petroleum ether: ethyl acetate = 1:0 to 5:1) to obtain **8-3.** ¹H NMR (400 MHz, CDCl₃) δ 7.80 (d, J = 1.6 Hz, 1H), 4.22-4.58 (m, 4H), 3.51 - 3.89 (m, 2H), 1.90-2.04 (m, 2H), 1.66 - 1.84 (m, 2H), 1.53 (s, 9H).

### Step 3:

**Int-5** (333.98 mg, 1.23 mmol), **8-3** (502 mg, 1.03 mmol), and potassium phosphate (435.17 mg, 2.05 mmol) were dissolved in tetrahydrofuran (10 mL) and water (2.5 mL), then the mixture was replaced with nitrogen for three times, and methanesulfonato(2-dicyclohexylphosphino-2',4',6'-tri-i-propyl-1,1'-biphenyl)(2'-amino-1,1'-biphenyl-2-yl)palladium(II) (86.76 mg, 102.50 µmol, 0.1 eq) was added thereto, then the reaction mixture was replaced with nitrogen for three times, stirred at 35°C for 3 hours. The reaction mixture was cooled, filtered, and concentrated under reduced pressure. The crude product was purified by column chromatography (eluent: petroleum ether: ethyl acetate = 1:0 to 4:1) to obtain **8-4.** ¹H NMR (400 MHz, CDCl₃) δ 7.81 (d, J = 1.6 Hz, 1H), 6.90 (dd, J = 7.2, 2.4 Hz, 1H), 6.65 (dd, J = 5.2, 2.4 Hz, 1H), 4.29 - 4.56 (m, 4H), 3.51 - 3.87 (m, 2H), 1.93 - 2.03 (m, 2H), 1.77 (br d, J = 8.0 Hz, 2H), 1.54 (s, 9H).

### Step 4:

**8-4** (330 mg, 595.25 µmol) and *N*-iodosuccinimide (200.88 mg, 892.87 µmol) were dissolved in *N*,*N*-dimethylformamide (3.3 mL), replaced with nitrogen for three times, heated at 50°C, and stirred for 28 hours. The reaction mixture was poured into 5 mL of saturated sodium bicarbonate aqueous solution and 5 mL of saturated sodium thiosulfate aqueous solution, and extracted with ethyl acetate (5 mL * 3). The organic phases were combined, dried over anhydrous sodium sulfate, filtered, and concentrated under reduced pressure. The crude product was purified by column chromatography (eluent: petroleum ether: ethyl acetate = 1:0 to 3:1) to obtain compound **8-5.** ¹H NMR (400 MHz, CDCl₃) δ 7.85 (d, J = 1.6 Hz, 1H), 7.11 (d, J = 8.4 Hz, 1H), 4.30 - 4.63 (m, 4H), 3.52 - 3.91 (m, 2H), 1.94 - 2.05 (m, 2H), 1.72 - 1.88 (m, 2H), 1.54 (s, 9H).

### Step 5:

Sodium hydride (36.69 mg, 917.26 µmol, 60%) was dissolved in tetrahydrofuran (1 mL), replaced with nitrogen for three times, cooled to 0°C, and a solution of **2-7** (151.56 mg, 917.26 µmol) in tetrahydrofuran (1.5 mL) was added thereto, warmed to 20°C, and stirred for 10 min. The mixture was cooled to 0°C, added with a solution of **8-5** (208 mg, 305.75 µmol) in tetrahydrofuran (2.5 mL), warmed to 20°C, and stirred for 4 hours. The reaction mixture was poured into 10 mL of saturated ammonium chloride aqueous solution and extracted with ethyl acetate (2 mL * 3). The organic phases were combined, dried over anhydrous sodium sulfate, filtered, and concentrated under reduced pressure. The crude product was purified by column chromatography (eluent: petroleum ether: ethyl acetate = 1:0 to 1:2) to obtain **8-6.** MS m/z: 825.1 [M+1]⁺.

### Step 6:

**8-6** (180 mg, 218.05 µmol), methylboronic acid (39.16 mg, 654.14 µmol), and potassium carbonate (60.27 mg, 436.09 µmol) were dissolved in dioxane (2 mL) and water (0.5 mL), replaced with nitrogen for three times, [1,1'-bis(diphenylphosphino)ferrocene]dichloropalladium(II) (15.95 mg, 21.80 µmol, 0.1 eq) was added thereto, and the reaction mixture was replaced with nitrogen for three times, heated to 80°C, and stirred for 60 hours. The reaction mixture was concentrated under reduced pressure. The residue was purified by column chromatography (eluent: petroleum ether: ethyl acetate = 1:0 to 1:2) to obtain a crude product. The crude product was purified by preparative SFC (chromatographic column: DAICEL CHIRALPAK AD (250 mm * 30 mm, 10 µm); mobile phase: A (supercritical carbon dioxide) and B (isopropanol, containing 0.1% ammonia); gradient: B% = 45% to 45%) to obtain a mixture of **8-7A** and **8-7B, 8-7C,** and **8-7D,** respectively. The retention time of **8-7C** in the SFC analytical method (chromatographic column: Chiralpak AD-3, 50 × 4.6 mm, 3 µm; mobile phase: A (supercritical carbon dioxide) and B (isopropanol, containing 0.1% isopropylamine); gradient: B% = 5% to 50% to 5%, 3.0 min) was 1.468 min, with an enantiomeric excess of 97.36%. The retention time of **8-7D** in the SFC analytical method (chromatographic column: Chiralpak AD-3, 50 × 4.6 mm, 3 µm; mobile phase: A (supercritical carbon dioxide) and B (isopropanol, containing 0.1% isopropylamine); gradient: B% = 5% to 50% to 5%, 3.0 min) was 1.603 min, with an enantiomeric excess of 100%.

### Step 7:

The mixture of **8-7A** and **8-7B** (32 mg, 44.84 µmol) was dissolved in dichloromethane (0.6 mL), then trifluoroacetic acid (0.2 mL) was added thereto, and the reaction mixture was stirred at 15°C for 0.5 hours. The reaction mixture was adjusted to pH = 9 by adding ammonia water dropwise and concentrated under reduced pressure. The crude product was purified by preparative high-performance liquid chromatography (chromatographic column: Phenomenex Luna 100 * 30 mm * 3 µm; mobile phase A: water (0.2% formic acid), mobile phase B: acetonitrile; running gradient: B%: 1% to 30%, run for 8 min) to obtain a crude product. The crude product was purified by preparative SFC (chromatographic column: DAICEL CHIRALPAK AD (250 mm * 30 mm, 10 µm); mobile phase: A (supercritical carbon dioxide) and B (ethanol, containing 0.1% ammonia); gradient: B% = 50% to 50%, run for 11 min) to obtain **8A** and **8B.**

The retention time of **8A** in the SFC analytical method (chromatographic column: Chiralpak IG-3, 50 × 4.6 mm, 3 µm; mobile phase: A (supercritical carbon dioxide) and B (ethanol, containing 0.1% isopropylamine); gradient: B% = 5% to 50% to 5%, 3.0 min) was 1.727 min, with an enantiomeric excess of 100%. ¹H NMR (400 MHz, CD₃OD) δ: 7.91 (s, 1 H), 7.01 (d, J=8.4 Hz, 1 H), 4.95 - 5.10 (m, 2 H), 4.47 (br d, J=12.4 Hz, 2 H), 4.25 - 4.42 (m, 2 H), 3.56 - 3.75 (m, 5 H), 3.26-3.30 (m, 1 H), 3.20 (br d, J=9.6 Hz, 1 H), 2.91 (br d, J=16.4 Hz, 1 H), 2.79 (br dd, J=9.2, 3.6 Hz, 1 H), 2.53 (br d, J=17.2 Hz, 1 H), 1.97 (s, 3 H), 1.81 (br s, 5 H), 1.56 - 1.70 (m, 1 H), 0.70 (q, J=4.0 Hz, 1 H), 0.49 - 0.59 (m, 1 H). MS m/z: 613.2 [M+1]⁺.

The retention time of **8B** in the SFC analytical method (chromatographic column: Chiralpak IG-3, 50 × 4.6 mm, 3 µm; mobile phase: A (supercritical carbon dioxide) and B (ethanol, containing 0.1% isopropylamine); gradient: B% = 5% to 50% to 5%, 3.0 min) was 2.216 min, with an enantiomeric excess of 99.22%. ¹H NMR (400 MHz, CD₃OD) δ: 7.92 (d, J=2.0 Hz, 1 H), 7.01 (d, J=8.4 Hz, 1 H), 4.96 - 5.10 (m, 2 H), 4.47 (br t, J=13.2 Hz, 2 H), 4.25 - 4.41 (m, 2 H), 3.57 - 3.74 (m, 5 H), 3.26-3.30 (m, 1 H), 3.20 (d, J=9.6 Hz, 1 H), 2.91 (br d, J=16.8 Hz, 1 H), 2.79 (dd, J=9.6, 4.0 Hz, 1 H), 2.53 (br d, J=16.4 Hz, 1 H), 1.76 - 1.91 (m, 8 H), 1.56 - 1.69 (m, 1 H), 0.70 (q, J=4.4 Hz, 1 H), 0.49-0.59(m, 1 H). MS m/z: 613.2 [M+1]⁺.

### Step 8:

**8-7C** was dissolved in dichloromethane (0.6 mL), and trifluoroacetic acid (0.2 mL) was added thereto. The reaction mixture was stirred at 15°C for 0.5 hours. The reaction mixture was adjusted to pH = 9 by adding ammonia water dropwise and concentrated under reduced pressure. The crude product was purified by preparative high-performance liquid chromatography (chromatographic column: Phenomenex Luna 100 * 30 mm * 3 µm; mobile phase A: water (0.2% formic acid), mobile phase B: acetonitrile; running gradient: B%: 1% to 30%, run for 8 min) to obtain the formate of **8C.** ¹H NMR (400 MHz, CD₃OD) δ 8.51 (br s, 1H), 7.94 (s, 1H), 7.02 (d, J = 8.0 Hz, 1H), 5.09 (d, J = 16.4 Hz, 2H), 4.50 - 4.63 (m, 3H), 4.41 (d, J = 10.8 Hz, 1H), 3.86 - 4.08 (m, 3H), 3.76 (br dd, J = 12.8, 8.8 Hz, 2H), 3.48 (br d, J = 15.6 Hz, 1H), 3.34 - 3.41 (m, 1H), 2.92 - 3.03 (m, 2H), 2.66 (br d, J = 16.4 Hz, 1H), 1.93 - 2.07 (m, 7H), 1.81-1.92 (m, 1H), 1.66-1.76 (m, 1H), 0.71-0.78 (m, 1H), 0.60 - 0.69 (m, 1H). MS m/z: 613.2 [M+1]⁺.

### Step 9:

**8-7D** (21 mg, 29.43 µmol) was dissolved in dichloromethane (0.6 mL), then trifluoroacetic acid (0.2 mL) was added thereto, and the reaction mixture was stirred at 15°C for 0.5 hours. The reaction mixture was adjusted to pH = 9 by adding ammonia water dropwise and concentrated under reduced pressure. The crude product was purified by preparative high-performance liquid chromatography (chromatographic column: Phenomenex Luna 100 * 30 mm * 3 µm; mobile phase A: water (0.2% formic acid), mobile phase B: acetonitrile; running gradient: B%: 1% to 30%, run for 8 min) to obtain the formate of **8D.** ¹H NMR (400 MHz, CD₃OD) δ 8.53 (br s, 1H), 7.93 (s, 1H), 7.02 (d, J = 8.0 Hz, 1H), 5.07 (br d, J = 18.8 Hz, 2H), 4.42 - 4.65 (m, 3H), 4.37 (br d, J = 10.8 Hz, 1H), 3.81 - 3.97 (m, 3H), 3.73 (br d, J = 12.4 Hz, 2H), 3.43 (br d, J = 15.6 Hz, 1H), 3.34 (br s, 1H), 2.84 - 3.03 (m, 2H), 2.62 (br d, J = 17.2 Hz, 1H), 1.97 (s, 7H), 1.81 - 1.89 (m, 1H), 1.64 - 1.73 (m, 1H), 0.69-0.81 (m, 1H), 0.57 - 0.66 (m, 1H). MS m/z: 613.2 [M+1]⁺.

### Experimental example 1. p-ERK inhibition test of GP2D cells

### 1. Purpose

Through the HTRF method, compounds that can effectively inhibit p-ERK in GP2D cells were screened out.

### 2. Experimental process

1). GP2D cells were seeded in a transparent 96-well cell culture plate, with 80 µL of cell suspension per well and 8000 cells per well, and the cell plate was incubated in a carbon dioxide incubator at 37°C overnight;
2). 2 µL of the compound was added to 78 µL of cell culture medium; after mixing well, 20 µL of the compound solution was taken and added to the corresponding cell plate wells, and the cell plate was put back into the carbon dioxide incubator to incubate for another 1 hour;
3). After the incubation was completed, the cell supernatant was discarded, and 50 µL of 1X cell lysate was added to each well, and incubated at room temperature for 30 minutes with shaking;
4). Phospho-ERK1/2 Eu Cryptate antibody and Phospho-ERK1/2 d2 antibody were 20-fold diluted using detection buffer;
5). The supernatant of the cell lysate was added to a new 384-well white microplate at 16 µL per well, then 2 µL of Phospho-ERK1/2 Eu Cryptate antibody diluent and 2 µL of Phospho-ERK1/2 d2 antibody diluent were added thereto and incubated at room temperature for at least 4 hours;
6). After the incubation was completed, a multimode microplate reader was used to read HTRF excitation: 320 nm, emission: 615 nm, 665 nm;
7) The IC₅₀ of the test compound was calculated.

### 3. Experimental results

The results are shown in Table 2.

**Table 2 IC₅₀ values of compounds inhibiting p-ERK in GP2D cells**

| **Compound No.** | **GP2D p-ERK IC₅₀ (nM)** |
|---|---|
| Compound **1** | 4.75 |
| Formate of compound **2A** | 2.69 |
| Compound **3** | 2.4 |
| Formate of compound **4** | 19.4 |

Experimental conclusion: The compounds of the present disclosure have significant p-ERK inhibitory effects in GP2D cells.

### Experiment example 2. GP2D 3D CTG experiment

### 1. Experimental purpose:

This experiment aims to verify the inhibitory effect of the compounds of the present disclosure on the proliferation of GP2D human colon cancer cells with KRAS^{G12D} mutation.

### 2. Experimental materials:

Cell line GP2D and DMEM medium were purchased from GIBCO, FBS was purchased from Hyclone, and L-glutamine was purchased from Invitrogen. The 96-well plate was purchased from Ultra Low Cluster. CellTiter-Glo^{®} 3D Cell Viability Assay reagents (chemiluminescent 3D cell viability assay reagents) were purchased from Promega, and 2104 EnVision plate reader was purchased from PerkinElmer.

### 3. Experimental methods:

GP2D cells were cultured in an incubator at 37°C with 5% CO₂ under the conditions of DMEM + 10% FBS + 2 mM L-glutamine. Regular passaging was performed, and cells in the logarithmic growth phase were selected for plating. GP2D cells were seeded in a 96-well U-bottom cell culture plate, and each well of 135 µL cell suspension contained 6000 GP2D cells. The culture plate was cultured overnight in a 37°C, 5% COz incubator with 100% relative humidity. The test compound was diluted 5-fold to the eighth concentration using a pipette, ranging from 200 µM to 2.56 nM, and tested in duplicate. 78 µL of culture medium per well was added to an intermediate plate. Subsequently, 2 µL of the gradient diluted compound per well was transferred to the corresponding wells of the intermediate plate and mixed well. 20 µL of the mixture per well was then transferred to the cell plate. The concentration of the compound transferred to the cell plate ranged from 1 µM to 0.0128 nM. The cell plate was incubated for 5 days in a COz incubator. After the incubation of the cell plate with the compound was completed, chemiluminescent cell viability assay reagent was added to the cell plate at 100 µL per well, and incubated at room temperature for 10 minutes to stabilize the luminescent signal. A multimode microplate reader was used for reading.

### 4. Data analysis:

Using the equation (Sample - Min) / (Max - Min) * 100% to convert the raw data into inhibition rate, the IC₅₀ value can be obtained by curve fitting with four parameters (obtained by "log (inhibitor) vs. response -- Variable slope" mode in GraphPad Prism).

### 5. Experimental results:

The results are shown in Table 3.

**Table 3 IC₅₀ values of compounds against anti-proliferation of GP2D cells**

| **Compound No.** | **GP2D IC₅₀ (nM)** |
|---|---|
| Compound **3** | 0.54 |
| Compound **7A2** | 1.37 |

**Experimental conclusion:** The compounds of the present disclosure have significant anti-proliferative activity against GP2D cells with KRAS^{G12D} mutation.

### Experimental example 3. AsPC-1 3D CTG experiment

### 1. Experimental purpose:

This experiment aims to verify the inhibitory effect of the compounds of the present disclosure on the proliferation of AsPC-1 human pancreatic cancer cells with KRAS^{G12D} mutation.

### 2. Experimental materials:

Cell line AsPC-1 and RPMI-1640 medium were purchased from GIBCO, and FBS was purchased from Hyclone. The 96-well plate was purchased from Ultra Low Cluster, CellTiter-Glo^{®} 3D Cell Viability Assay reagents (chemiluminescent 3D cell viability assay reagents) were purchased from Promega, and 2104 EnVision plate reader was purchased from PerkinElmer.

### 3. Experimental methods:

AsPC-1 cells were cultured in an incubator at 37°C with 5% CO₂ the conditions of RPMI-1640 + 10% FBS. Regular passaging was performed, and cells in the logarithmic growth phase were selected for plating. AsPC-1 cells were seeded in a 96-well U-bottom cell culture plate, and each well of 135 µL cell suspension contained 500 AsPC-1 cells. The culture plate was cultured overnight in a 37°C, 5% CO₂ incubator with 100% relative humidity. The test compound was diluted 5-fold to the eighth concentration using a pipette, ranging from 200 µM to 2.56 nM, and tested in duplicate. 78 µL of culture medium per well was added to an intermediate plate. Subsequently, 2 µL of the gradient diluted compound per well was transferred to the corresponding wells of the intermediate plate and mixed well. 20 µL of the mixture per well was then transferred to the cell plate. The concentration of the compound transferred to the cell plate ranged from 1 µM to 0.0128 nM. The cell plate was incubated for 7 days in a CO₂ incubator. After the incubation of the cell plate with the compound was completed, chemiluminescent cell viability assay reagent was added to the cell plate at 100 µL per well, and incubated at room temperature for 10 minutes to stabilize the luminescent signal. A multimode microplate reader was used for reading.

### 4. Data analysis:

Using the equation (Sample - Min) / (Max - Min) * 100% to convert the raw data into inhibition rate, the IC₅₀ value can be obtained by curve fitting with four parameters (obtained by "log (inhibitor) vs. response -- Variable slope" mode in GraphPad Prism).

### 5. Experimental results:

The results are shown in Table 4.

**Table 4 IC₅₀ values of compounds against anti-proliferation of AsPC-1 cells**

| **Compound No.** | **AsPC-1 IC₅₀ (nM)** |
|---|---|
| Compound 3 | 5.74 |

**Experimental conclusion:** The compounds of the present disclosure have significant anti-proliferative activity against AsPC-1 cells with KRAS^{G12D} mutation.

### Experiment example 4. PANC04.03 3D CTG experiment

### 1. Experimental purpose:

This experiment aims to verify the inhibitory effect of the compounds of the present disclosure on the proliferation of PANC04.03 human pancreatic cancer cells with KRAS^{G12D} mutation.

### 2. Experimental materials:

Cell line PANC04.03 and RPMI-1640 medium were purchased from GIBCO, FBS was purchased from Hyclone, and human insulin was purchased from Yeasen. The 96-well plate was purchased from Ultra Low Cluster, CellTiter-Glo^{®} 3D Cell Viability Assay reagents (chemiluminescent 3D cell viability assay reagents) were purchased from Promega, and 2104 EnVision plate reader was purchased from PerkinElmer.

### 3. Experimental methods:

PANC04.03 cells were cultured in an incubator at 37°C with 5% CO₂ under the conditions of RPMI-1640 + 15% FBS + 5 µg/mL human insulin. Regular passaging was performed, and cells in the logarithmic growth phase were selected for plating. PANC04.03 cells were seeded in a 96-well U-bottom cell culture plate, and each well of 135 µL cell suspension contained 2000 PANC04.03 cells. The culture plate was cultured overnight in a 37°C, 5% CO₂ incubator with 100% relative humidity. The test compound was diluted 5-fold to the eighth concentration using a pipette, ranging from 200 µM to 2.56 nM, and tested in duplicate. 78 µL of culture medium per well was added to an intermediate plate. Subsequently, 2 µL of the gradient diluted compound per well was transferred to the corresponding wells of the intermediate plate and mixed well. 20 µL of the mixture per well was then transferred to the cell plate. The concentration of the compound transferred to the cell plate ranged from 1 µM to 0.0128 nM. The cell plate was incubated for 7 days in a CO₂ incubator. After the incubation of the cell plate with the compound was completed, chemiluminescent cell viability assay reagent was added to the cell plate at 100 µL per well, and incubated at room temperature for 10 minutes to stabilize the luminescent signal. A multimode microplate reader was used for reading.

### 4. Data analysis:

Using the equation (Sample - Min) / (Max - Min) * 100% to convert the raw data into inhibition rate, the IC₅₀ value can be obtained by curve fitting with four parameters (obtained by "log (inhibitor) vs. response -- Variable slope" mode in GraphPad Prism).

### 5. Experimental results:

The results are shown in Table 5.

**Table 5 IC₅₀ values of compounds against anti-proliferation of PANC04.03 cells**

| **Compound No.** | **PANC04.03 IC₅₀ (nM)** |
|---|---|
| Compound 3 | 34.17 |

**Experimental conclusion:** The compounds of the present disclosure have significant anti-proliferative activity against PANC04.03 cells with KRAS^{G12D} mutation.

### Experimental example 5. In vivo pharmacokinetic experiment

### 1. Experimental purpose:

This experiment aims to investigate the pharmacokinetic properties of the compounds of the present disclosure administrated by oral and intravenous injection in CD-1 mice.

### 2. Experimental methods:

The test compound was mixed with 10% dimethyl sulfoxide + 90% (10% hydroxypropyl-β-cyclodextrin (HP-β-CD) aqueous solution), vortexed, and sonicated to prepare 0.6, 3.0, and 10.0 mg/mL clear solutions, respectively. Male CD-1 mice aged 7 to 10 weeks were selected and intravenously injected (i.v.) with a solution of the candidate compound at a dose of 3 mg/kg (administration concentration of 0.6 mg/mL). The solution of the candidate compound was administered orally (p.o.) at a dose of 30 mg/kg (administration concentration of 3.0 mg/mL) or 100 mg/kg (administration concentration of 10.0 mg/mL). Whole blood was collected for a certain period of time and prepared into plasma. The drug concentration was analyzed by the LC-MS/MS method, and the pharmacokinetic parameters were calculated using Phoenix WinNonlin software (Pharsight, USA).

### 3. Experimental results:

The results are shown in Table 6.

**Table 6 PK properties of compound 3 in CD-1 mice**

| Compound **3** | | |
|---|---|---|
| i.v. 3 mpk | T_{1/2} (h) | 9.30 |
| | Vd (L/kg) | 41.1 |
| | Cl (mL/kg/min) | 50.8 |
| | AUC₀₋ₗₐₛₜ (nM.h) | 1556 |
| | AUC_{0-∞} (nM.h) | 1660 |
| p.o. 30 mpk | T_{1/2} (h) | 5.41 |
| | Tₘₐₓ (h) | 0.583 |
| | Cₘₐₓ (nmol/L) | 439 |
| | AUC₀₋ₗₐₛₜ (nM.h) | 1154 |
| | AUC_{0-∞} (nM.h) | 1183 |
| p.o. 100 mpk | T_{1/2} (h) | 4.24 |
| | Tₘₐₓ (h) | 1.25 |
| | Cₘₐₓ (nmol/L) | 3114 |
| | AUC₀₋ₗₐₛₜ (nM.h) | 13149 |
| | AUC_{0-∞} (nM.h) | 13306 |
| | F (%) | 24.0 |

**Experimental conclusion:** The compound of the present disclosure has good pharmacokinetic properties in mice.

### Experimental example 6. In vivo pharmacodynamic experiment of GP2D tumor model

### 1. Experimental purpose:

*In vivo* pharmacodynamics of GP2D cell subcutaneously transplanted tumors in Balb/c Nude mice model with human colon cancer

### 2. Experimental methods:

Cell culture: Human colon cancer GP2D cells were cultured in monolayer *in vitro.* The culture conditions were DMEM/F12 medium + 20% fetal bovine serum, and cultured in a 37°C, 5% carbon dioxide incubator. Routine digestion with trypsin-EDTA was performed twice a week for passaging. When the cell saturation reached 80% to 90% and the required quantity was reached, the cells were harvested, counted, resuspended in an appropriate amount of PBS, and mixed with matrigel at a ratio of 1:1 to obtain a cell suspension with a cell density of 25 × 10⁶ cells/mL.

Cell inoculation: 0.2 mL (5 × 10⁶ cells/mouse) of GP2D cells (added with Matrigel with a volume ratio of 1:1) were subcutaneously inoculated into the right back of each mouse.

Experimental operation: When the average tumor volume reached 140 mm³, the mice were randomly divided into groups according to the tumor volume, with 6 animals in each group. The blank group was administered with a dose of 0, and the test group was orally administered with a dose of 30 mg/kg, 100 mg/kg, and a volume of 10 µL/g, respectively, and was administered twice a day for 28 days.

### 3. Tumor measurement and experimental indicators:

Tumor diameters were measured twice a week with a vernier caliper. The formula for calculating tumor volume is: V = 0.5a × b², wherein a and b represent the long and short diameters of the tumor, respectively.

The tumor inhibitory efficacy of the compound was evaluated by TGI (%) or relative tumor proliferation rate T/C (%). Relative tumor proliferation rate T/C (%) = TRTV / CRTV × 100% (TRTV: RTV of treatment group; CRTV: RTV of negative control group). The relative tumor volume (RTV) was calculated based on the results of tumor measurements. The calculation formula is RTV = Vₜ / V₀, where V₀ is the average tumor volume measured at the beginning of administration in groups (*i.e.*, D₀), and Vt is the average tumor volume measured at a certain measurement. TRTV and CRTV used the data from the same day.

TGI (%) reflected the tumor growth inhibition rate. TGI (%) = [1 - (average tumor volume at the end of administration in a treatment group - average tumor volume at the beginning of administration in this treatment group) / (average tumor volume at the end of treatment in the vehicle control group - average tumor volume at the beginning of treatment in the vehicle control group)] × 100%.

### 4. Experimental results:

The experimental results are shown in Table 7.

**Table 7 Pharmacodynamic results of compound 3 in the in vivo pharmacodynamic model of GP2D tumor model**

| Dose | 30 mpk, p.o., BID | 100 mpk, p.o., BID |
|---|---|---|
| Days of administration | 28 | 28 |
| TGI | 85.57% | 98.95% |

**Experimental conclusion:** The compound of the present disclosure has excellent tumor inhibitory effects.

### Experimental example 7. In vivo pharmacodynamic experiment of PANC04.03 tumor model

### 1. Experimental purpose:

*In vivo* pharmacodynamics of PANC04.03 cell subcutaneously transplanted tumors in Balb/c Nude mice model with human pancreatic cancer

### 2. Experimental methods:

Cell culture: Human pancreatic cancer PANC04.03 cells were cultured in monolayer *in vitro.* The culture conditions were RPMI-1640 + 15% FBS + 10 units/mL insulin, and cultured in a 37°C, 5% carbon dioxide incubator. Routine digestion with trypsin-EDTA was performed twice a week for passaging. When the cell saturation reached 80% to 90% and the required quantity was reached, the cells were harvested, counted, and resuspended in an appropriate amount of PBS to obtain a cell suspension with a cell density of 25 × 10⁶ cells/mL.

Cell inoculation: 0.2 mL (5 × 10⁶ cells/mouse) of PANC04.03 cells were subcutaneously inoculated into the right back of each mouse.

Experimental operation: When the average tumor volume reached 190 mm³, the mice were randomly divided into groups according to the tumor volume, with 6 animals in each group. The blank group was administered with a dose of 0, and the test group was orally administered with a dose of 30 mg/kg, 100 mg/kg, 150 mg/kg, and a volume of 10 µL/g, respectively, and was administered twice a day for 28 days.

### 3. Tumor measurement and experimental indicators:

Tumor diameters were measured twice a week with a vernier caliper. The formula for calculating tumor volume is: V = 0.5a × b², wherein a and b represent the long and short diameters of the tumor, respectively.

The tumor inhibitory efficacy of the compound was evaluated by TGI (%) or relative tumor proliferation rate T/C (%). Relative tumor proliferation rate T/C (%) = TRTV / CRTV × 100% (TRTV: RTV of treatment group; CRTV: RTV of negative control group). The relative tumor volume (RTV) was calculated based on the results of tumor measurements. The calculation formula is RTV = Vₜ / V₀, where V₀ is the average tumor volume measured at the beginning of administration in groups (*i.e.*, D₀), and Vt is the average tumor volume measured at a certain measurement. TRTV and CRTV used the data from the same day.

TGI (%) reflected the tumor growth inhibition rate. TGI (%) = [1 - (average tumor volume at the end of administration in a treatment group - average tumor volume at the beginning of administration in this treatment group) / (average tumor volume at the end of treatment in the vehicle control group - average tumor volume at the beginning of treatment in the vehicle control group)] × 100%.

### 4. Experimental results:

The experimental results are shown in Table 8.

**Table 8 Pharmacodynamic results of compound 3 in the in vivo pharmacodynamic model of mouse PACN04.03**

| Dose | 30 mpk, p.o., BID | 100 mpk, p.o., BID | 150 mpk, p.o., BID |
|---|---|---|---|
| Days of administration | 28 | 28 | 28 |
| TGI | 95.41% | 108.41% | 115.80% |

**Experimental conclusion:** The compound of the present disclosure has excellent tumor inhibitory effects.

## Claims

1. The present disclosure provides a compound represented by formula (III-1) or a pharmaceutically acceptable salt thereof, 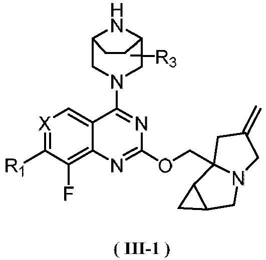 wherein
X is selected from CH, C-Rx, N, and N⁺-O⁻; preferably, X is selected from N and N⁺-O⁻; Rx is selected from F, Cl, and Br;
R₁ is selected from 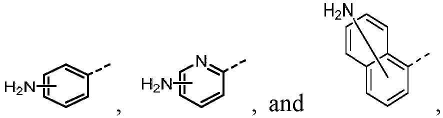 and the 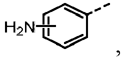 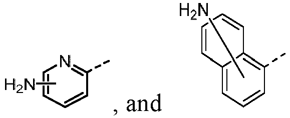 are each independently and optionally substituted by 1, 2, 3, or 4 Rₐ;
R₃ is selected from H and D;
each Rₐ is independently selected from F, Cl, Br, I, OH, NH₂, C₁₋₃ alkyl, C₁₋₃ alkoxy, C₁₋₃ haloalkyl, C₂₋₄ alkenyl, C₂₋₄ alkynyl, -C₁₋₃ alkyl-cyclopropyl, and cyclopropyl, and the C₁₋₃ alkyl, C₁₋₃ alkoxy, C₂₋₄ alkenyl, C₂₋₄ alkynyl, cyclopropyl, and -C₁₋₃ alkyl-cyclopropyl are each independently and optionally substituted by 1, 2, or 3 R;
each R is independently selected from F, Cl, Br, I, CH₂F, CHF₂, and CF₃.

2. A compound represented by formula (III-1) or a pharmaceutically acceptable salt thereof, 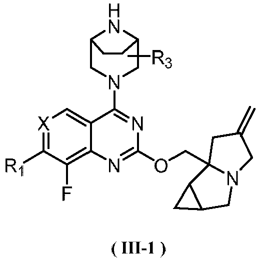 wherein
X is selected from CH, N, and N⁺-O⁻; preferably, X is selected from N and N⁺-O⁻;
R₁ is selected from 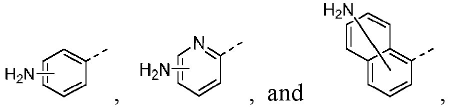 and the 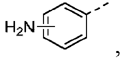 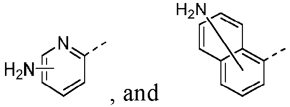
are each independently and optionally substituted by 1, 2, 3, or 4 Rₐ;
R₃ is selected from H and D;
each Rₐ is independently selected from F, Cl, Br, I, OH, NH₂, C₁₋₃ alkyl, C₁₋₃ alkoxy, C₂₋₄ alkenyl, C₂₋₄ alkynyl, -C₁₋₃ alkyl-cyclopropyl, and cyclopropyl, and the C₁₋₃ alkyl, C₁₋₃ alkoxy, C₂₋₄ alkenyl, C₂₋₄ alkynyl, cyclopropyl, and -C₁₋₃ alkyl-cyclopropyl are each independently and optionally substituted by 1, 2, or 3 R;
each R is independently selected from F, Cl, Br, I, CH₂F, CHF₂, and CF₃.

3. Compounds represented by formulas (II) and (III-2) or a pharmaceutically acceptable salt thereof, 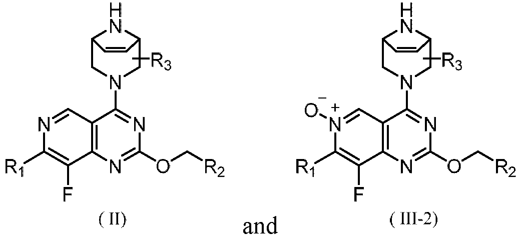 wherein
R₁ is selected from phenyl, pyridyl, and naphthyl, and the phenyl, pyridyl, and naphthyl are each independently and optionally substituted by 1, 2, 3, or 4 Rₐ;
R₂ is selected from 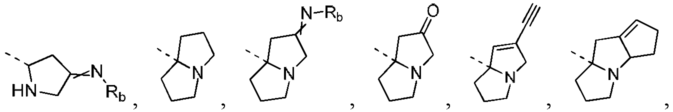 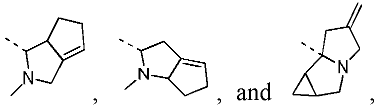 and the 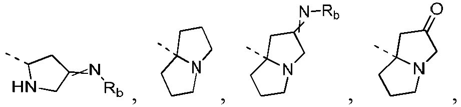 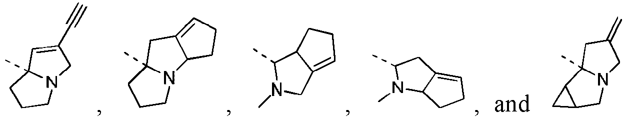 are each independently and optionally substituted by 1, 2, or 3 R_{c};
R₃ is selected from H and D;
each Rₐ is independently selected from F, Cl, Br, I, OH, NH₂, C₁₋₃ alkyl, C₁₋₃ alkoxy, C₂₋₄ alkenyl, C₂₋₄ alkynyl, -C₁₋₃ alkyl-cyclopropyl, and cyclopropyl, and the C₁₋₃ alkyl, C₁₋₃ alkoxy, C₂₋₄ alkenyl, C₂₋₄ alkynyl, cyclopropyl, and -C₁₋₃ alkyl-cyclopropyl are each independently and optionally substituted by 1, 2, or 3 R;
R_{b} is selected from H, CN, CH₃, and OCH₃;
each R_{c} is independently selected from F, Cl, Br, I, CH₂F, CHF₂, CF₃, and CH₂CF₃;
each R is independently selected from F, Cl, Br, I, CH₂F, CHF₂, and CF₃.

4. The compound or the pharmaceutically acceptable salt thereof according to any one of claims 1 to 3, wherein each Rₐ is independently selected from F, Cl, OH, NH₂, CH₃, CH₂CH₃, CH(CH₃)₂, OCH₃, OCH₂CH₃, OCH(CH₃)₂, 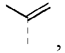 -C≡CH -C≡CCH₃, 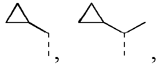 and cyclopropyl, and the CH₃, CH₂CH₃, CH(CH₃)₂, OCH₃, OCH₂CH₃, OCH(CH₃)₂, 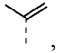 -C≡CH, -C≡CCH₃, 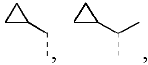 and cyclopropyl are each independently and optionally substituted by 1, 2, or 3 R.

5. The compound or the pharmaceutically acceptable salt thereof according to any one of claims 1 to 3, wherein each Rₐ is independently selected from F, Cl, OH, NH₂, CH₃, CHF₂, CF₃, CH₂CF₃, CH(CH₃)CF₃, OCH₃, OCF₃, 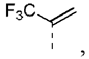 -C≡CH, -C≡CCH₃, , ,

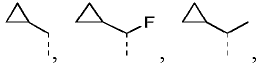

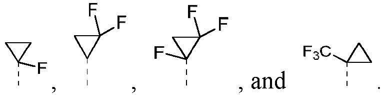

6. The compound or the pharmaceutically acceptable salt thereof according to any one of claims 1 to 3, wherein R₁ is selected from

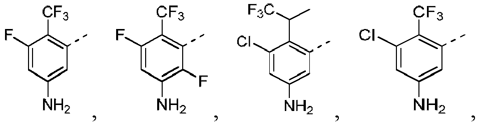

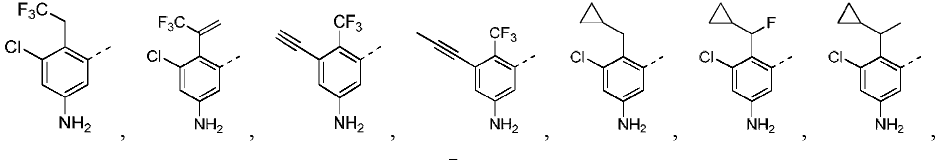

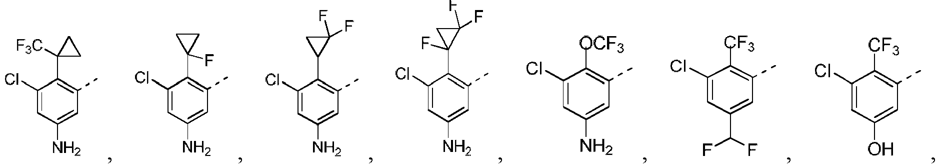

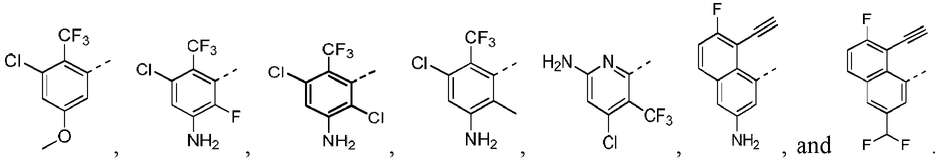

7. The compound or the pharmaceutically acceptable salt thereof according to any one of claims 1 to 3, wherein R₁ is selected from 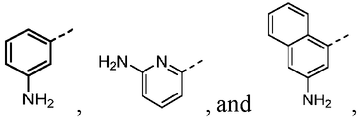 and the 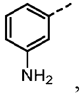 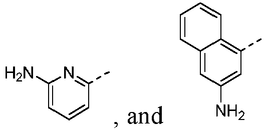 are each independently and optionally substituted by 1, 2, 3, or 4 Rₐ.

8. The compound or the pharmaceutically acceptable salt thereof according to any one of claims 1 to 3, wherein R₁ is selected from

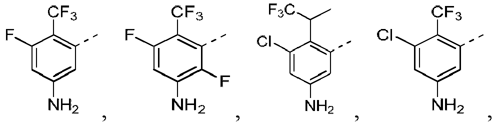

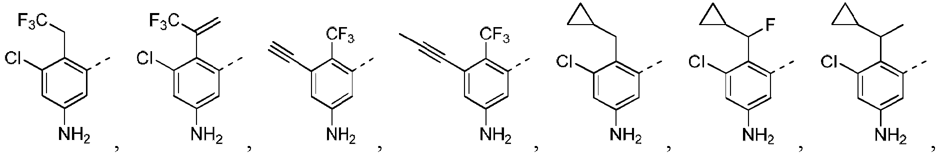

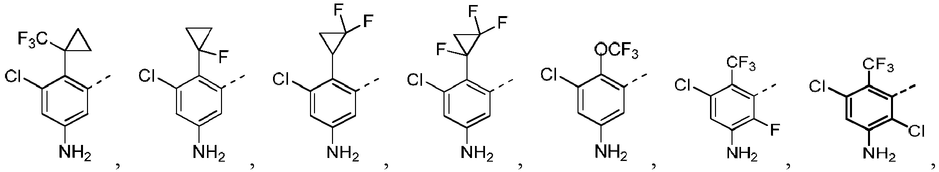

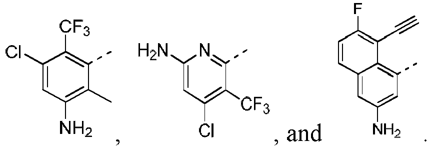

9. The compound or the pharmaceutically acceptable salt thereof according to any one of claims 1 to 3, wherein R₁ is selected from

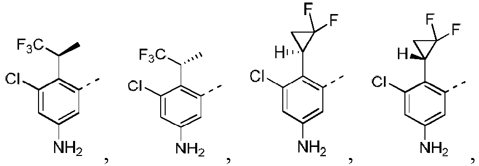

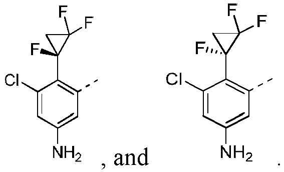

10. The compound or the pharmaceutically acceptable salt thereof according to claim 3, wherein R₂ is selected from

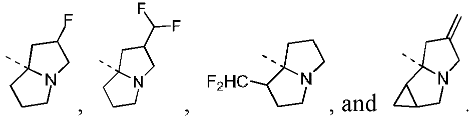

11. The compound or the pharmaceutically acceptable salt thereof according to claim 3, wherein R₂ is selected from

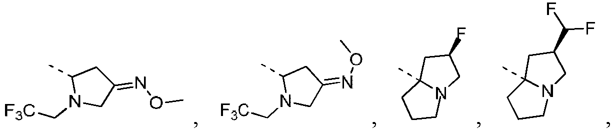

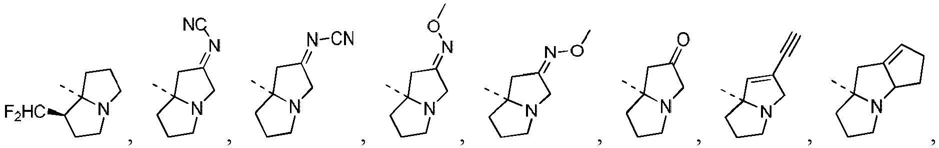

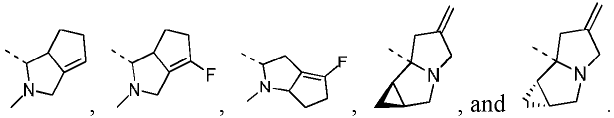

12. The compound or the pharmaceutically acceptable salt thereof according to claim 1 or 2, wherein X is N.

13. The compound or the pharmaceutically acceptable salt thereof according to claim 1 or 2, wherein the compound is selected from: 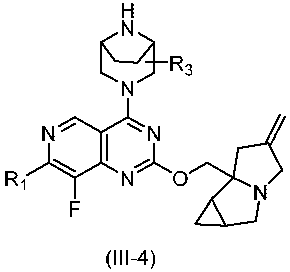 wherein R₁ and R₃ are as defined in claim 1.

14. The compound or the pharmaceutically acceptable salt thereof according to claim 1 or 2, wherein the compound is selected from: 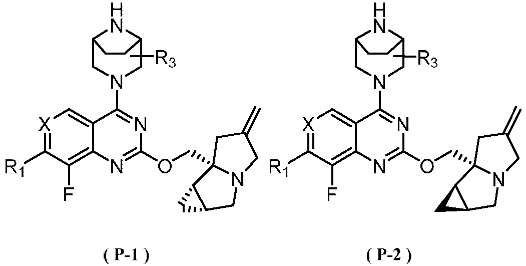 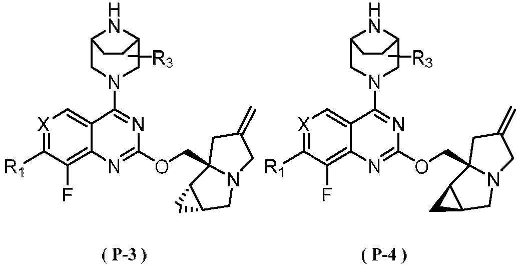 wherein X, R₁, and R₃ are as defined in claim 1.

15. The compound or the pharmaceutically acceptable salt thereof according to claim 3, wherein the compound is selected from: 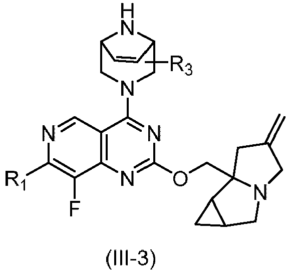 wherein R₁ and R₃ are as defined in claim 3.

16. A compound as shown below or a pharmaceutically acceptable salt thereof, 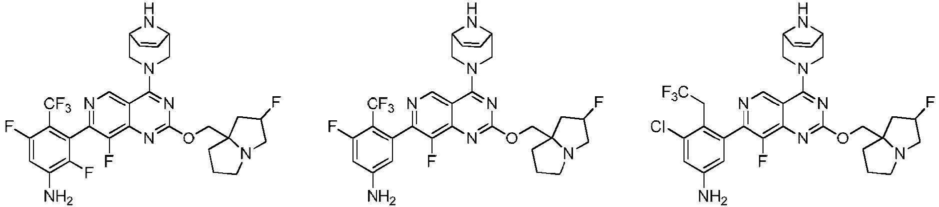 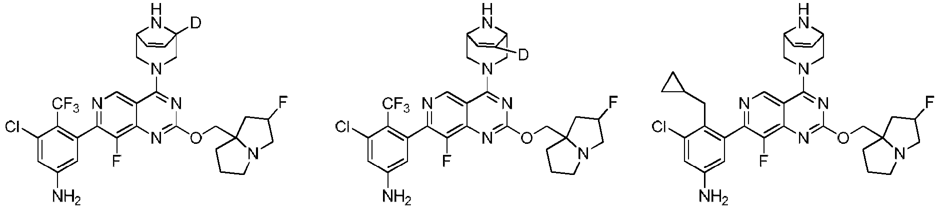 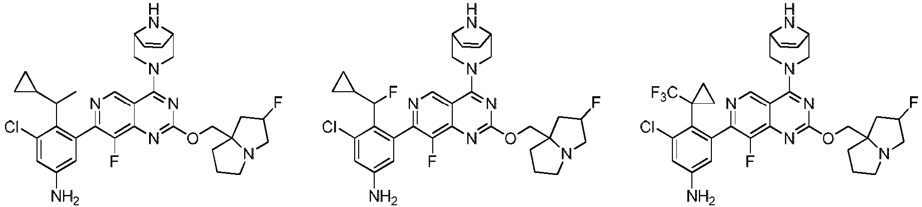 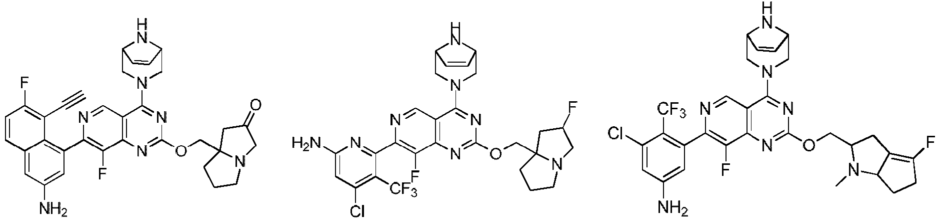 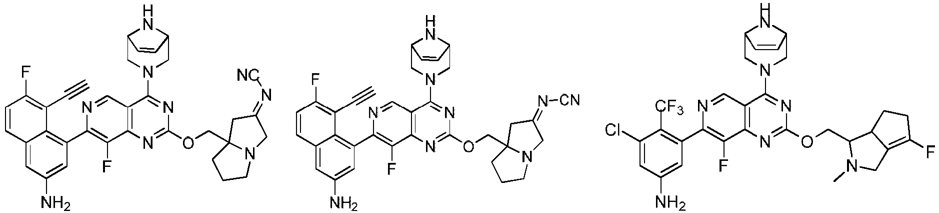 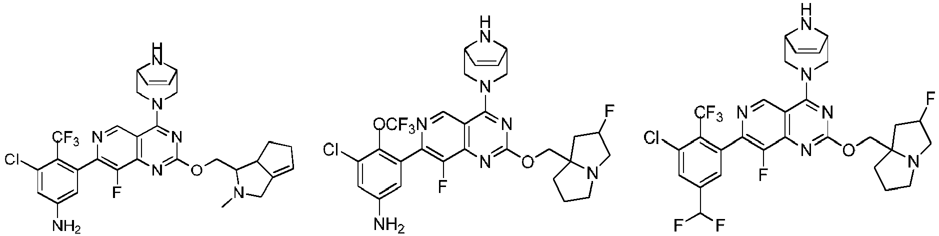 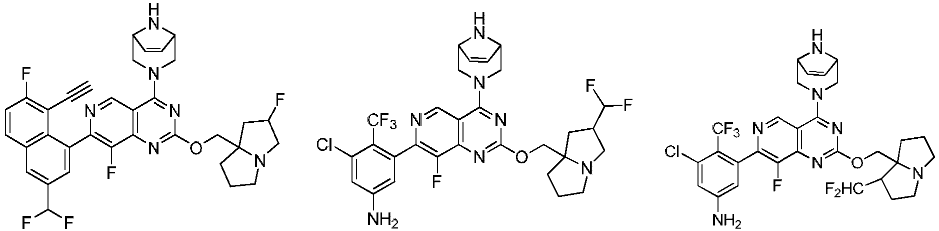 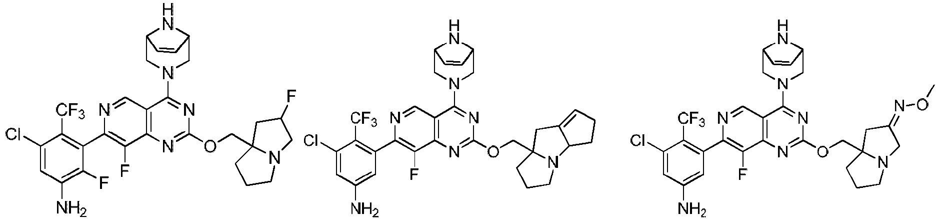 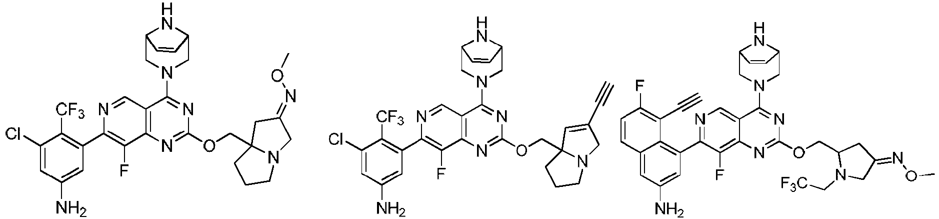 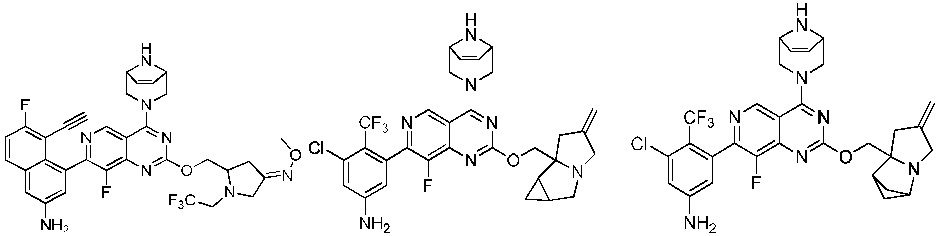 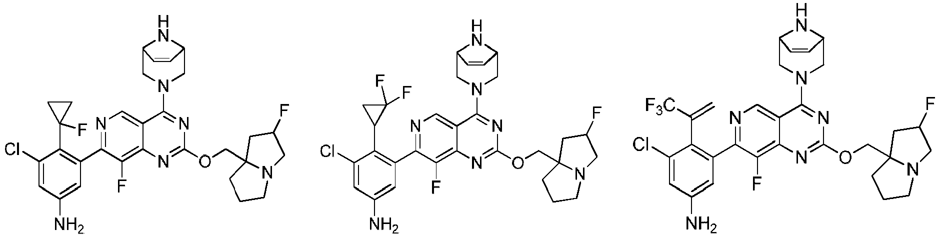 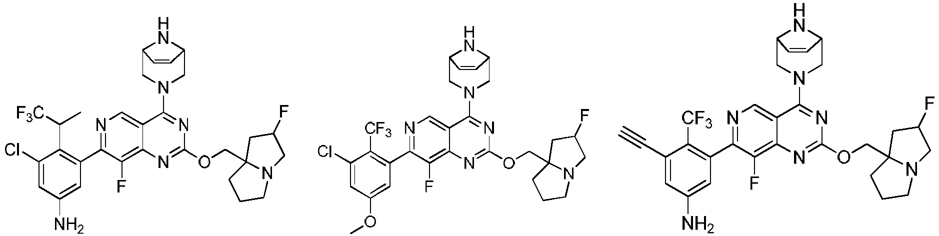 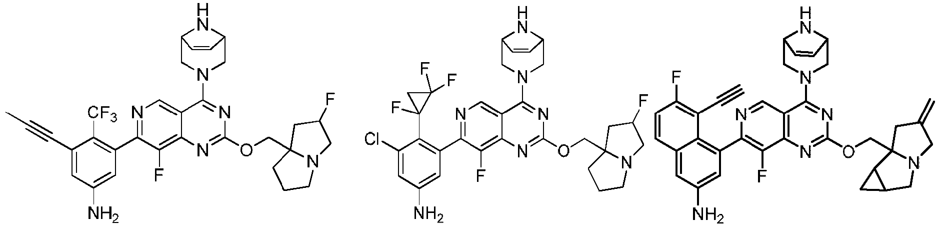 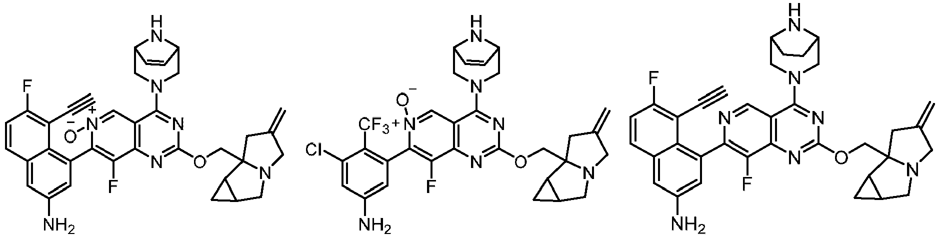 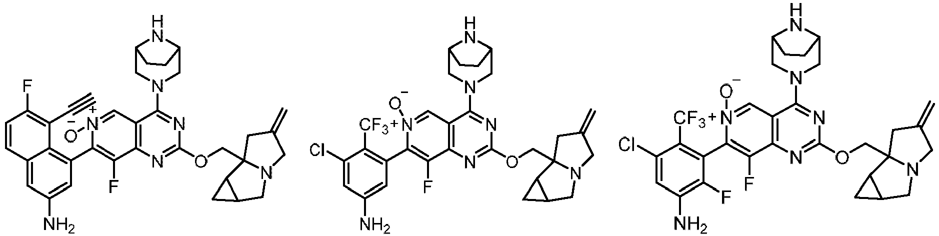 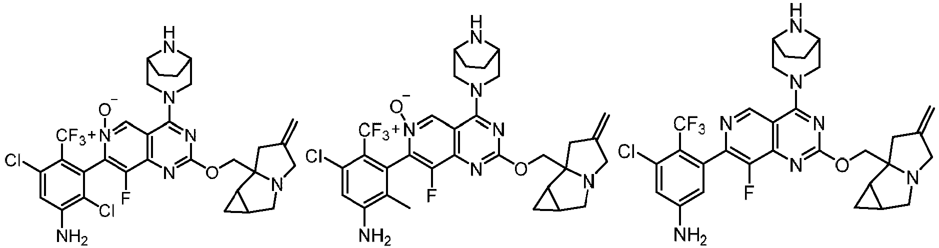 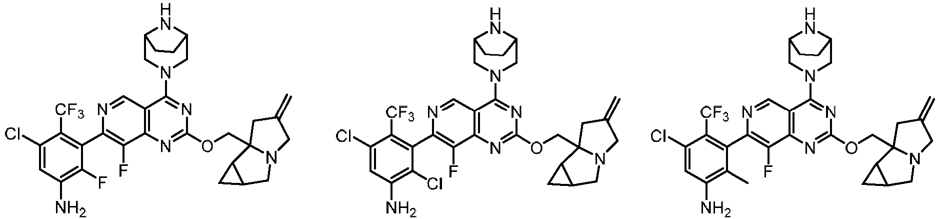 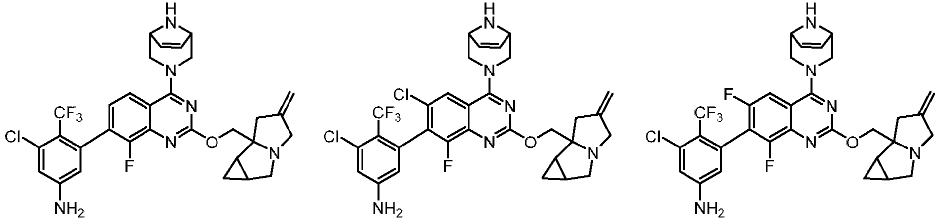 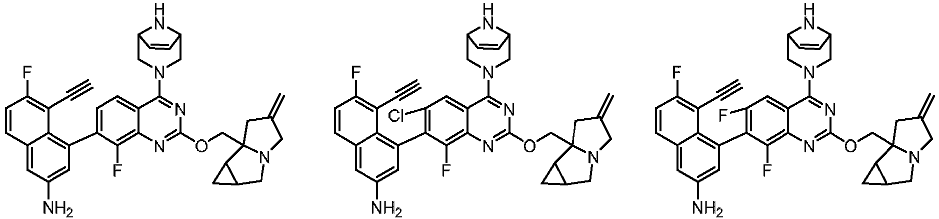 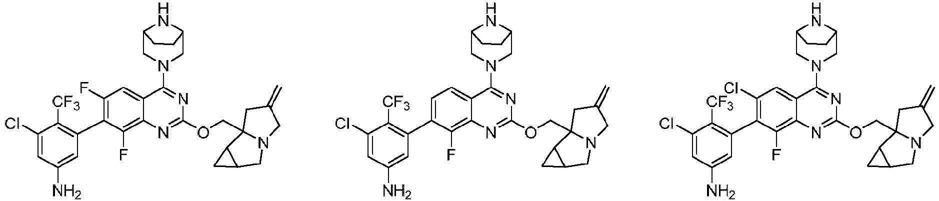 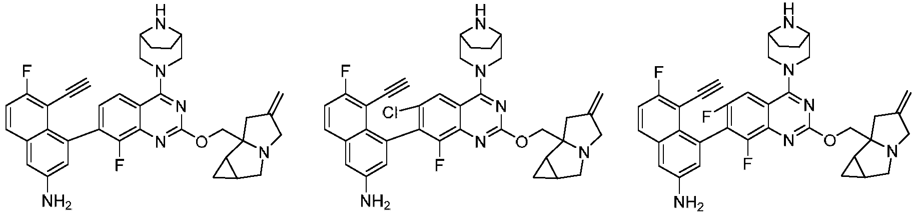 or

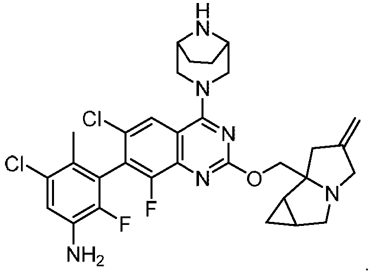

17. The compound or the pharmaceutically acceptable salt thereof according to claim 16, selected from, 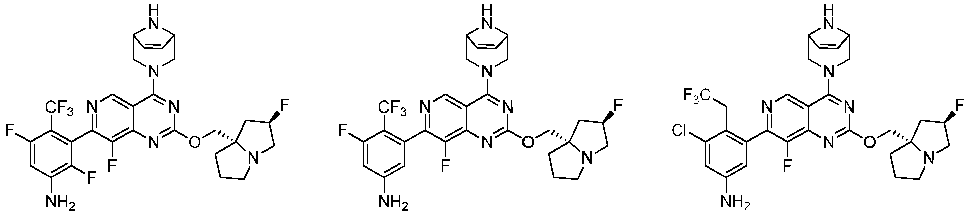 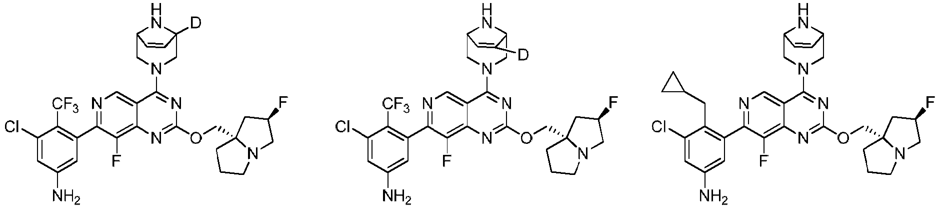 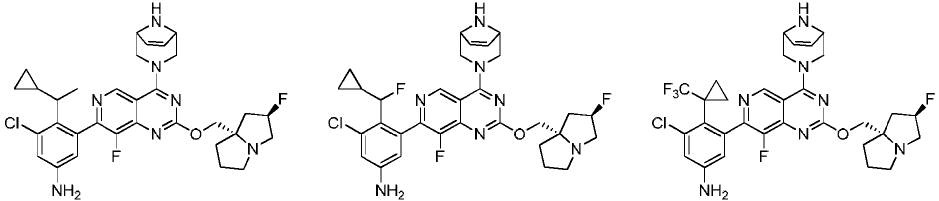 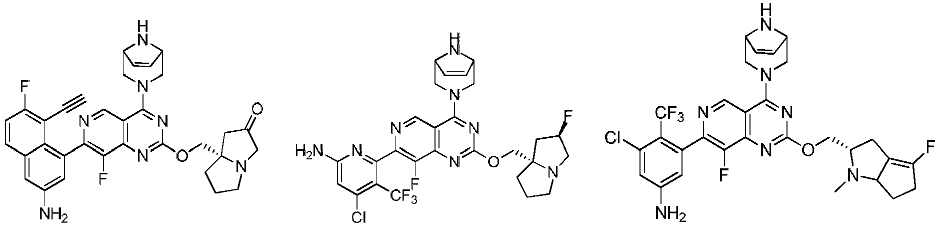 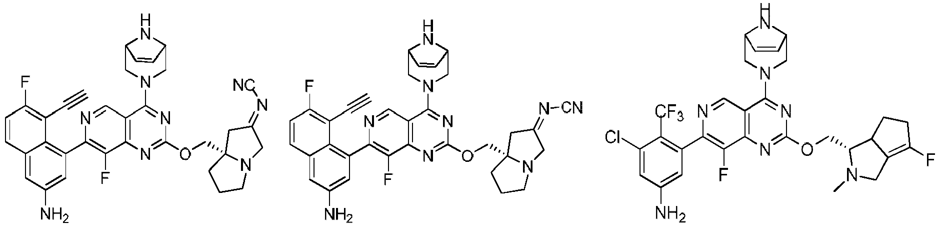 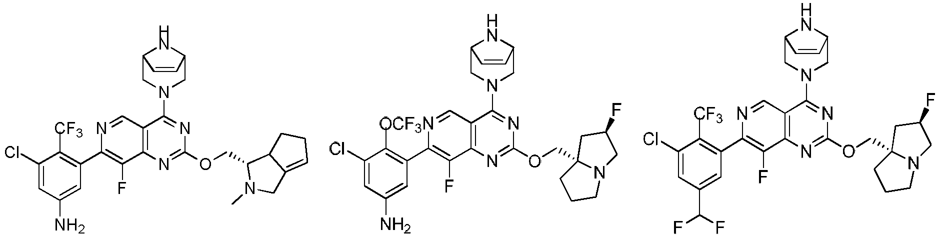 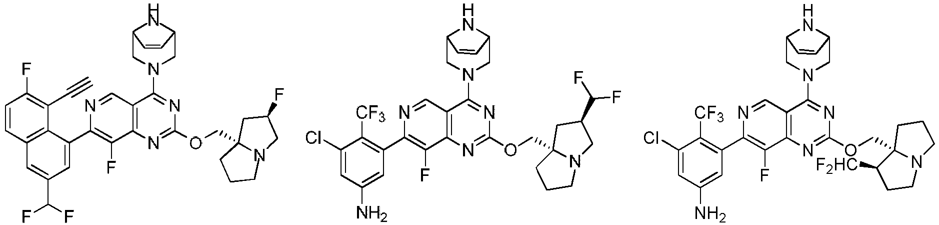 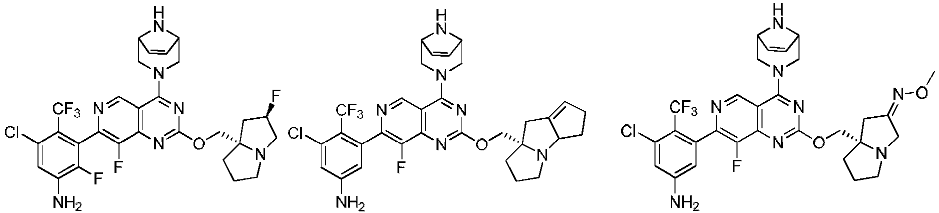 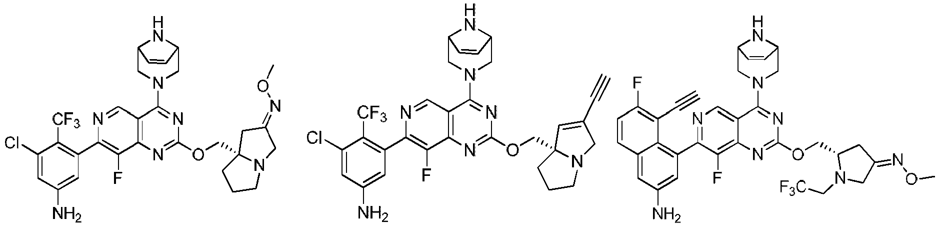 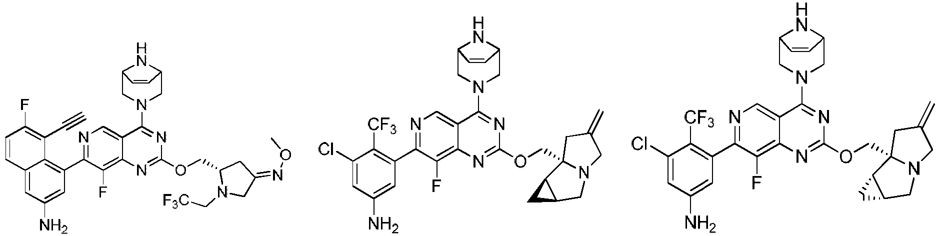 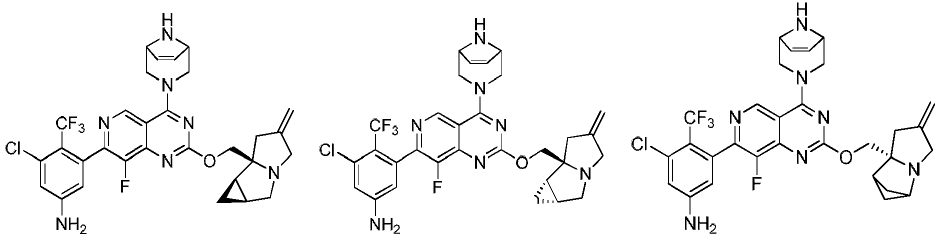 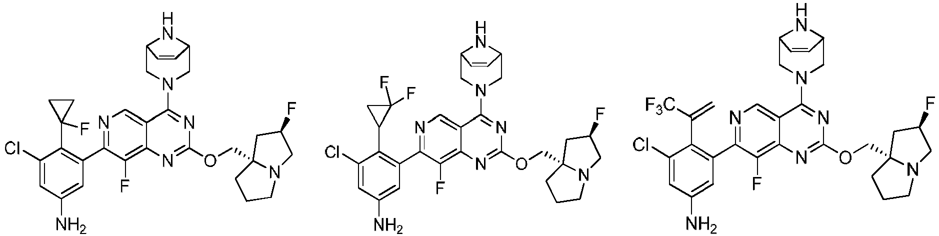 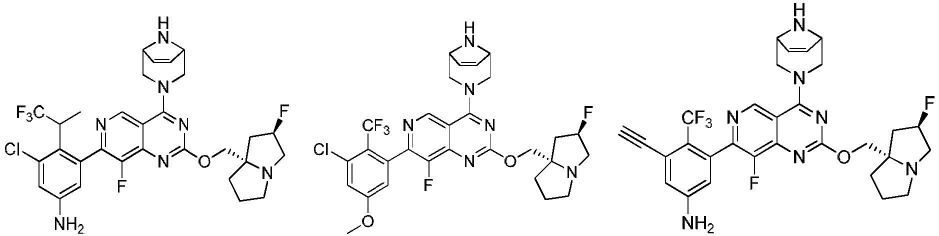 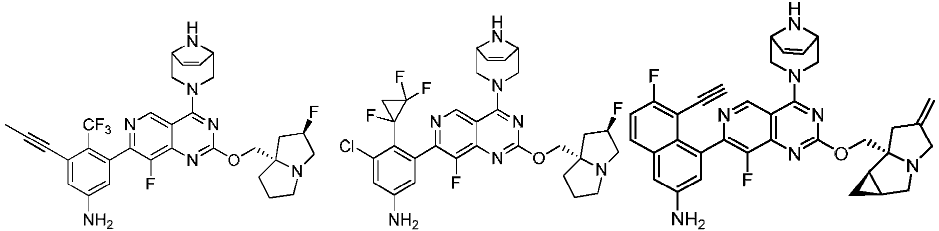 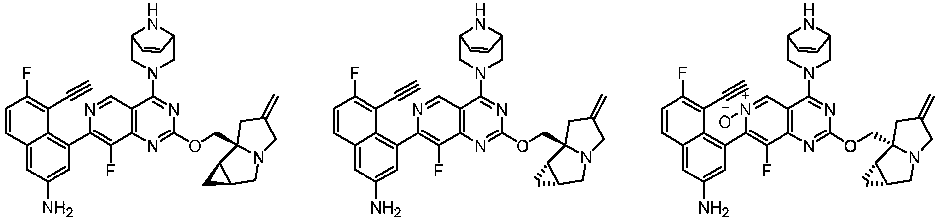 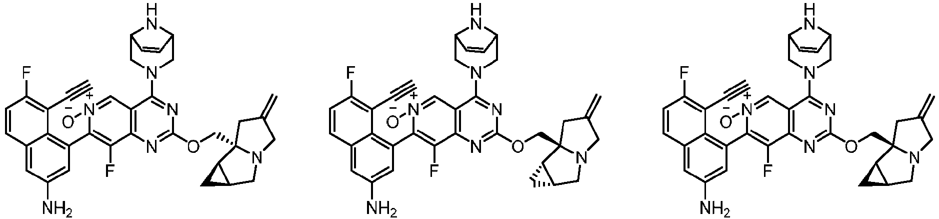 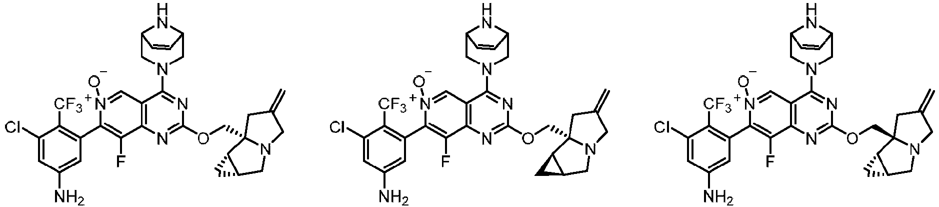 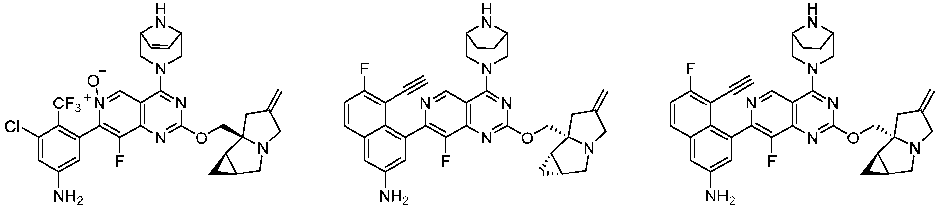 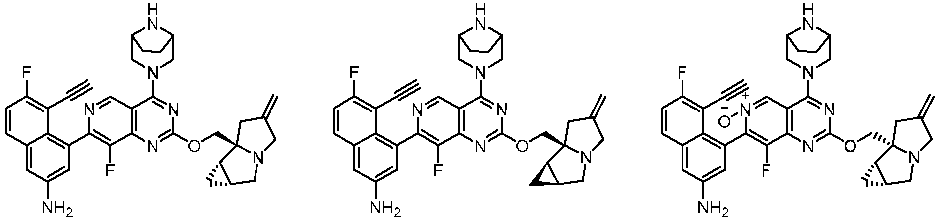 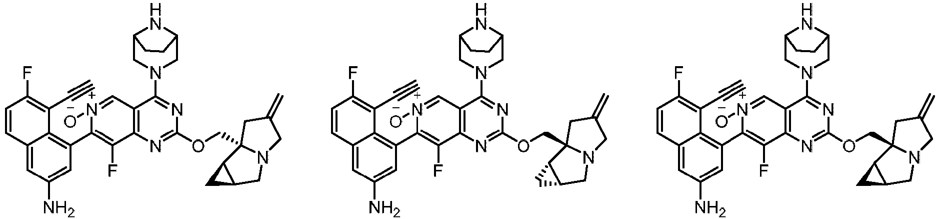 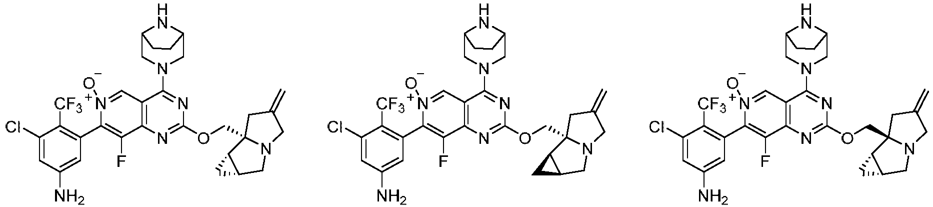 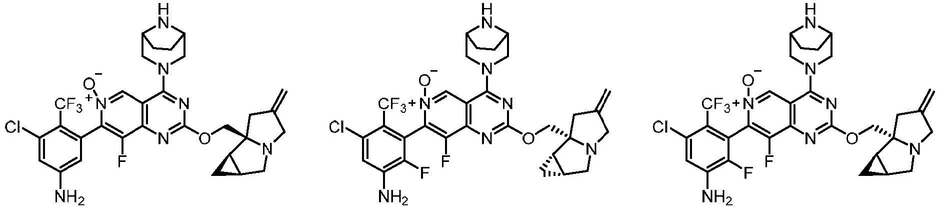 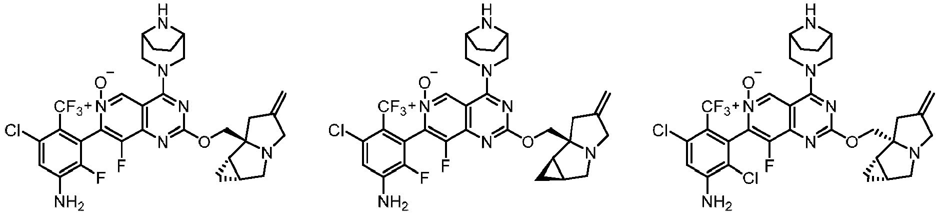 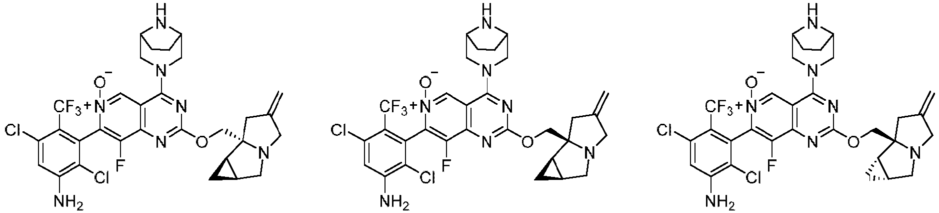 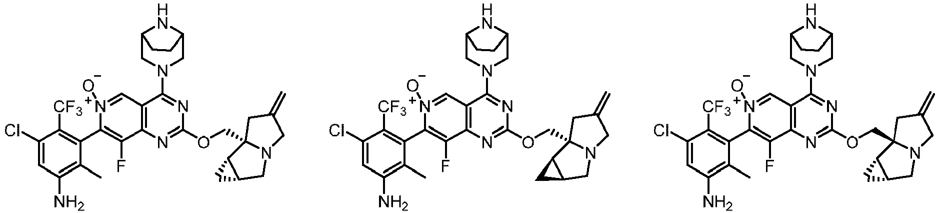 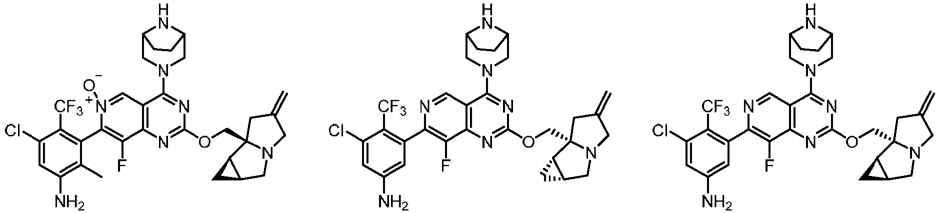 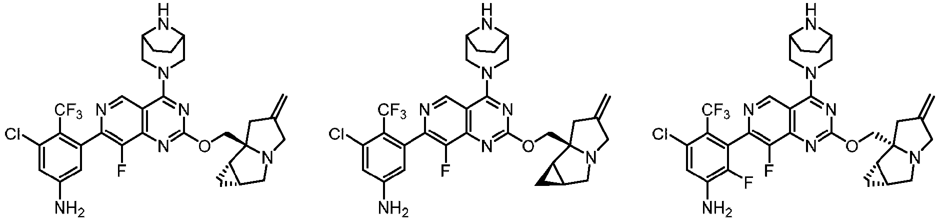 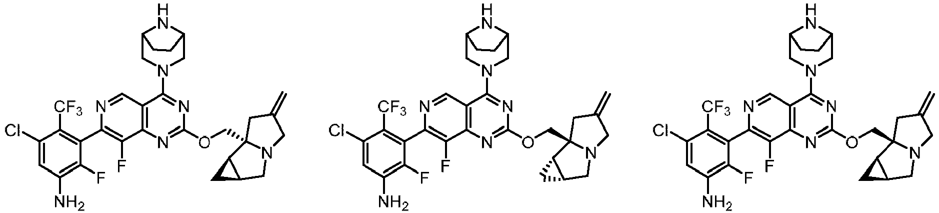 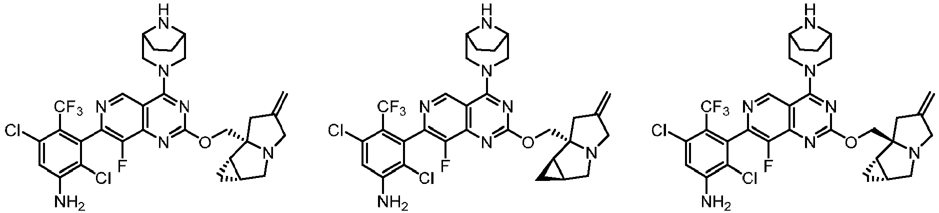 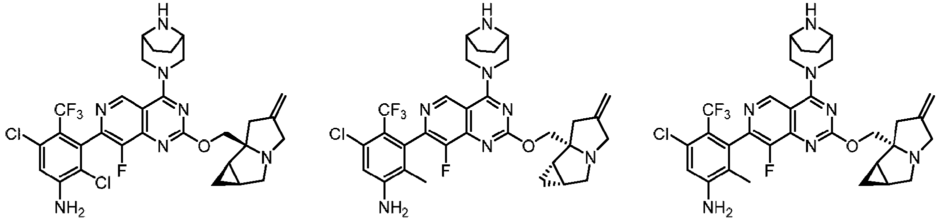 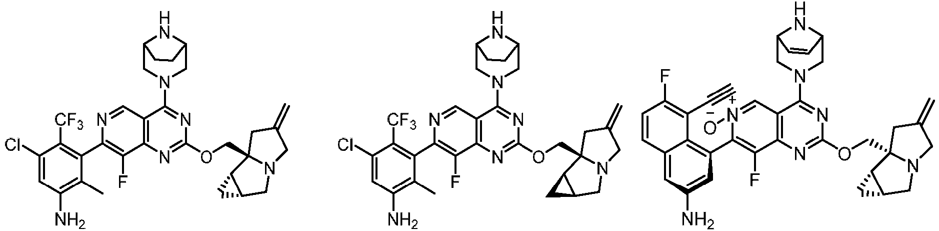 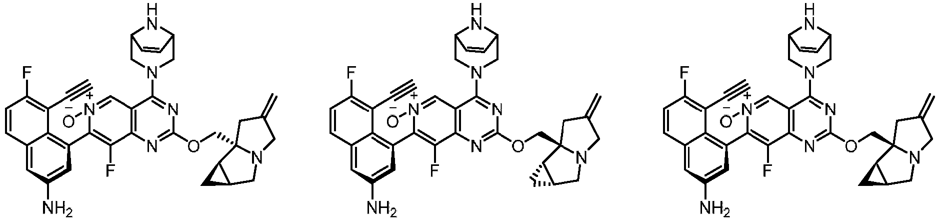 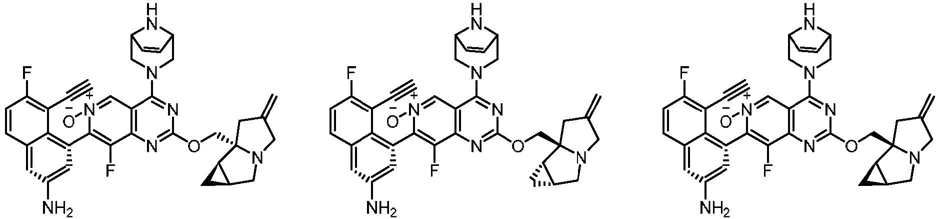 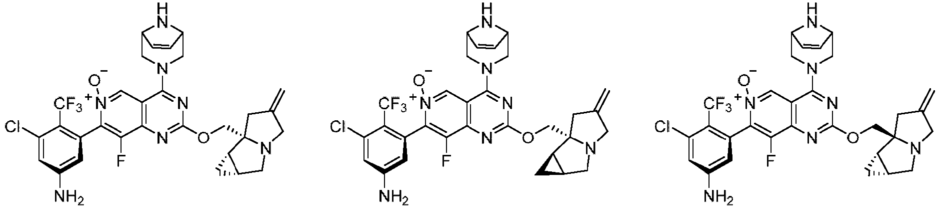 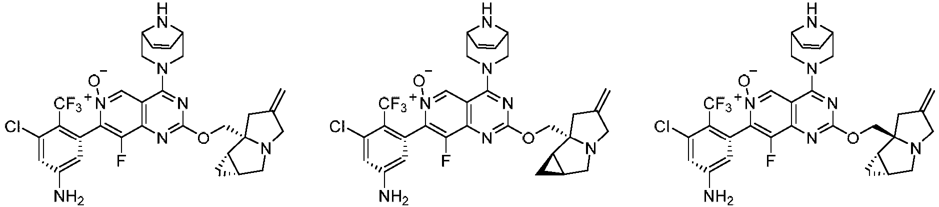 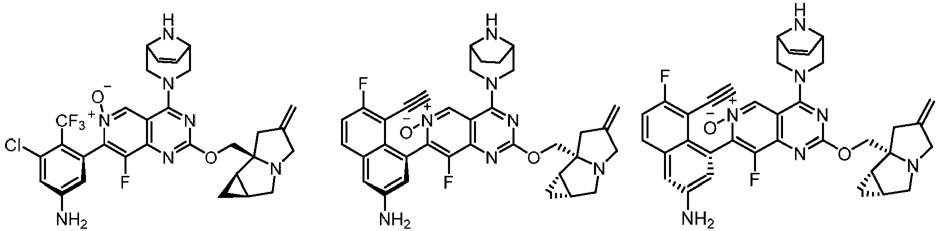 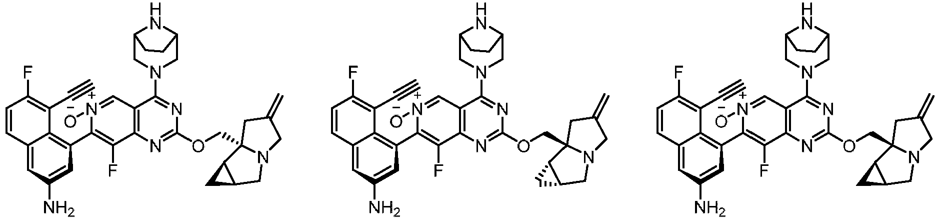 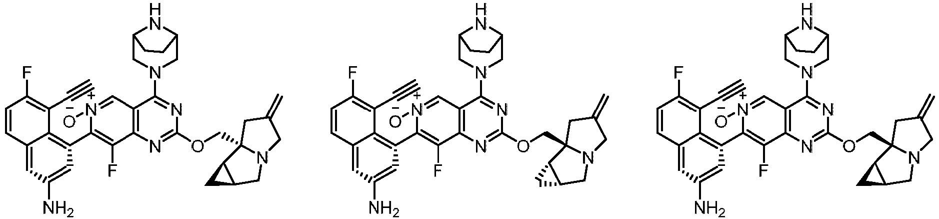 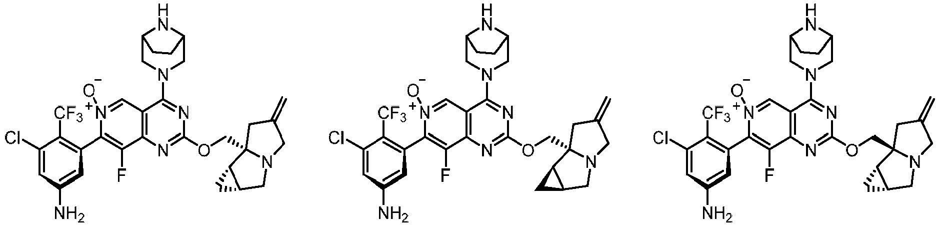 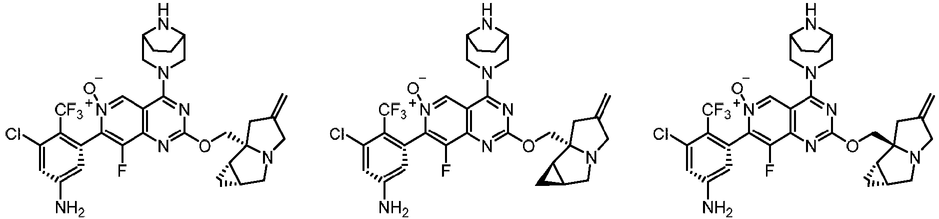 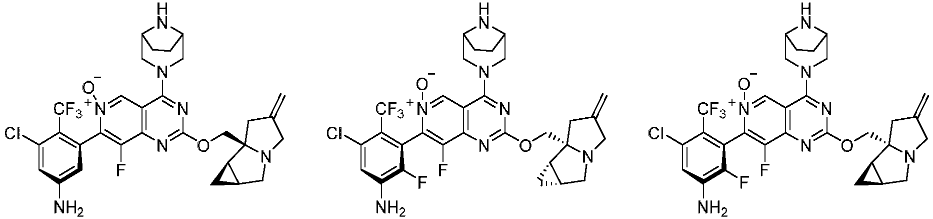 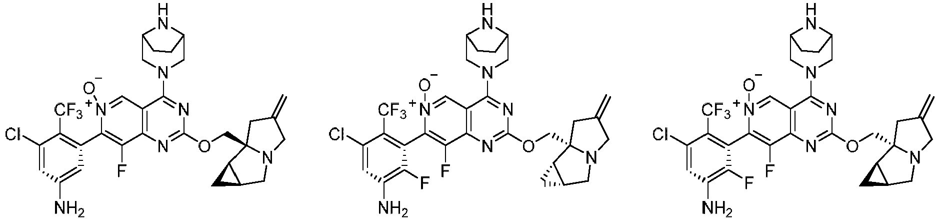 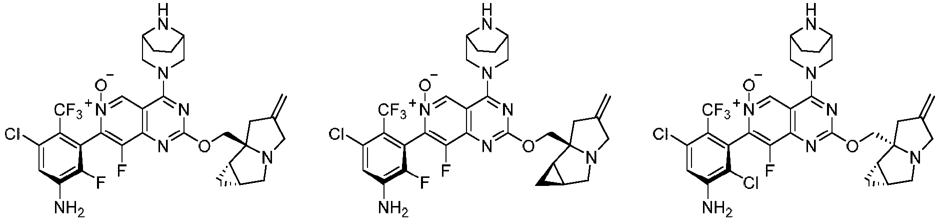 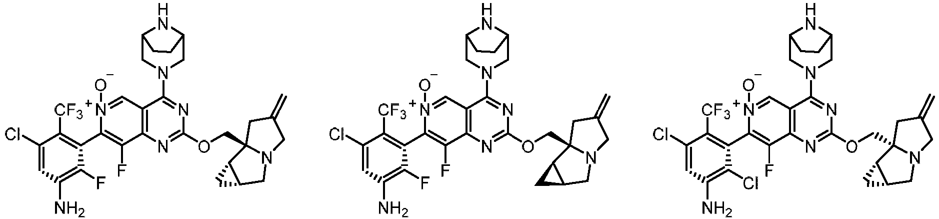 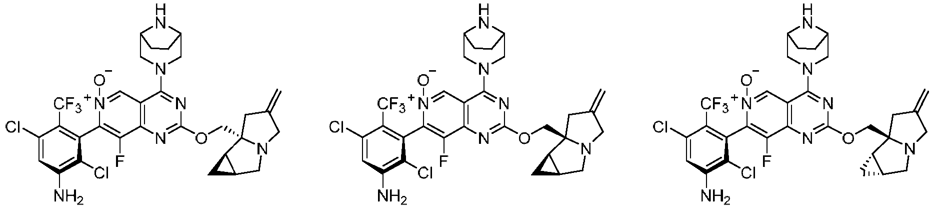 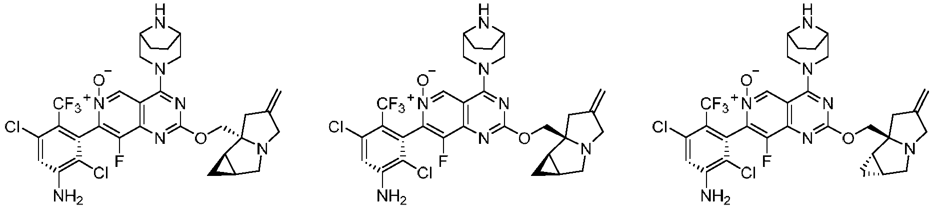 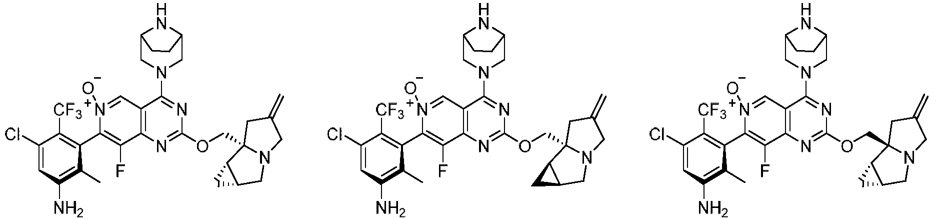 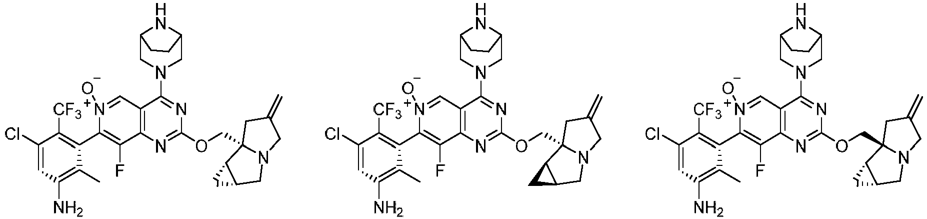 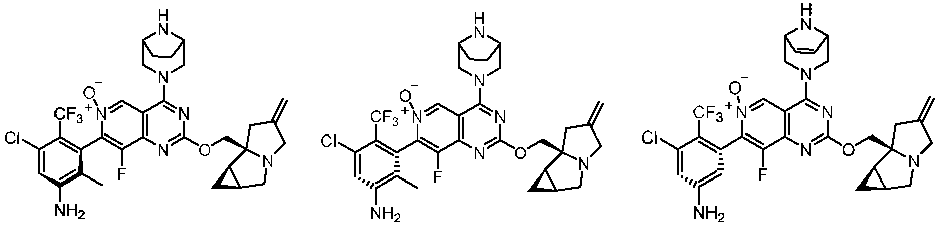 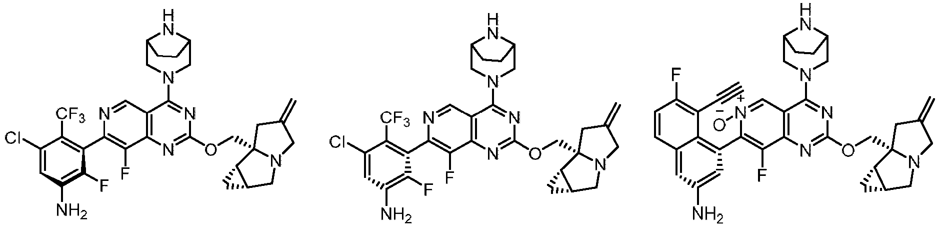 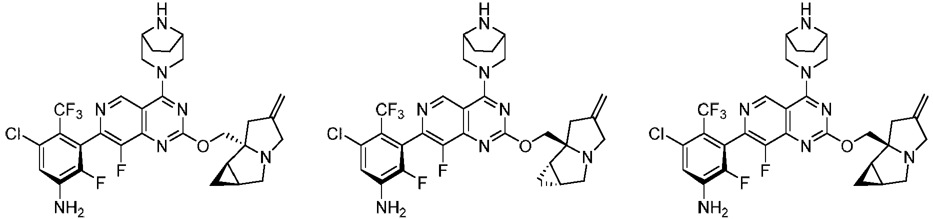 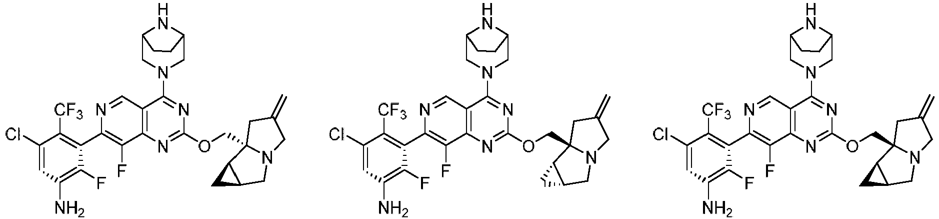 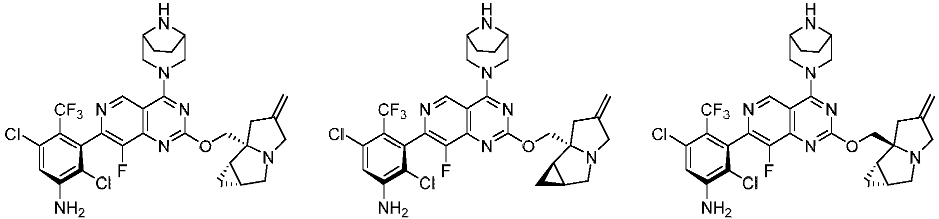 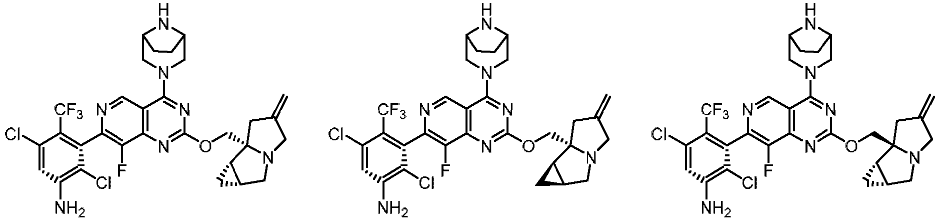 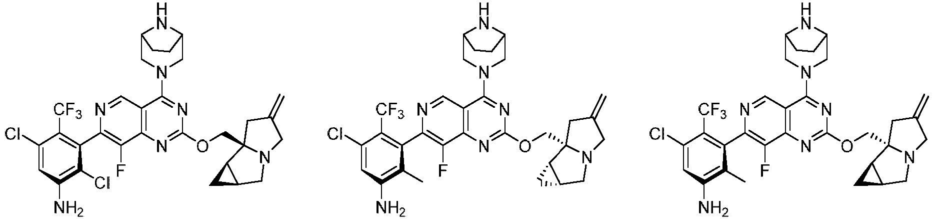 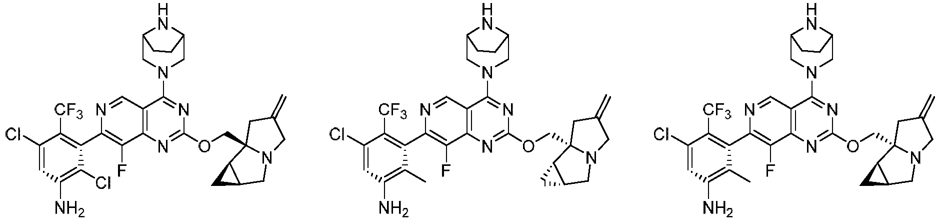 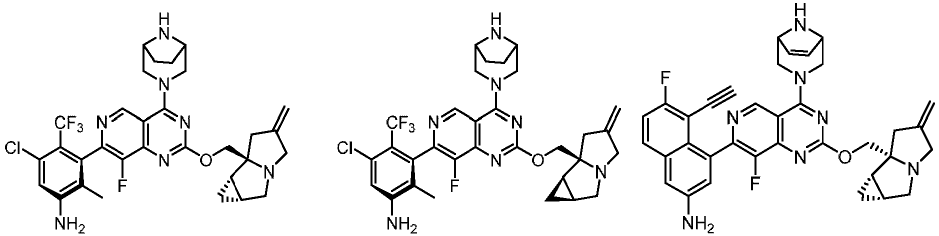 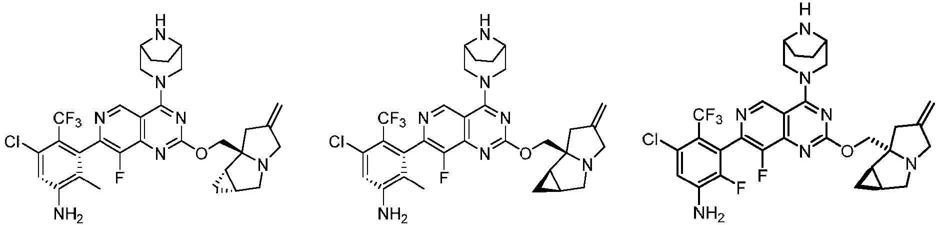 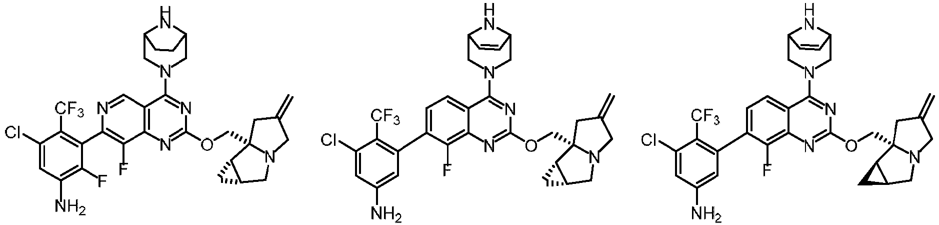 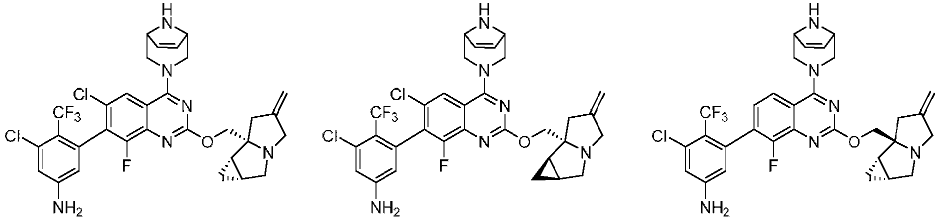 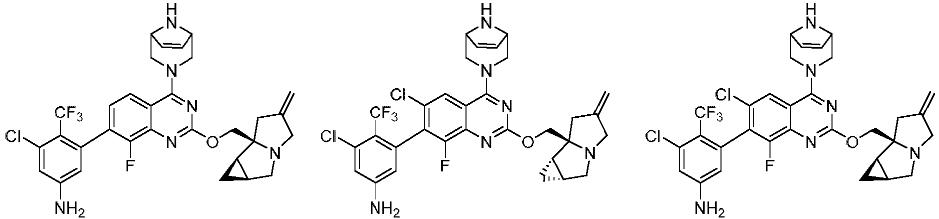 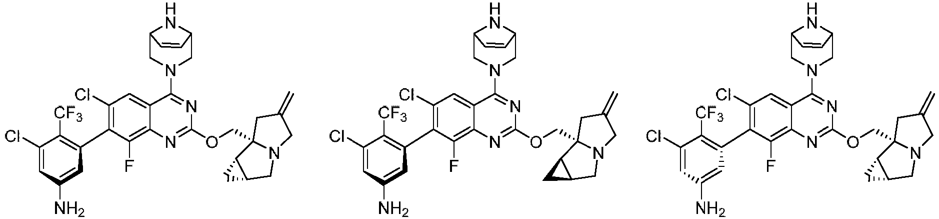 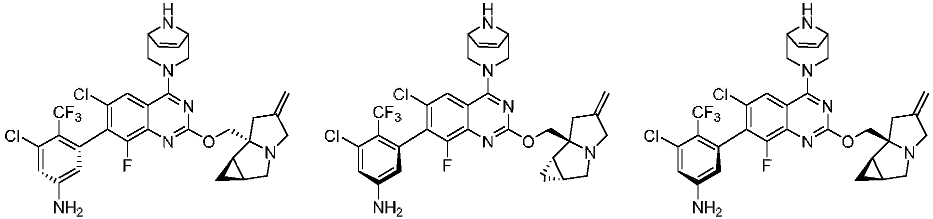 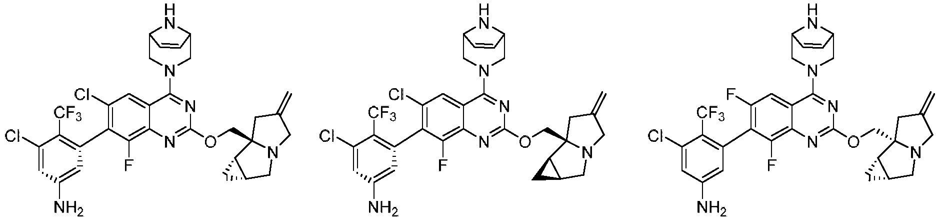 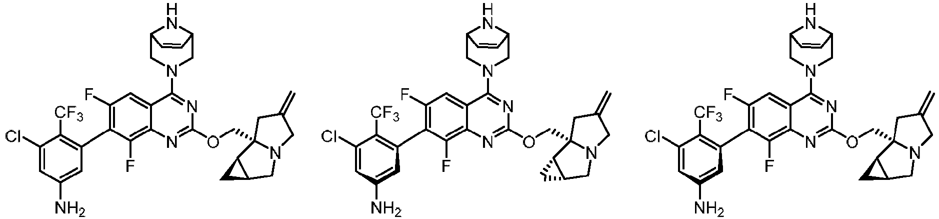 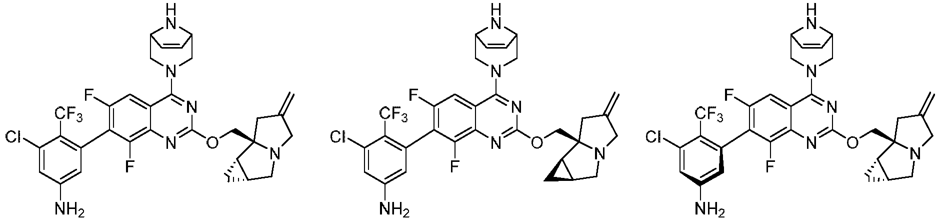 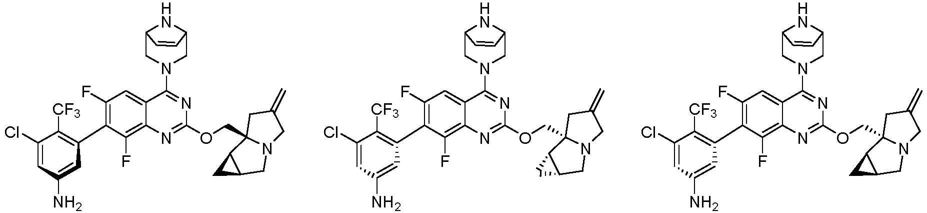 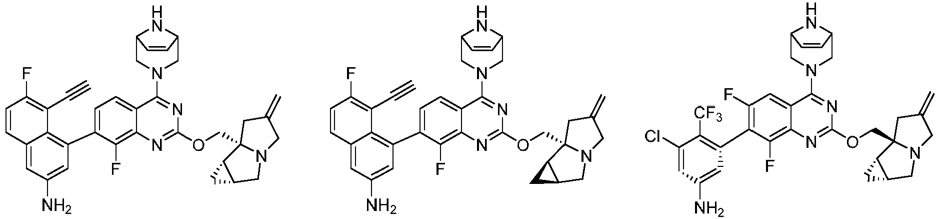 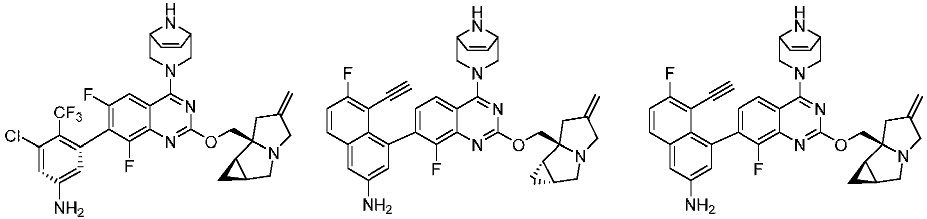 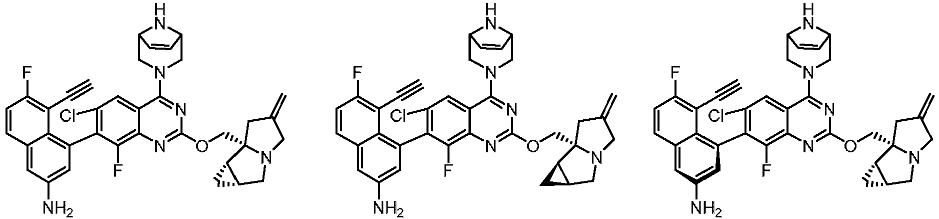 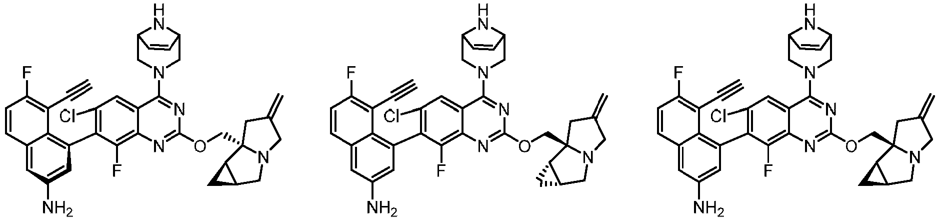 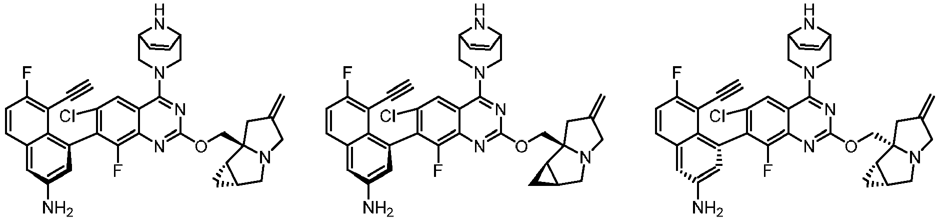 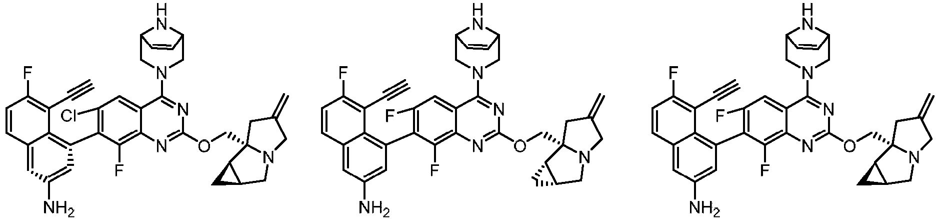 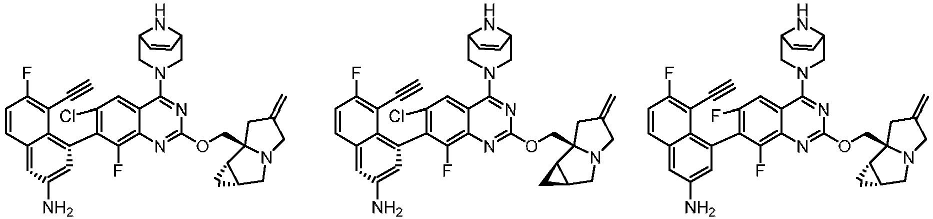 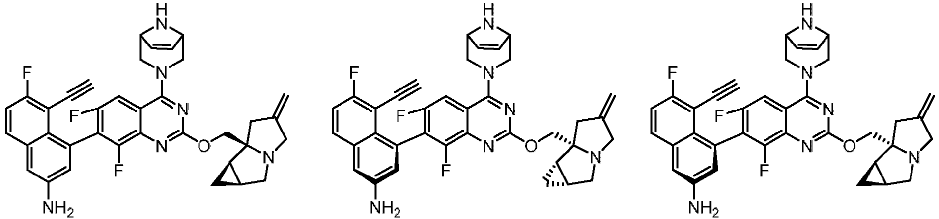 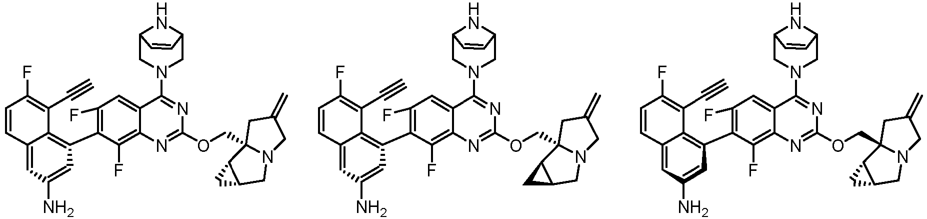 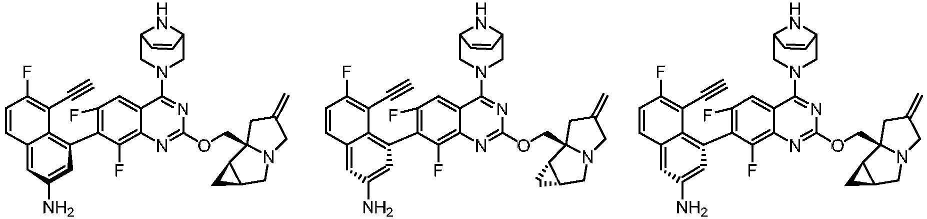 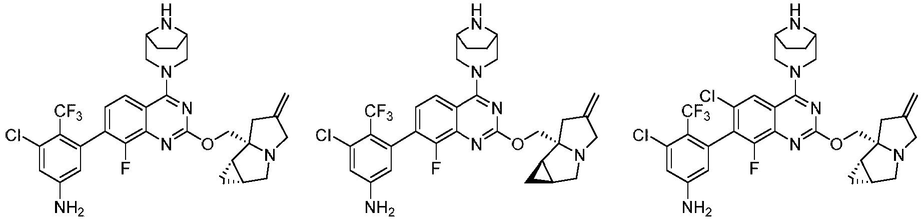 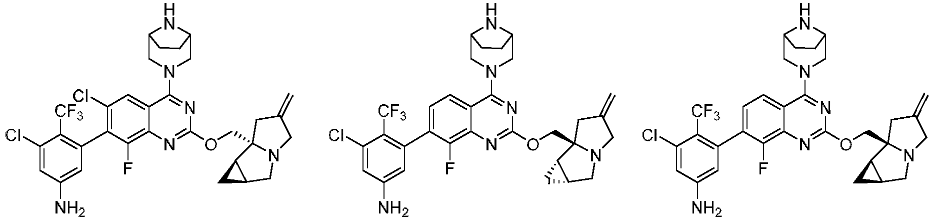 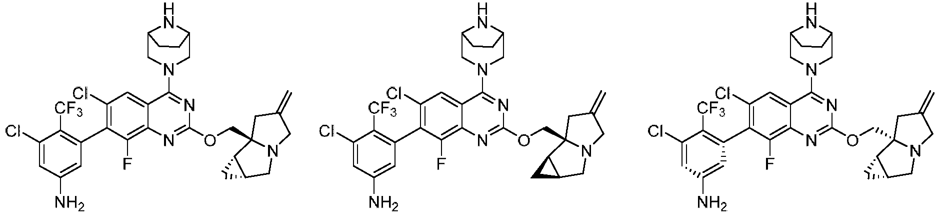 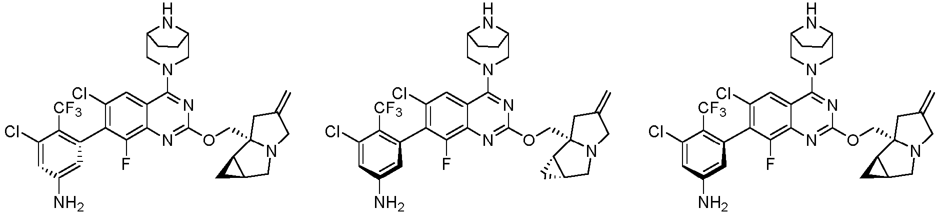 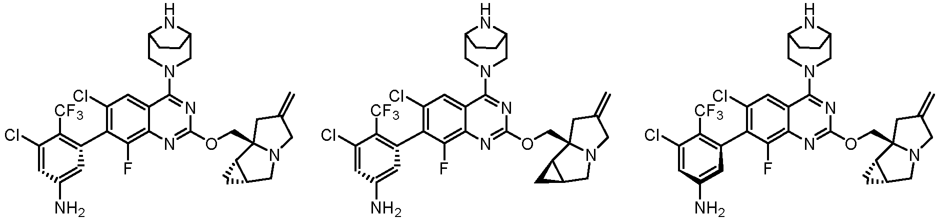 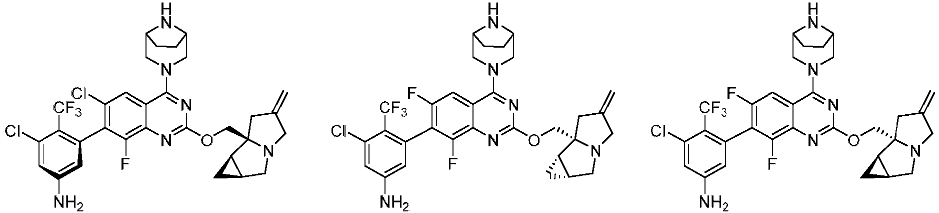 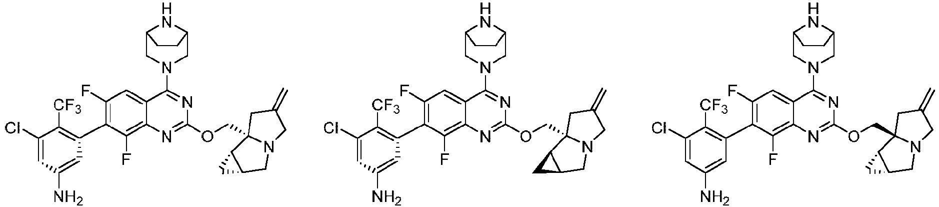 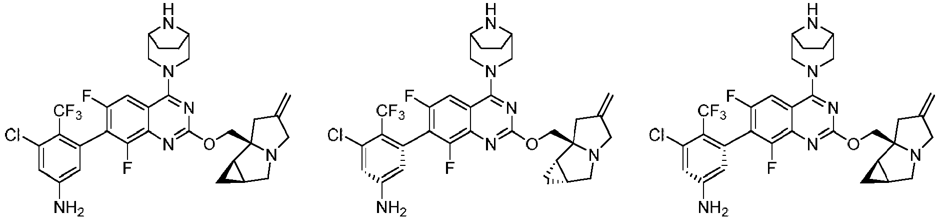 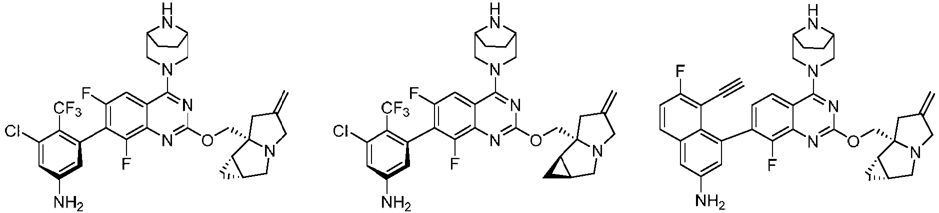 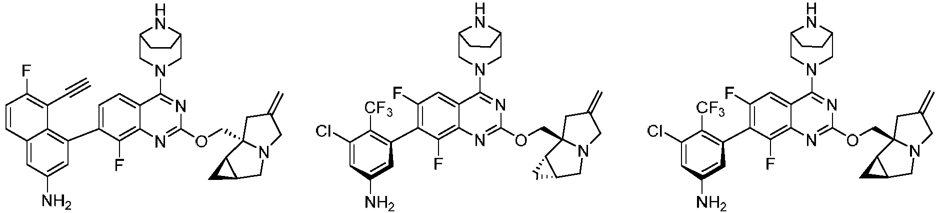 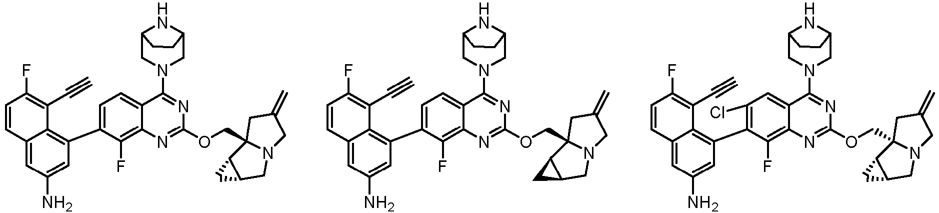 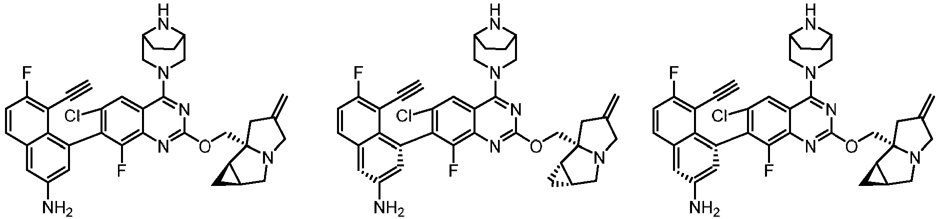 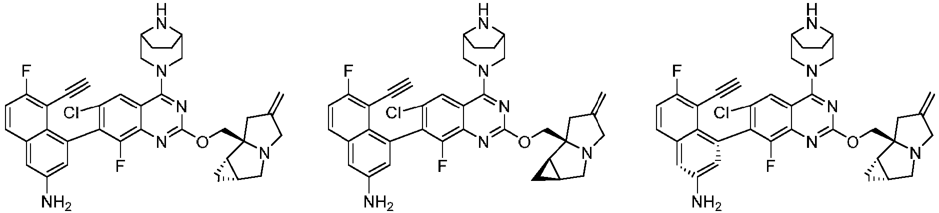 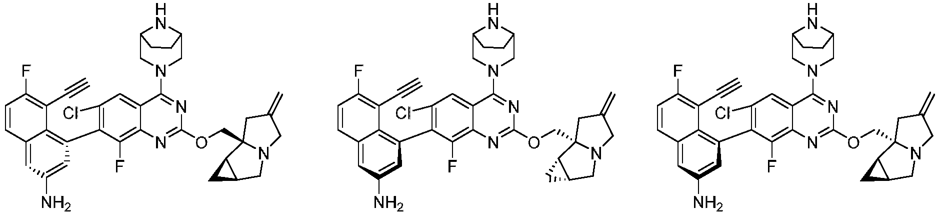 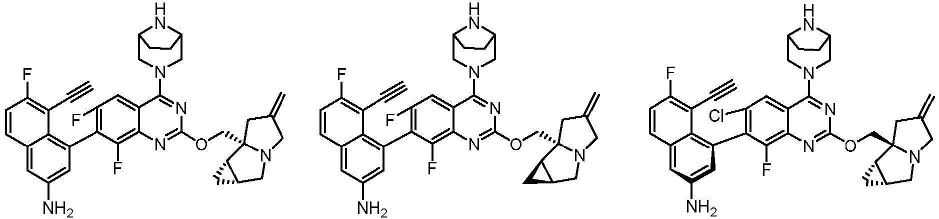 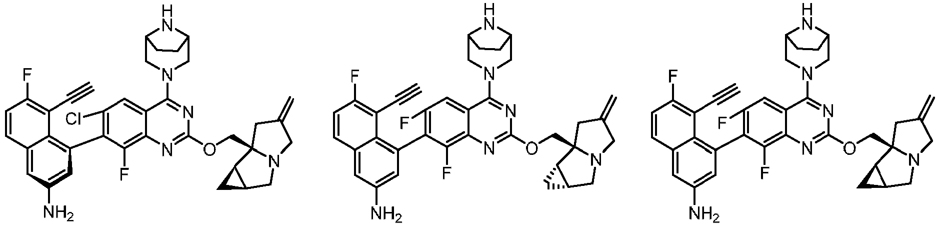 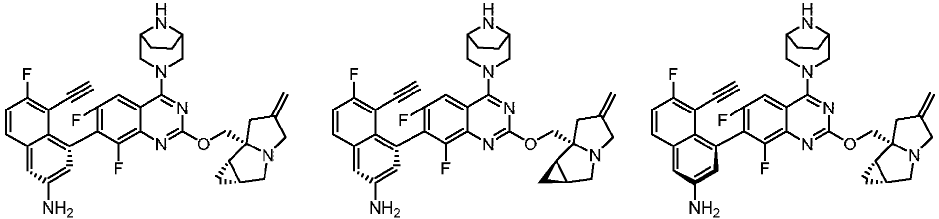 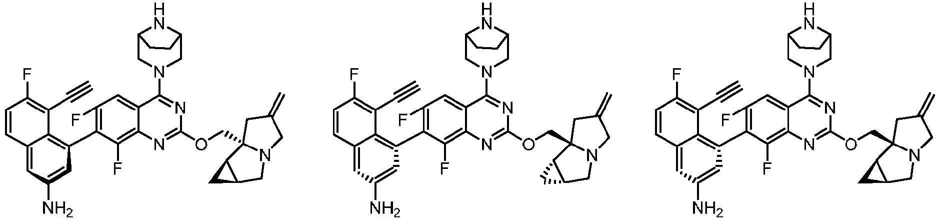 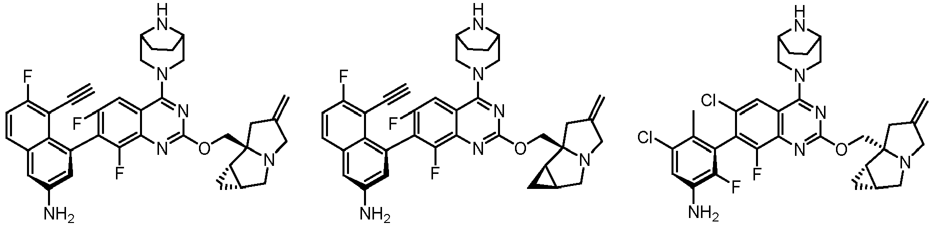 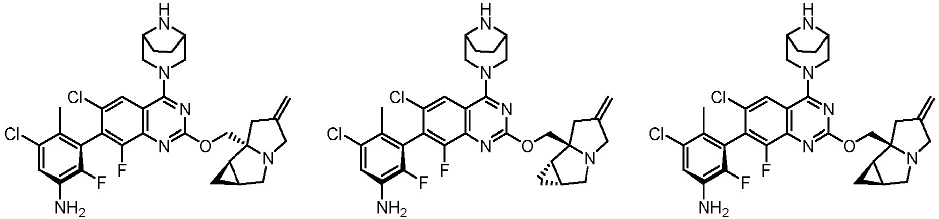 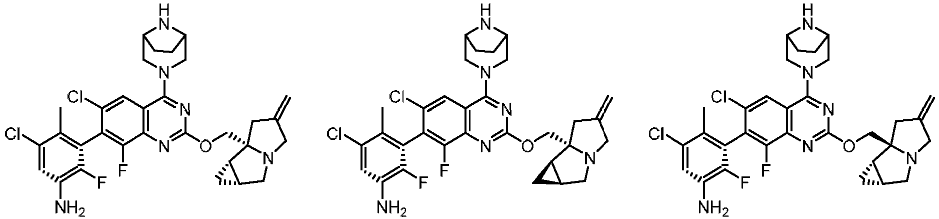 and

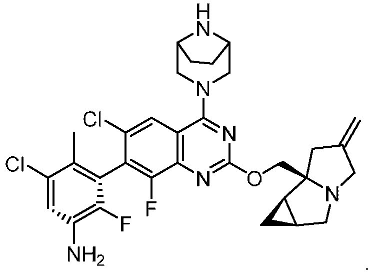

18. The compound or the pharmaceutically acceptable salt thereof according to claim 17, wherein the compound is selected from: 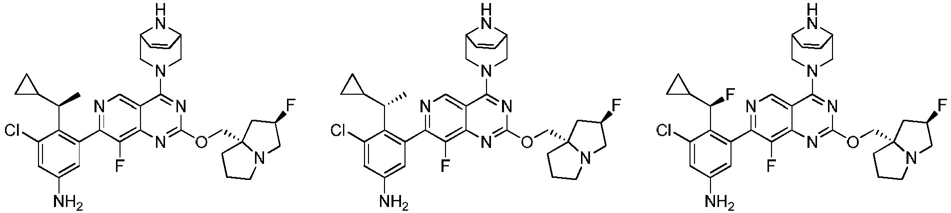 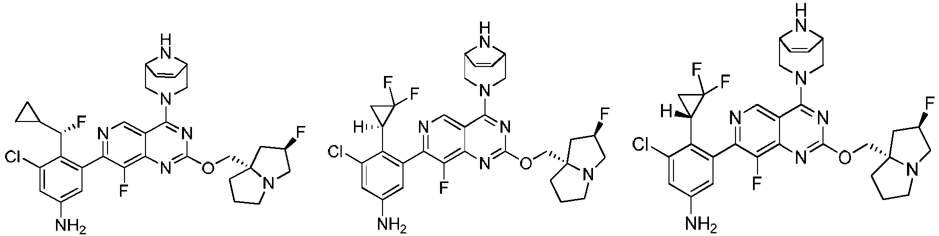 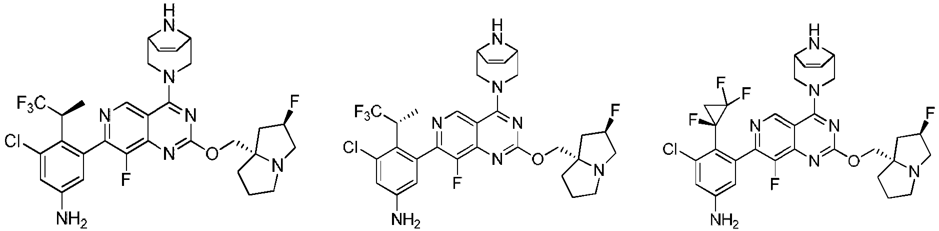 and

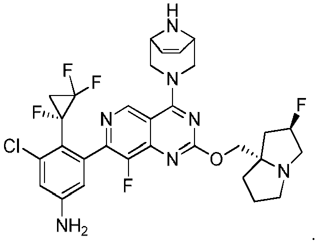

19. A use of the compound or the pharmaceutically acceptable salt thereof as defined in any one of claims 1 to 18 in the manufacture of a compound for treating solid tumors with KRAS^{G12D} mutation.
